(19) Europäisches Patentamt
European Patent Office
Office européen des brevets

(11) **EP 3 955 257 A1**

(12) **EUROPEAN PATENT APPLICATION**

(43) Date of publication:
**16.02.2022 Bulletin 2022/07**

(21) Application number: **20191014.8**

(22) Date of filing: **13.08.2020**

(51) International Patent Classification (IPC):
***G16H 20/00*** *(2018.01)*    ***G16H 50/00*** *(2018.01)*

(52) Cooperative Patent Classification (CPC):
**G16H 20/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Oncomfort SA
1300 Wavre (BE)**

(72) Inventors:
• **Jooris, Diane
3080 Tervuren (BE)**
• **Huyghe, Mario
8792 Desselgem (BE)**
• **Toussaint, Clémence
4210 Burdinne (BE)**

(74) Representative: **De Clercq & Partners
Edgard Gevaertdreef 10a
9830 Sint-Martens-Latem (BE)**

(54) **A METHOD AND SYSTEM FOR MEASUREMENT OF A LEVEL OF ANXIETY COMBINED WITH AND/OR CORRELATED WITH A LEVEL OF A MODIFIED STATE OF CONSCIOUSNESS AND/OR A LEVEL OF PAIN**

(57)    There is described a computer-implemented method for measurement of a level of anxiety combined with and/or correlated with a level of a modified state of consciousness and/or a level of pain. There is received measured data comprising EEG data. From the EEG data there is extracted a group 8 indicator based on a power, P-power (dt), associated with a delta-theta frequency band, dt, within a delta-theta frequency range. From the EEG data there is further extracted a group 1 indicator based on, a power, F-power(dt), associated with a delta-theta frequency band, dt, within a delta-theta frequency range; and/or a group 4 indicator corresponding to a power, power (ta), associated with a theta-alpha frequency band, ta, within a theta-alpha frequency range. There is determined, based on said group 8 indicator, a level of anxiety. There is further determined, based on said group 1 indicator, a depth of state, DoS, and/or, based on said group 4 indicator, and optionally said group 3 indicator and/or group 5 indicator, a level of pain. A correlation based on a plurality of LoA, LoP and/or DoS measurements can then be determined.

FIG. 5

**Description**

**Field of the invention**

The present invention is in a field of a computer-implemented method and system for automatically measuring a level of anxiety, a level of a modified state of consciousness, and/or a level of pain in a subject.

**Background to the invention**

Anxiety and, for example, the impact of anxiety on patient comfort and the effectiveness of medical treatments, is a well-known and universal problem. Anxiety management is thus an important branch of medicine. However prior art methods for assessment of the level of anxiety a subject is experiencing have important drawbacks and are not able to provide for an efficient, reliable, robust, objective and reproducible indication of the level of anxiety as experienced by a subject. This problem is immediately apparent when the subject is not capable of providing for a reliable or clear description of the level of anxiety experienced, such as for example in the case of non-verbal children, persons with a disability, .... Further, similarly, there is a need for an improved measurement of the level of pain as perceived by a subject. Still further, similarly there is specifically a need for a reliable measurement of the level of anxiety or the level of pain, while also being able to reliably quantify any modified state of consciousness that the subject might be experiencing. Such an improved measurement of the level of anxiety, level of pain and/or modified state of consciousness is important to robustly and reliably assess the impact of each of these parameters and/or any interactions between them on the subject. These measurements will provide a more reliable insight into the condition in which the subject is. Such insights might then be used to optimize a treatment plan and/or to reduce risks associated with treatments aimed at modifying the level of anxiety, the level of pain and/or modifying the state of consciousness of a subject.

Currently, in order to assess the level of anxiety as experienced by the patient or the level of pain as perceived by the subject, use is made of self-reporting tools. However, it is clear that such manual tools that rely on self-reported assessment of the patient are prone to undesired subjective bias and lack efficiency, reliability, precision and reproducibility. Further such self-reporting tools lack the ability to efficiently take into account the multi-dimensional aspect of anxiety, the level of pain as perceived by a subject, and are difficult to apply in a context of a modified state of consciousness, such as for example in the case of pharmacological or non-pharmacological sedation. Typically, such self-reporting tools are extensive and burdensome to administer and complex and intensive to process because of a large number of questions.

Accurately and reliably measuring anxiety, pain and/or a modified state of consciousness is important in a medical setting as it for example helps to determine an accurate diagnosis, a treatment plan, and evaluate the effectiveness of treatment. Additionally, there is a growing problem of treatment overdosing, especially in the context of pharmacological anxiety and pain treatments, such as for example anxiolytic and/or analgesic overdosing. There is also a need, when for example modifying a level of consciousness pharmacologically, for example by means of a sedative, to avoid any overdosing and/or underdosing in order to achieve the desired level of modified state of consciousness while minimizing any adverse events related to overdosing and/or underdosing. Similarly, also in non-pharmacological treatments, care workers are in need of more reliable tools in order to provide for a safe and effective measurement of anxiety, pain and a level of modified state of consciousness. It is further clear that anxiety and pain and the treatment thereof have an impact on medical outcomes and patient recovery. Anxiety has an impact on health-related effects such as for example fatigue, nausea, insomnia, pain, for example as experienced by a subject before, during and/or after a treatment, such as for example a treatment including surgery and/or any other suitable medical and/or non-medical treatment. Pain measurement can be used to screen for potential diseases or medical problems, to perform a triage by determining the urgency of the needs of one patient over the other, can be used to determine whether it is safe to engage and/or continue with a medical treatment, etc..

There thus exists a need for an improved, robust, reliable and efficient measurement of the level of anxiety as experienced by a subject, the level of pain as perceived by a subject and/or a modified state of consciousness of a subject.

**Summary**

According to a first aspect, there is provided a computer-implemented method for measurement of a level of anxiety combined with and/or correlated with a level of a modified state of consciousness, and/or a level of pain, wherein the method comprises the steps of:

- receiving measured data comprising EEG data, collected from at least one of:

  - at least one parietal (P) electrode configured for collection of parietal EEG (P-EEG) data from the scalp anatomical region corresponding to a parietal lobe of a subject;
- at least one frontal (F) electrode configured for collection of frontal EEG (F-EEG) data from the scalp anatomical region corresponding to a frontal lobe of a subject,

and optionally, electromyography, EMG, data; and
- extracting from the EEG data collected from the P-EEG data a group 8 indicator based on a power, P-power (dt), associated with a delta-theta frequency band, dt, within a delta-theta frequency range; combined with:

  - extracting from the EEG data collected from the F-EEG data a group 1 indicator based on, a power, F-power(dt), associated with a delta-theta frequency band, dt, within a delta-theta frequency range;

    - and, optionally, from the EEG data collected from the F-EEG data, a group 2 indicator based on a mean signal peak-to-peak amplitude, F-MSPA;

  and/or
  - extracting from the EEG data, a group 4 indicator corresponding to a power, power (ta), associated with a theta-alpha frequency band, ta, within a theta-alpha frequency range;

    - and, optionally, from the EEG data, a group 3 indicator corresponding to a mean signal peak-to-peak amplitude, MSPA;
    - and, optionally, from the EMG data, a group 5 indicator corresponding to a mean signal peak-to-peak amplitude, EMG-MSPA; and

- determining, based on said group 8 indicator, a level of anxiety, LoA, which is a value indicative for the level of anxiety in the subject; combined with:

  - determining, based on said group 1 indicator, and optionally said group 2 indicator, a depth of state, DoS, which is a value indicative for the level of a modified state of consciousness in the subject; and/or
  - determining, based on said group 4 indicator, and optionally said group 3 indicator and/or group 5 indicator, a level of pain, LoP, which is a value indicative for the level of pain in the subject.

Measuring the LoA combined with DoS, the LoA combined with LoP or the LoA, LoP and DoS all three in combination, it for example becomes possible to identify how the LoA influences the LoP or vice versa, how the LoA is modulated by a modified state of consciousness, how the ability to reach a particular modified state of consciousness is influenced by the LoA and/or the LoP, etc.. By measuring the LoA, the LoP, the DoS in any suitable combination of at least two of these parameters, in this improved way, this enables an automated, efficient, accurate, reproducible, and objectivized determination and/or monitoring of the level of anxiety as experienced by the subject, the level of pain as perceived by the subject and the level of modified state of consciousness. As will be explained in further detail below measuring the LoA, LoP and/or DoS in a combined way is specifically advantageous as for example, a part of the LoA could be triggered by the LoP. As will be explained in further detail below there could be measured and/or determined interactions and/or correlations between the LoA, LoP and/or DoS, which for example allow to reduce the LoA by reducing the LoP, or are the cause that an increase in the LoA leads to an increases in the LoP as perceived by a subject, or which for example enable by a modification of the state of consciousness to reduce both LoP and LoA, or lead to the fact that a high level of anxiety can have a negative impact on a treatment aiming at modifying the level of consciousness, .... It is further clear that in addition to the embodiments described, there are further alternative suitable embodiment for correlating a plurality of LoA, LoP and/or DoS measurements and/or determining a correlation between a plurality of LoA, LoP and/or DoS measurements.
As will be explained in further detail below, with respect to P-power(dt) for determining the LoA, there was only found a specific correlation for this specific combination of brain wave frequencies of data that was collected at this specific location with an EEG electrode. Further, for both power(ta) for determining the LoP and P-power(dt) for determining the LoA, calculating the power from this data, which can be collected from a single EEG electrode, can be accomplished in a simple and efficient manner.
This improved, more robust and reliable measurement of LoA, LoP and DoS helps in determining a more accurate diagnosis, an improved and safer treatment plan, and an improved evaluation of the effectiveness of treatment. As a clear allocation can be made to the impact of anxiety, pain, and a modified state of consciousness, there the risk of administering a pain treatment when an anxiety treatment is needed or vice versa is reduced. Also the risk for overdosing and/or underdosing is reduced, for example as this measurement allows to make a clear decision on whether to use anxiolytics to treat anxiety and/or to use analgesics to treat pain and/or to use sedatives to modify the state of consciousness, and to optimize their dosing in function of the measured combination of LoA, LoP and/or DoS. It is however clear that such a measurement is not limited to optimization of pharmacological treatments, but is also useful when optimizing non-pharmacological treatments, and/or any combination of pharmacological and/or non-pharmacological treatments directed at treating anxiety, pain, and/or modifying a state of consciousness, or any other symptom related to anxiety, pain, and/or a specific state of consciousness, or any other suitable treatment.

It is thus clear that such improved measurement of a combination of at least two of LoA, LoP and DoS of a subject is beneficial when under any non-pharmacological and/or pharmacological treatment and/or mixed treatment, such as for example anxiety treatments, pain treatments, treatments leading to a modified state of consciousness, ..., and/or even when not under any treatment, in order to measure the state of a subject. It is clear that treatments modifying the state of consciousness of the subject, for example pharmacologically by means of sedatives, or non-pharmacologically by treatments adjusting the level of dissociation, the level of absorption, etc. of a subject, impact the level of anxiety as experienced by the subject and/or the level of pain as perceived by the subject. It is further clear that pain triggers anxiety and that higher levels of anxiety lead to higher levels of pain as perceived by a subject, and measurement of each of these parameters in combination thus allows to measure and display these interactions. In this way measurement of at least two of LoA, DoS and LoP, allow to objectively measure and display such interactions, and for example allows for such measurements to be used for the creation of and/or to be based on individual and/or population-based benchmarks. This will allow to make more reliable objective decisions, especially in the context of anxiety and pain management, particularly when there are involved pharmacological and/or non-pharmacological treatments that modify the state of consciousness managing pain and/or anxiety indirectly, and/or in combination with pharmacological pain and/or anxiety treatments.

It should be clear that according to some embodiments, this method is limited to merely obtaining information, such as data and physical quantities from the living human body and is not directed to finding any significant deviation, i.e. a symptom during a comparison of the collected data with standard values and is not directed to the attribution of such a deviation to a particular clinical picture, i.e. the deductive medical decision phase. Preferably, according to some embodiments, the method is performed on measured data, received, after it has been collected, or in other words, the method excludes a step of collection of data practiced on the human body. According to some embodiments the computer-implemented method only concerns obtaining information, for measurement of the level of anxiety as experienced by a subject, which is a psychophysiological quality of the subject, for the measurement of the level of pain as perceived by the subject and/or for the measurement of a level of modified state of consciousness. According to some embodiments, the computer-implemented method excludes any steps for curing of a disease or malfunction of the body, nor any step relating to a therapeutic effect on the body of the subject.

It is clear that the level of anxiety as measured comprises at least a level of anxiety experienced by a subject as measured at the level of the cortex of the brain of the subject or in other words comprising a cognitive dimension. Anxiety, being a response involving a psychophysiological and emotional aspect, clearly involves such a cognitive dimension. It has been shown based on the experiments above that anxiety as measured above is able to capture this cognitive dimension and is not limited to measurement of only non-cognitive parameters. Anxiety is for example associated with increased activity in the amygdala, and the activity of the amygdala can be influenced by activity in the higher brain structure, for example including the anterior cingulate cortex and the pre-frontal cortex responsible for focused attention. It is thus clear that anxiety as experienced by a subject comprises a cognitive dimension, optionally in addition to any other suitable dimensions.

It is clear that the level of pain relates to the perceived level of pain, or in other words the consciously perceived level of pain by a subject as measured at the level of the cortex of the brain of the subject. The level of pain thus corresponds to the consciously perceived level of pain perception of the subject. The level of pain as perceived by the subject is thus not directly correlated to the intensity of the external pain stimulus itself, but relates to the level of pain perception. In other words, the perception of the level of pain as it appears in the consciousness of the subject, typically associated with the cortex, after potential modulation and/or suppression by for example the thalamus and/or other downstream and/or upstream neural pathways.

This for example allows to dose local and/or IV medication to improve and optimize an anxiety, pain or sedative treatment. If a patient is doing fine during a treatment, and at one point there is measured an increase or a peak in the level of anxiety, level of pain or an undesired change to a modified state of consciousness, a local anesthetic, and/or IV analgesics dosages could be increased based on a combination of an objectivized DoS, a LoP as perceived by the subject, a LoA as experienced by the subject and not on a subjective estimation of patient's state of consciousness, pain and anxiety, for example by an anesthesiologist before, during and after a surgical treatment. Measurement of any combination of DoS, LoP and/or LoA also allows for an anxiety and/or a pain diagnostic for non-verbal patients (children, adults who are disabled, patients with dementia, ...). The measured combination of any of DoS, LoP and/or LoA is for example also a good indicator of the need or not for more sedation and/or analgesics and/or anxiolytics. In this way, a safe and reliable dose or titration of sedatives and/or analgesics and/or anxiolytics can be realized, with a reduced risk of overdosing and/or negative collateral effects of the analgesic or sedative or anxiolytic treatment.

Such an improved measurement of the LoA combined with DoS and/or LoP is for example advantageous in the following specific clinical cases:

- A patient is undergoing a procedure with light sedation. Based on the measured LoA, DoS and/or LoP, the Anesthesiologist will be able to objectively monitor LoA, DoS and/or LoP to adapt sedation if needed. As example, increasing IV sedation dosages could be replaced by increasing local anesthesia dosage as anesthesiologist will be able to monitor if local anesthesia is enough to manage patient's pain perception and/or when the patients level of anxiety permits it. As a further example, based on these measurements it could be determined whether in addition to and/or instead of pharmacological treatments there could be provided non-pharmacological treatments, which for example influence the patient's pain perception by means of modifying the patient's level of anxiety and/or state of consciousness.

- Limitation of analgesics, sedatives, anxiolytics, ... usage will be possible if the patient does not need it. A higher level of anxiety is associated with a higher level of pain as perceived by a subject. By measuring the LoA in combination with the LoP, it can be determined whether a high LoP is associated with a high LoA, and it can then for example be decided in such a situation that, instead of increasing the use of analgesics, it is more beneficial to make use of an anxiety treatment, such as for example by means of anxiolytics or any suitable non-pharmacological anxiety treatment to first reduce the LoA, to a desired level so that the anxiety related part of the LoP as perceived by the subject can be reduced and the use of analgesics can be limited. Additionally, the measured LoA combined with DoS and/or LoP allows to limit titration of sedation when, by for example ensuring that before any sedation is administered the LoA is reduced below a predetermined threshold, as unnecessary high LoA might prevent or hinder the subject from easily reaching a desired modified state of consciousness. Further, for example after first reducing the LoA below a desired threshold, it will allow to measure the DoS and/or LoP without the impact of an undesirably high LoA, thereby providing for a measurement of DoS and/or LoP which might reduce the risk of the use of an unnecessarily high dosage of sedation and/or analgesics. On the other hand, if DoS for example indicates that the patient is sufficiently sedated, but LoA indicates that still an unacceptable level of anxiety is experienced by the subject and/or LoP indicates that still an unacceptable level of pain is being perceived by the subject, then it can be decided not to increase the use of sedatives, thereby preventing overdosing, but to make increased use of for example a pharmacological and/or non-pharmacological anxiety treatment and/or pain treatment, such as for example by means of anxiolytics and/or analgesics.

- Objective Measure of efficacy of non-pharmacological anxiety management treatments (such as clinical hypnosis, digital sedation, acupuncture, psychological treatments, mind-body treatments, ...) during anxious moments of a treatment, especially when such treatments make use of non-pharmacologically induced modified state of consciousness as currently there are no prior art methods that reliably and objectively measure the level of anxiety combined with the state of consciousness and/or the level of pain as perceived by the subject for such non-pharmacological methods.

- Non-verbal patients (palliative, injured...) will be able to undergo a procedure with an objective monitoring of the DoS, LoA experienced by the patient and/or LoP as perceived by the patient. Better sedation, anxiety and pain management will be possible.

According to an embodiment the method comprises the steps of:

- determining a plurality of LoA at different DoS from measurement data and/or measurement data comprising simultaneous measurement of LoA and DoS;
- determining the modulation of LoA by DoS, by evaluating the evolution of the LoA at different DoS and/or the evolution of the LoA with respect to the evolution of DoS from the measurement data.

In this way the modulation of the LoA by DoS can be determined. This is useful as, as will be described in further detail anxiety is inversely correlated with a modified state of consciousness or in other words a depth of state, such as for example corresponding to a depth of dissociation of the subject, a level of absorption of the subject, etc. This for example means that the LoA could be decreased by modifying the level of consciousness. It is further beneficial as, as a higher level of anxiety experienced before a treatment session can be associated with a lower level of dissociation during a treatment session. In this way for patients which experience a high level of anxiety, for example prior to a treatment involving a modified state of consciousness, there could now be determined more objectively if they could benefit from an anxiety treatment before initiation of the treatment involving a modified state of consciousness. It is clear that alternative embodiments are possible in which a particular treatment is first replaced and/or supplemented by another treatment based on the modulation of the LoA by DoS and/or vice versa.

A modified level of consciousness associated with a deeper depth of state, such as for example a deeper depth of dissociation, a higher level of absorption, etc. as further detailed seems to decrease the level of anxiety experienced by a subject. In this way, measurement of the LoA combined with DoS might enable to determine which part of anxiety could be treated by modifying the state of consciousness of the subject, for example modifying the DoS, such as for example modifying the level of absorption, depth of dissociation, ... and additionally it might allow to differentiate between immediate and long-term effect of such changes to the depth of state to reduce anxiety, such as for example when used non-pharmacological treatments such as for example in psychotherapies, ...

According to an embodiment the method comprises the steps of:

- determining a plurality of LoP at different DoS and/or LoA from measurement data and/or measurement data comprising simultaneous measurement of LoA and DoS;
- determining the modulation of LoP by DoS and/or LoA, by evaluating the evolution of the LoP at different DoS and/or LoA and/or the evolution of the LoP with respect to the evolution of DoS and/or LoA from the measurement data.

In this way, it might be possible to determine how LoP is modulated by DoS and/or it may be possible to determine how LoP is modulated by LoA and/or vice versa. This is useful as an increased level of anxiety before, during and/or after a treatment session might increase the level of pain as perceived by a subject and can even trigger pain perception. This is important as for example anxiety plays a role in chronic pain. Based on the embodiment above, it would become for example possible to determine patients with a high LoA that may benefit from an anxiety reducing treatment to reduce LoP. Further anxiety is a risk factor for developing persistent/chronic pain following acute pain, and the embodiment above could thus for example be useful for determining a high risk group with respect to development of chronic pain and/or to reduce the risk of developing chronic pain. Further, also the LoP has an impact on the LoA as pain is an important anxiety inducing event..

According to an embodiment the method comprises the further step of:

- adjusting said LoA based on said DoS to a level of normalized anxiety, NLoA, which is a value indicative for the level of anxiety in the subject at a predetermined reference DoS; and/or
- adjusting said LoA based on said LoP to a level of normalized anxiety, NLoA, which is a value indicative for the level of anxiety in the subject at a predetermined reference LoP.

In this way the value for NLoA would provide for a value of LoA at a predetermined reference DoS and/or LoP of interest, independently of the current DoS / LoP and/or any changes to the current DoS / LoP, thereby for example providing an indicator for the equivalent amount of anxiety that would be experienced when the subject would be at, or return to a normal awake state of consciousness and/or a painless state, when this reference DoS is the normal awake state and/or the reference LoP corresponds to a painless state.

According to an embodiment the method comprises the further step of:

- adjusting said LoP based on said DoS and/or LoA to a level of normalized pain, NLoP, which is a value indicative for the level of pain in the subject at a predetermined reference DoS and/or a predetermined reference LoA;

In this way the value for NLoP would provide for a value of LoP at a predetermined reference DoS and/or LoA of interest, independently of the current DoS / LoA and/or any changes to the current DoS / LoA, thereby for example providing an indicator for the equivalent amount of pain that would be consciously perceived when the subject would be, or return to a normal awake state of consciousness and/or in a state without anxiety, when this reference DoS is the normal awake state and/or the reference LoA is a state without anxiety.

According to an embodiment the method comprises the further step of:

- calculating, based on said LoA and/or said LoP, at least one limit and/or range for said DoS, DoSLimit, which is indicative for the at least one limit and/or range of the modification of the level of consciousness attainable when the subject experiences said LoA and/or said LoP; and/or
- calculating, based on said LoP, at least one limit and/or range for said LoA, LoALimit, which is indicative for the at least one limit and/or range of the level of anxiety attainable when the subject experiences said LoP.

In this way, it is possible to determine, when for example the LoA and/or the LoP are too high to reach a desired modified state of consciousness, for example by means of a non-pharmacological method involving for example a suitable depth of dissociation, level of absorption, etc.

According to an embodiment the method comprises the further step of:

- calculating, based on said LoA, and/or DoS at least one limit and/or range for said LoP, LoPLimit, which is indicative for the at least one limit and/or range of the level of pain attainable when the subject experiences said LoA and/or DoS.

This is advantageous as when for example LoP is too high, anxiety becomes difficult to manage, and when LoA is too high pain becomes difficult to manage and might peak naturally. In such cases anxiety and/or pain treatments might experience difficulty in managing the LoA and/or LoP to desired levels. According to a further example when LoA and/or LoP are too high, it might be difficult to reach a desired modified level of consciousness, and for example no, insufficient, or too limited depth of state might be reached.

According to a second aspect there is provided a computer-implemented method for measuring and/or monitoring and/or predicting and/or aggregating a level of anxiety combined with and/or correlated with a level of a modified state of consciousness and/or a level of pain in a subject, preferably before, during and/or after a treatment, wherein the method comprises the steps of:

-    measurement of the LoA combined with the DoS and/or the LoP according to the first aspect at at least two different points in time, preferably before, during and/or after the treatment; and
-    measuring and/or monitoring and/or predicting an evolution, preferably a treatment-induced evolution, of the LoA combined with the DoS and/or the LoP, based on a respective comparison of at least two LoA combined with and/or correlated with at least two DoS and/or at least two LoP measured at at least two different points in time; and/or
-    aggregating a level of anxiety score or LoAS and/or a Depth of State Score or DoSS and/or a level of pain score or LoPS based on an aggregation of at least two LoA and/or at least two DoS and/or at least two LoP measured at at least two different points in time and/or over a period of time.

It is clear that the LoA, DoS and/or LoP only need to be measured respectively at at least two different points in time, and thus the LoA / DoS / LoP must not necessarily be measured concurrently.

In this way a measurement of an evolution of LoA combined with DoS and/or LoP is realized.

According to an embodiment, the method comprises the step of:

-    determining the treatment-induced evolution of the LoA combined with DoS and/or LoP, based on a respective comparison of the LoA combined with DoS and/or LoP determined during and/or after a treatment, with the LoA combined with DoS and/or LoP determined before a treatment.

In this way the measurement of a treatment-induced evolution becomes possible. Similarly, as mentioned above, although concurrent measurements of LoA, DoS and/or LoP are possible according to some embodiments, it is clear that alternative embodiments are possible in which LoA, DoS and/or LoP are not necessarily measured concurrently.

According to an embodiment:

-    the treatment is a pain management treatment and the method comprises the step of:

    -    replacing and/or combining the pain management treatment with an anxiety management treatment when the LoP and/or LoA is outside a predetermined range and/or until the LoP and/or the LoA is inside the predetermined range;

-    the treatment is a treatment modifying the state of consciousness and the method comprises the step of:

    -    replacing and/or combining the treatment modifying the state of consciousness with an anxiety and/or pain reducing treatment when the LoP and/or LoA is outside a predetermined desired range and/or until the LoP and/or the LoA is inside the predetermined desired range; and/or

-    the treatment is an anxiety management treatment and the method comprises the step of:

    -    replacing and/or combining the anxiety management treatment with a pain management treatment when the LoP and/or the LoA is outside a predetermined range and/or until the LoP and/or the LoA is inside the predetermined range;

-    the treatment is an anxiety management treatment and the method comprises the step of:

    -    replacing and/or combining the anxiety management treatment with a treatment modifying the level of consciousness when the DoS and/or LoA is outside a predetermined range and/or until the DoS and/or the LoA is inside the predetermined range.

It is clear that the predetermined range could be defined by one or more suitable limit values, and/or one or more desired ranges, thresholds, limits, ... which define the predetermined range for the LoP and/or LoA and/or DoS , as the range in which an anxiety management treatment, a pain management treatment and/or treatment modifying the state of consciousness still remains effective. The range could be defined in such a way that outside this range an anxiety management treatment, a pain-reducing treatment and/or treatment modifying the state of consciousness is no longer effective or is too difficult to manage. It is clear that the predetermined range could, according to some embodiments, be automated, dynamic, adaptive, etc. for example according to some embodiments be referred to as a form of auto-ranging, which means that the predetermined range is adapted based on for example earlier measured values of a subject (such as for example a reference range measured during a calibration phase, etc.) or population (such as for example predetermined ranges based on population characteristics, etc.).

According to an embodiment, the treatment is an anxiety-reducing treatment and the method comprises the step of:

- determining and/or monitoring the efficacy of an anxiety-reducing treatment based on the treatment-induced evolution of the NLoA.

According to an embodiment the anxiety treatment could for example be an anxiety-reducing treatment, or alternatively an anxiety-controlling or anxiety-preventing treatment that controls or prevents any undesired levels of anxiety, or any other suitable anxiety treatment.

According to an embodiment the pain treatment could for example be a pain-reducing treatment, or alternatively a pain-controlling or pain-preventing treatment that controls or prevents any undesired levels of pain, or any other suitable pain treatment.

According to an embodiment the treatment is a treatment modifying the state of consciousness for reducing anxiety and the method comprises the steps of:

- measuring, the DoS at at least two different points in time, before, during and/or after the treatment modifying the state of consciousness; and/or
- determining the treatment-induced evolution of the DoS and/or the LoA, based on a comparison of the DoS and/or the LoA measured during and/or after the treatment modifying the state of consciousness with the DoS and/or the LoA measured before the treatment modifying the state of consciousness.

According to a third aspect there is provided a computer-implemented method for determining a treatment and/or determining an adjustment to a treatment and/or determining an intensity of a treatment and/or determining the efficacy of a treatment and/or determining the choice of a treatment, wherein the method comprises the steps of:

- measuring and/or monitoring and/or predicting and/or aggregating a NLoA and/or NLoP and/or DoSLimit and/or LoPLimit and/or LoALimit in a subject according to an above-mentioned embodiment; and
- determining a treatment and/or determining an adjustment to a treatment and/or determining an intensity of a treatment and/or determining the efficacy of a treatment and/or determining the choice of a treatment based on the measured and/or monitored and/or predicted and/or aggregated NLoA and/or NLoP and/or DoSLimit and/or LoPLimit and/or LoALimit.

According to an embodiment the method comprises the steps of:

- receiving reference data comprising measured data of a subject and/or a population correlated to at least one predetermined reference LoA, combined with DoS and/or LoP respectively;
- determining at least one correlation of at least one predetermined reference LoA, combined with DoS and/or LoP respectively and at least one respective group indicator extracted from the reference data; and
- scaling and/or indexing a subsequently measured LoA, combined with DoS and/or LoP with respect to the at least one predetermined reference LoA, combined with DoS and/or LoP respectively by means of said at least one respective correlation, and optionally determining a level of anxiety index or LoAI, and a Depth of State index or DoSI and/or a level of pain index or LoPI therefrom.

According to an embodiment the method comprises the step of:

- receiving the measured data of the subject comprising the electroencephalogram, EEG, data collected from one or more of the following EEG electrodes:

    - the frontal (F) EEG electrode configured for collection of F-EEG electrode data from the scalp anatomical region corresponding to a frontal lobe of the subject,
- the parietal (P) EEG electrode configured for collection of P-EEG electrode data from the scalp anatomical region corresponding to a parietal lobe of the subject,
- a central (C) EEG electrode configured for collection of C-EEG electrode data from the scalp anatomical region corresponding to a precentral and postcentral gyrus of the subject.

It is clear that further alternative embodiments are possible in which in addition to the EEG electrodes, there is also present a ground electrode and/or a reference electrode. Preferably the data from the EEG electrodes can be processed independently, which means that there is no need to provide for calculations on the interaction and/or interrelation of the signals of two or more EEG electrode.

According to an embodiment the method comprises the steps of:

- receiving reference data comprising measured data of a subject and/or a population correlated to at least one predetermined reference LoA, combined with DoS and/or LoP respectively;
- determining at least one correlation of at least one predetermined reference LoA, combined with DoS and/or LoP respectively and at least one respective group indicator extracted from the reference data; and
- scaling and/or indexing a subsequently measured LoA, combined with DoS and/or LoP with respect to the at least one predetermined reference LoA, combined with DoS and/or LoP respectively by means of said at least one respective correlation, and optionally determining a level of anxiety index or LoAI, and a Depth of State index or DoSI and/or a level of pain index or LoPI therefrom.

A scaled and/or indexed LoA may for example be referred to as a level of anxiety index or LoAI.
A scaled and/or indexed LoP may for example be referred to as a level of pain index or LoPI.
A scaled and/or indexed DoS may for example be referred to as a Depth of State index or DoSI.

According to a fourth aspect of the invention, there is provided a computer-implemented method for measuring and/or monitoring and/or predicting an evolution and/or aggregating a level of anxiety combined with and/or correlated with a level of pain, and/or a level of modified state of consciousness of a subject, comprising the method for measurement of a level of anxiety combined with and/or correlated with a level of modified state of consciousness and a level of pain according to the first aspect of the invention applied on a subject by receiving the measured data of a subject.

The measured data of the subject could for example be real-time data as being received from suitable sensors, however, alternative embodiments are possible in which historic data, previously stored data, ... and/or any combination thereof is used for measurement of a level of anxiety, a level of pain and/or a level of modified state of consciousness.

It is clear that according to particular embodiments, the treatment could for example comprise an anxiety treatment or a pain treatment, such as for example an anxiety-reducing treatment or a pain-reducing treatment. However it is clear that alternative embodiments of treatments are possible, such as for example a surgical treatment or any other medical intervention during which one or more anxiety or pain inducing acts are performed, such as for example a needle puncture, placement of a port of a catheter, an incision, introduction of a wire, etc.. During some of these treatments the desired treatment-induced evolution will comprise a reduction of the LoA or LoP, however in alternative treatments, it might be more important to monitor the LoA or LoP for any undesired changes during the treatment, such that for example the need for extra anxiety or pain management measures could be reliably assessed. Similarly, a desired treatment induced evolution of DoS might comprise a desired change to the DoS by the treatment, or the prevention of an undesired change to the DoS by the treatment.

According to an embodiment the theta-alpha frequency band, ta, comprises one or more of the following:

- a frequency band encompassing both theta and alpha brain waves;
- a frequency band extending from 6Hz up to and including 12Hz; and/or
- a bandwidth of at least 2Hz and a frequency band comprising at least fast theta waves extending from 6Hz up to and including 8Hz, and/or

the theta-alpha frequency range:

- comprises a frequency range encompassing both theta and alpha brain waves; and/or
- extends from 4 Hz up to and including 12 Hz, and/or

the delta-theta (dt) frequency band, dt, comprises one or more of the following:

- a frequencies band encompassing both delta and theta brain waves;
- a frequency band extending from 0 Hz up to and including 8 Hz.

- a bandwidth of at least 2 Hz and a frequency band comprising at least slow theta brain wave extending from 3 Hz up to and including 6 Hz; and/or

the delta-theta frequency range:

- comprises a frequency range encompassing both delta and alpha brain waves; and/or
- extends from 1 Hz up to and including 6 Hz.

According to an advantageous embodiment the delta-theta frequency band consists of a combination of:

- at least part of a delta frequency band; and
- at least part of a theta frequency band.

According to an advantageous embodiment the delta-theta frequency band consists of a combination of at least part of a delta frequency band and at least part of a theta frequency band.
In other words, the delta-theta (dt) frequency band comprises, and preferably consists of a combination of at least part of the delta frequency band and at least part of the theta frequency band.
It is clear that such a delta-theta frequency band comprising and/or consisting of a combination of two brain wave frequencies, or at least a part thereof, is different from a frequency band only comprising a single brain wave frequency band and different from a frequency band comprising more than two brain wave frequency bands.
It has been found that such a combined frequency band, consisting of two specific brain frequency bands, surprisingly provides for a better correlation with the level of anxiety, than each of the individual brain frequency bands, for data collected from the specific location of a P-EEG electrode.
According to an advantageous embodiment the delta-theta frequency band consists of a combination of:

- at least part of a delta frequency band; and
- at least part of a theta frequency band.

According to an advantageous embodiment the theta-alpha frequency band consists of a combination of at least part of a theta frequency band and at least part of an alpha frequency band.
In other words, the theta-alpha (ta) frequency band comprises, and preferably consists of a combination of at least part of the theta frequency band and at least part of the alpha frequency band.
It is clear that such a theta-alpha frequency band comprising and/or consisting of a combination of two brain wave frequencies, or at least a part thereof, is different from a frequency band only comprising a single brain wave frequency band and different from a frequency band comprising more than two brain wave frequency bands.
It has been found that such a combined frequency band, consisting of two specific brain frequency bands, surprisingly provides for a better correlation with the level of pain, than each of the individual brain frequency bands, for EEG data collected from a suitable electrode.
According to an embodiment, the method comprises steps of:

- receiving measured data further comprising cardiovascular data
- extracting from the cardiovascular data:

  - a group 7 indicator based on a heart rate and/or heart rate variability, preferably a combination of heart rate and heart rate variability, from the cardiovascular data; and

- determining the LoA based on said group 8 indicator and at least the group 7 indicator; and/or

According to such embodiments in which the level of anxiety is measured by means of a combination of a specific signal extracted from EEG data, which is more directly linked to the cognitive dimension of anxiety, and one or more specific signals like heart rate, heart rate variability, breathing frequency, breathing variability, oxygen saturation, ... and/or other suitable specific embodiments of physiological parameters as mentioned below, which are more directly linked to the autonomic-emotional dimension provides for a specifically advantageous multidimensional measurement of the level of anxiety as experienced by a subject. Based on the specific signals extracted from both specific EEG data and at least one specific other signal from a specific physiological parameter thus allows for a measurement that takes into account the multi-dimensionality of anxiety, in which however the cognitive dimension as linked to the specific EEG data is essential in order to provide for a measurement that relates to the level of anxiety as experienced by the subject.
As will be explained in further detail below, a specific combination of heart rate and heart rate variability data, provides for a particularly advantageous and simple measurement with an improved correlation with the level of anxiety as experienced by a subject. However, it is clear that alternative embodiments are possible in which any other suitable cardiovascular data and/or combination thereof is used, such as for example cardiac activity data, hemodynamic activity data, ....

According to an embodiment, the method comprises steps of:

- receiving measured data further comprising respiratory data;
- extracting from the respiratory data:

  - a group 6 indicator based on respiration rate and/or a respiration variability and/or oxygen saturation and/or predetermined respiratory patterns from the respiratory data;

- determining the LoA based on said group 8 indicator and at least the group 6 indicator.

According to an embodiment, the method comprises steps of:

- receiving measured data further comprising

  - ocular movement data;

- extracting from the ocular movement data:

  - a group 9 indicator based on an ocular movement rate and/or ocular movement amplitude and/or predetermined ocular movement patterns from the ocular movement data;

- determining the LoA based on said group 8 indicator and at least the group 9 indicator.

According to an embodiment:

- the ocular movement data comprises EOG data; and/or
- the group 9 indicator is based on the detection of saccadic eye movements from the ocular movement data, and optionally one or more characteristics of the saccadic eye movements.

According to an embodiment, the method comprises steps of:

- receiving measured data further comprising

  - muscular activity data;

- extracting from the muscular activity data:

  - a group 10 indicator based on a muscular activity from the muscular activity data;

- determining the LoA based on said group 8 indicator and at least the group 10 indicator; and/or

According to an embodiment, the method comprises steps of:

- the muscular activity data comprises electromyography, EMG, data; and/or
- the group 10 indicator is based on an electromyographic, EMG, activity from the EMG data.

According to a preferred embodiment the EMG data is captured at two or more electrode locations such that global muscle tension can be determined and locally isolated artefacts can be detected and removed.

According to an embodiment, the method comprises steps of:

- receiving measured data further comprising

  - body temperature data;

- extracting from the ocular movement data:

    - a group 11 indicator based on a body temperature from the body temperature data;

- determining the LoA based on said group 8 indicator and at least the group 11 indicator.

According to such embodiments, the LoA is measured taken into account the multidimensional aspect of anxiety.

According to an embodiment, there is provided a method, wherein the treatment is an anxiety treatment and the method comprises the step of:

- determining and/or monitoring the efficacy of an anxiety treatment based on the treatment-induced evolution of the LoA combined with DoS and/or LoP.

The anxiety treatment could for example be an anxiety-reducing treatment, or alternatively an anxiety-controlling or anxiety-preventing treatment that controls or prevents any undesired levels of anxiety, or any other suitable anxiety treatment.

According to an embodiment, there is provided a method, wherein the treatment is an anxiety treatment and the method comprises the step of:

- optimizing and/or adapting an anxiety treatment based on the treatment-induced evolution of the LoA combined with DoS and/or LoP.

As already mentioned above the anxiety treatment could for example be an anxiety-reducing treatment, or alternatively an anxiety-controlling or anxiety-preventing treatment that controls or prevents any undesired levels of anxiety, or any other suitable anxiety treatment.

According to a fifth aspect of the invention, there is provided a computer-implemented method for determining a treatment and/or determining an adjustment to a treatment and/or determining an intensity of a treatment and/or determining the efficacy of a treatment and/or determining the choice of a treatment, wherein the method comprises the steps of:

- measuring a LoA combined with DoS and/or LoP according to the first aspect of the invention;
- determining a treatment and/or determining an adjustment to a treatment and/or determining an intensity of a treatment and/or determining the efficacy of a treatment and/or determining the choice of a treatment based on the measured of the LoA combined with DoS and/or LoP.

According to a sixth aspect of the invention, there is provided a system configured to measure a LoA combined with and/or correlated with a DoS and/or a LoP according to the method according to any previous aspect and/or embodiments, the system comprising:

- a monitoring apparatus configured to obtain measured data comprising EEG data comprising data, collected from at least one of:

    - at least one parietal (P) electrode configured for collection of P-EEG data from the scalp anatomical region corresponding to a parietal lobe of a subject;
    - at least one frontal (F) electrode configured for collection of F-EEG data from the scalp anatomical region corresponding to a frontal lobe of a subject,

- the measured data optionally further comprising electromyography, EMG, data; and
- a controller module configured for:

    - receiving the measured data from the monitoring apparatus;
    - extracting:

        - from the EEG data collected from the P-EEG data, a group 8 indicator based on a power, P-power (dt), associated with a delta-theta frequency band, dt, within a delta-theta frequency range; and

    - extracting:

- from the EEG data, a group 1 indicator based on, a power, F-power(dt), associated with a delta-theta frequency band, dt, within a delta-theta frequency range extracted from the at least one F- EEG electrode data;

  - and, optionally, from the EEG data collected from the F-EEG data, a group 2 indicator based on a mean signal peak-to-peak amplitude, F-MSPA;

  and/or
- from the EEG data, a group 4 indicator corresponding to a power, power(ta), associated with a theta-alpha frequency band, ta, within a theta-alpha frequency range;

  - and, optionally, from the EEG data, a group 3 indicator corresponding to a mean signal peak-to-peak amplitude, MSPA;
  - and, optionally, from the EMG data, a group 5 indicator corresponding to a mean signal peak-to-peak amplitude, EMG-MSPA;

- determining, based on said group 8 indicator, a level of anxiety, LoA, which is a value indicative for the level of anxiety in the subject; and
- determining:

  - based on said group 1 indicator, and optionally said group 2 indicator, a depth of state, DoS, which is a value indicative for the level of a modified state of consciousness; and/or
  - based on said group 4 indicator and optionally said group 3 indicator and optionally said group 5 indicator, a level of pain, LoP, which is a value indicative for the level of pain in the subject.

According to an embodiment, wherein the monitoring apparatus comprises, for obtaining measured data, one or more of:

- one or more electrodes configured for collection of electroencephalogram, EEG, data comprising:

  - one or more parietal (P) electrodes configured for collection of P-EEG data from the scalp anatomical region corresponding to a parietal lobe of the subject,
  - one or more frontal (F) electrodes configured for collection of F-EEG data from the scalp anatomical region corresponding to a frontal lobe of the subject,
  - optionally, one or more central (C) electrodes configured for collection of C-EEG data from the scalp anatomical region corresponding to a precentral and postcentral gyrus of the subject,
  - optionally, one or more further electrodes configured for collection of electroencephalogram, EEG, data;

- optionally a respiratory data capturing unit;
- optionally a cardiovascular data capturing unit;
- optionally an ocular movement data capturing unit;
- optionally a muscular activity data capturing unit;
- optionally a body temperature data capturing unit.

It is clear that still further alternative embodiments are possible comprising for example one or more sensors and/or capturing units for one or more other physiological measurements.
According to an embodiment, there is provided a system, further comprising a graphical user interface, GUI, configured to indicate numerically and/or graphically one or more of:

- at least two LoA, DoS and/or LoP respectively measured at different points in time, preferably a historic, current and/or expected LoA, DoS and/or LoP.
- the evolution of and/or ratio between and/or aggregation of at least two LoA, DoS, and/or LoP respectively measured at different points in time;
- optionally any (N)LoA(I)(S), (N)LoP(I)(S), and/or DoS(I)(S), LoALimit, LoPLimit and/or DoSlimit; and/or
- optionally, one or more components of the measured data, preferably one or more EEG data and optionally EMG data, respiratory data, cardiovascular data, muscular activity data, ocular movement data and/or body temperature data.

It is clear that in the context of this description measured at different points in time, could also refer to measured over a period of time.

It is clear that still further alternative embodiments are possible comprising for example one or more sensors and/or capturing units for one or more other electrophysiological and/or physiological measurements.

It is clear that alternative embodiments of the GUI are possible in which further data is displayed, such as for example ECG data, cardiovascular data, respiratory data, ocular movement data, EOG data, muscular activity data, EMG data, body temperature data, ....

It is clear that in the abovementioned embodiments of the method and/or systems any embodiments defined with reference to LoA, LoP and/or DoS, also refer to similar embodiments based on parameters derived from LoA, LoP and/or DoS, such as for example LoAI, LoPI, DoSI, LoA(I)S, LoP(I)S, etc..

**Brief description of the drawings**

FIG. 1 shows an embodiment of an experiment for measuring the level of anxiety as experienced by the subject and the level of pain as consciously perceived by the subject.

FIG. 2 is a graph indicating a correlation between self-reported anxiety and computed delta-theta power at P-EEG electrode.

FIG. 3 is a graph indicating a correlation between self-reported pain and computed theta-alpha power at EEG electrodes F, P and C.

FIG. 4 is a graph indicating a correlation between self-reported dissociation and computed delta-theta power at EEG electrodes F, P.

FIG. 5 shows an embodiment of a computer-implemented method for measuring the level of anxiety, the level of modified consciousness and/or the level of pain.

FIG. 6 shows an embodiment of a computer-implemented method for measuring and/or monitoring a level of anxiety, a level of modified consciousness and a level of pain in a subject before, during and/or after a treatment.

FIG. 7 - FIG. 9 illustrate exemplary embodiments of the use of NLoAI and NLoPI.

FIG. 10 shows an exemplary embodiment of a method in which a plurality of LoA and LoP are correlated.

FIG. 11 shows an exemplary embodiment of a method in which a plurality of LoA and DoS are correlated.

FIG. 12 shows an alternative embodiment of a method in which a plurality of LoA and LoP are correlated.

FIG. 13 shows an alternative embodiment of a method in which a plurality of LoA and DoS are correlated.

FIG. 14 shows an alternative embodiment of a method in which a plurality of LoA and LoP at different DoS are correlated.

FIG. 15 and FIG. 16 show alternative embodiments in which the impact of changes to DoS on LoA and LoP is illustrated.

FIG. 17 shows an exemplary visualization of a time domain response signal at a central EEG electrode during the experiment of FIG. 1.

FIG. 18 shows an exemplary visualization of a time domain response signal at a parietal EEG electrode during the experiment of FIG. 1.

FIG. 19 shows an exemplary visualization of a time domain response signal at a frontal EEG electrode during the experiment of FIG. 1.

FIG. 20 is a graph indicating the correlation between MSPA measured at the 3 EEG electrodes F, C and P and pain.

FIG. 21 is a graph indicating the correlation between MSPA measured at the EEG F-electrode and dissociation.

FIG. 22 is a graph indicating a correlation between self-reported pain and EMG-ERP amplitude.

FIG. 23 shows an exemplary visualization of a time domain response ECG signal and a heart rate and heart rate variability extracted therefrom.

FIG. 24 shows a heart rate variability for a subject with a higher level of anxiety than in FIG. 25.

FIG. 25 shows a heart rate variability for a subject with a lower level of anxiety than in FIG. 24.

FIG. 26 shows a box plot comparison of two embodiments of a group 7 indicator.

FIG. 27 shows correlation between self-reported anxiety and respiration rate.

FIG. 28 shows such correlation between the self-reported level of anxiety and respiration rate variability.

FIG. 29 shows an exemplary embodiment of time domain respiratory data, and Fig. 30 and FIG. 31 show such respiratory data for a subject at different levels of anxiety.

FIG. 32 and 33 show an exemplary embodiment of time domain ocular movement data for a subject at different levels of anxiety.

FIG. 34 shows an exemplary embodiment of time domain ocular movement data averaged for a plurality of subjects which is time-locked to a painful stimulus, at two different levels of anxiety.

FIG. 35 shows an exemplary embodiment of time domain muscular activity data of a subject at two different levels of anxiety.

FIG. 36 is a schematic illustration of Groups 1-11 indicators extracted from response data.

FIG. 37 shows a graph with a correlation between a level of anxiety and an embodiment of an indicator based on a combined heart rate and heart rate variability.

FIG. 38 to FIG. 45 illustrate examples of outputs to a graphical user interface.

FIG. 46 illustrates a wearable device incorporating an eye-mask incorporating a media renderer, electrodes and sensors.

FIG. 47 illustrates a rim of the eye-mask of the wearable device disposed with electrodes.

FIG. 48 illustrates rim of the eye-mask of the wearable device disposed with another configuration of electrodes and sensors.

FIG. 49 is similar to FIG. 46 devoid of a virtual reality viewer, headphones are present.

FIG. 50 is similar to FIG. 46 devoid of a virtual reality viewer and headphones.

FIG. 51 shows an exemplary visualization of an exemplary treatment session comprising four phases, as measured using DoSI and LoPI.

FIG. 52 - FIG. 54 illustrate further examples of outputs to a graphical user interface.

FIG. 55 illustrates a further embodiment of the NLoAI.

FIG. 56 schematically shows an embodiment of a recovery coefficient.

FIG. 57 shows a further embodiment of a GUI.

## Detailed description of exemplary embodiments

In order to generate experimental data, according to this particular embodiment, an experiment including a hypnotic treatment session was set up. However, it is clear that alternative embodiments are possible in which any other suitable treatment session is used and/or in which no use is made of a treatment session. This experiment resulted in a study with 12 healthy subjects, of which 10 were included in the subsequent analysis. The experiment comprised measurement of a level of anxiety, a level of pain, and a modified state of consciousness, by self-reporting of the subject using a validated questionnaire.

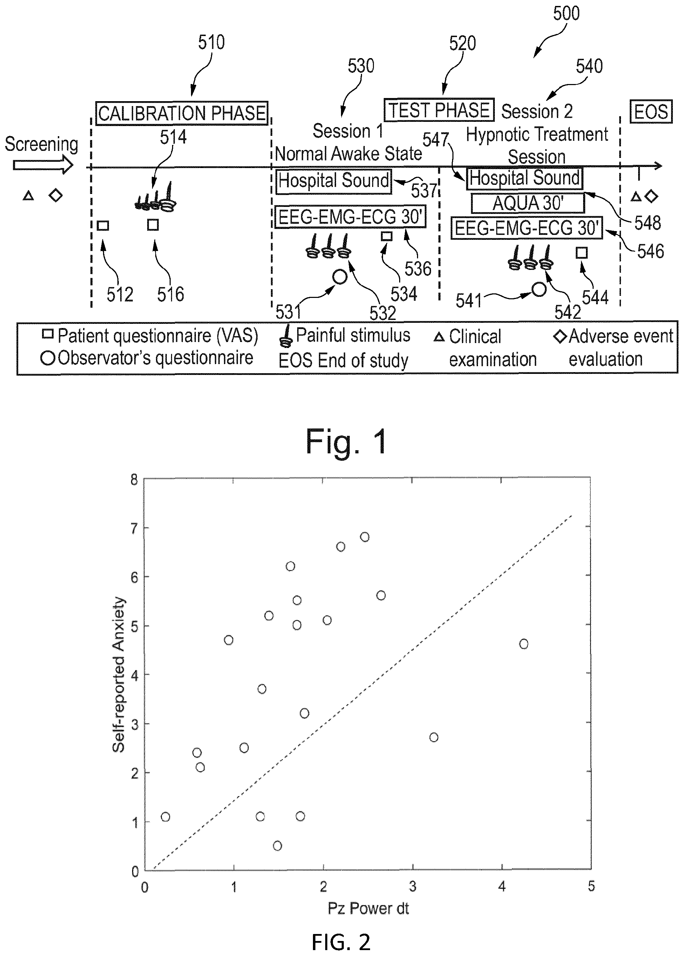

The setup of the embodiment of the experiment will be clarified by means of FIG. 1, which shows the different phases of the experiment. As shown, optionally prior to and after the different phases of the experiment clinical examination and an adverse event evaluation was performed as part of a screening and/or the end of study, EOS, in order to assure the safety of the subjects undergoing the experiment and to assure a high level of quality with respect to the self-reported data.

During an initial calibration phase 510, at a first step 512 the subjects were asked to provide self-reported values for rating parameters as explained in further detail below. Subsequently at step 514, by using a stair-case method, the subjects received a sequence of transcutaneous electrical stimulus, or in other words a pain stimulus, of increasing intensity, so that in step 516 the subject could indicate when the perceived level of pain corresponded to a level of pain with a value of 8 on a VAS pain scale. In other words, the intensity of the pain stimulus is calibrated for each subject individually using the stair-case method, by asking the subjects to indicate when they judged the perceived level of pain to corresponding to a value 8 on the pain scale, which corresponds to a level of pain qualified as "significantly painful and demanding some effort to tolerate". Such a pain scale for self-reporting of pain according to the embodiment of the experiment was determined by means of a Visual Analogue Scale or VAS, which is a measurement instrument that tries to measure a characteristic or attitude that is believed to range across a continuum of values and cannot easily be directly measured. According to this embodiment a VAS scale ranging from a value of 0 for "no pain" up to and including 10 for "severe or extreme amount of pain". Such instruments are often used in clinical research to measure the intensity of symptoms, such as for example, the level of pain as perceived by a patient. It is clear that alternative embodiments for self-reporting values are possible, such as for example the Numeric Rating Scale (NRS-11) is an 11-point scale for patient self-reporting of pain, which ranges from a value of 0 for "no pain" up to and including a value of 10 for "severe pain".

Similar as for the self-reported level of pain, at step 512, and 516 of the calibration phase 510, and as will be described in further detail below at further steps during the experiment, such as for example step 534 of the normal awake state 530 and step 544 of the hypnotic treatment session 540, patients were also asked to provide self-reported values for rating their experienced level of anxiety. According to this embodiment a VAS scale ranging from a value of 0 for "no anxiety" up to and including 10 for "severe or extreme amount of anxiety". It is clear that alternative embodiments for self-reporting values are possible.

As further schematically shown in FIG. 1, the calibration phase 510 was followed by a first phase 520, which was labeled as test phase 520 in Fig. 1, which comprises a first session 530 in a normal awake state of the subject. As schematically shown, during this first session 530, there were generated one or more pain stimuli, as calibrated during the previous calibration phase. As shown, according to this embodiment, there was for example generated a plurality, for example as shown in step 532 three pain stimuli that were qualified as 8 on the VAS pain scale during the calibration phase. In other words, according to this embodiment, the pain stimuli, when applied, provided for identical pain conditions, for example by means of transcutaneous electricals stimuli of identical intensity. It is however clear that alternative embodiments of the experiment are possible, in which a different number of pain stimuli and/or pain stimuli of other suitable intensities and/or varying intensities are applied. It is clear that according to this embodiment the calibrated pain stimuli 532, of a predetermined intensity, which were associated by the subject with a predetermined level of pain during the calibration phase 510, are applied at suitable times, with suitable intervals, during a time period of for example 30 minutes. However, it is clear that alternative embodiments of suitable time periods, during which the one or more pain stimuli 532, and/or any other suitable types of anxiety inducing stimuli are applied, are applied, are possible. During this time period, during which the pain stimuli 532, which also qualify as anxiety inducing stimuli, are applied, as shown at step 534, suitable sensors are configured to monitor and/or register physiological signals generated by the subject for provision to a suitable processor to generate measurement data corresponding to the state and/or activity of the body or bodily functions of the subject. Such physiological sensors provide an objective measure of physiological signals. Such physiological sensors provide for signals, that, when suitably processed, provide for measurement data based on recordings of electrical signals produced by the brain, the heart, the muscles, the skin, ... of the subject. Embodiments of such physiological sensors are for example sensors for measuring physiological signals configured to measure respiration, electrocardiography or ECG, electromyography or EMG, Blood Pressure or BP, Blood Volume Pressure or BVP, electrooculography or EOG, electroencephalography or EEG, .. Electroencephalography or EEG is an electrophysiological monitoring method to record electrical activity of the brain, and can be referred to as a cognitive, electrophysiological signal, and is related to corticoidal signals. Other physiological and/or electrophysiological signals can be referred to as autonomic physiological signals such as for example EMG, which measures muscle activity; EDA or skin conductance which measures the hydration in the epidermis and/or dermis of the skin; EKG or ECG, which measures heart activity, such as for example heart rate, inter-beat-interval, heart rate variability, ... ; Electrooculogram, which measures eye movements, BVP, which measures blood pressure; Respiration sensors, which measure for example the rate of respiration, depth of breath, effort, temperature, air pressure during inhalation and/or exhalation, .... According to the embodiment shown, electroencephalogram (EEG) data, cardiovascular data (heart rate, heart rate variability, ...), electromyogram (EMG) data, respiratory data (respiration rate, respiration variability, predetermined respiration patterns, effort, temperature, and air pressure (inhalation/exhalation)), electrooculography or EOG and body temperature, were collected as measured data from each subject, however it is clear that alternative embodiments are possible comprising the monitoring of alternative cognitive electrophysiological and/or autonomic physiological signals as for example described in further detail below.

Subsequent to the time period 536 during which the measurements of the physiological signals were made, the subject was asked to provide self-reported values for the level of anxiety, the level of pain, modified level of consciousness, etc. for example by means a suitable VAS patient questionnaire during step 534.

According to the embodiment of the experiment shown in FIG. 1, the test phase 520 subsequently comprises second session 540, which comprises an automated hypnotic treatment session 548 that was delivered using a system that comprises a virtual reality headset as will also be described in further detail below. However, it is clear that alternative embodiments are possible in which any other suitable treatment session is used and/or in which no use is made of a treatment session. The embodiment of the system, also referred to as a hypnotic package, is referred to as Sedakit, as manufactured by Oncomfort. The Sedakit was configured to the Aqua 30 minutes module embodiment of an automated hypnotic treatment session, which subjected the subject to an automatic hypnotic treatment session for a time period of 30 minutes at step 548. During this hypnotic treatment session 548, similar as described above, electroencephalogram (EEG), cardiovascular data, muscular activity data such as for example electromyogram or EMG data, respiratory data (respiration rate, respiration variability, predetermined respiration patterns, effort, temperature, and air pressure (inhalation/exhalation)), ocular movement data, such as for example electrooculography or EOG data and body temperature data, were collected as measured data from each subject, however it is clear that alternative embodiments are possible comprising the monitoring of alternative physiological signals. Also similar as described above, during the hypnotic treatment session 538, each subject was exposed to one or more transcutaneous painful stimuli, which also qualify as anxiety inducing events. For example, a sequence of a plurality of pain stimuli, such as for example shown as three painful stimuli 542. Similar as described above, according to this embodiment, the painful stimuli 542 that are applied during the hypnotic treatment session 538 were also qualified as 8 on the VAS pain scale during the calibration phase. In other words, according to this embodiment, the pain stimuli, when applied, provided for identical pain conditions, for example by means of transcutaneous electricals stimuli of identical intensity. It is however clear that alternative embodiments of the experiment are possible, in which a different number of pain stimuli and/or pain stimuli of other suitable intensities and/or varying intensities or any other suitable types of anxiety inducing stimuli are applied. It is clear that according to this embodiment the calibrated pain stimuli 532, of a predetermined intensity, which were associated by the subject with a predetermined level of pain during the calibration phase 510, are applied at suitable times, with suitable intervals, during a time period of for example 30 minutes. According to the embodiment of the experiment the external pain stimulus was provided in the form of an electric transcutaneous stimulus being provided at the fore-arm during the experiment, however it is clear that alternative embodiments of a suitable pain stimulus are possible. Similarly, as described above, at step 544, subsequently each subject was asked to rate the level of anxiety as they experienced it, the level of pain as they experienced it and the depth of dissociation, etc. during the hypnotic treatment session 548 by means of a patient questionnaire. Optionally, but preferably, during both session 1 530 in the normal awake state and session 2 540 with the hypnotic treatment session the subject is placed in a similar context. For example, in both sessions, preferably the subject is placed in a similar position, for example laying on chair. As further shown, for example the subject is experiencing similar sounds 537, 547, such as for example sounds of a hospital environment. Optionally, in addition to the patient questionnaire, there may be made use of an observer's questionnaire, for example assure the quality of the experiment and/or to provide for additional and/or alternative ratings for one or more of the self-reported ratings of pain, anxiety, dissociation, etc..

During the experiment, for the EEG measurements, EEG electrodes were used that were placed so as to cover the scalp and so as to detect local voltage fluctuations resulting from localized regions of the brain.

According to this embodiment, a high density EEG was employed with 256 sensors or channels, however it is clear that alternative embodiments with a different number of sensors are possible. According to the embodiment of the experiment, electrodes were placed according to the predefined layout for a 256-channel Hydrocel Geodesic Sensor Network. It is further known to a person skilled in the art that EEG electrodes mainly measure neural activity that occurs at the upper layers of the brain, or in other words the neural activity of the cortex of the brain. During the experiment, for the EMG measurements sensor, a pair of surface EMG sensors were placed on a leg of each patient to as to detect the electric potential generated by muscle cells when then muscles become activated. However it is clear that alternative embodiments are possible in which EMG sensors are provided at any other suitable location, such as for example the face, arm, .... It is however clear that according to alternative embodiments use can be made of other types of suitable EEG electrodes or sensors, EMG electrodes or sensors and/or any other suitable type of electrode or sensor for measuring EEG and/or EMG signals.

Below are the results of the above-mentioned experiment, according to the embodiment of FIG. 1. As mentioned above the experiment 500 comprises a test phase 520 with a "normal awake state" 530 and a "Hypnotic treatment session" for which both self-reported data with respect to the level of anxiety, the level of pain and a level of dissociation were collected in steps 538, 548 for each subject that was subjected to one or more calibrated pain stimuli, which also qualify as calibrated anxiety inducing events. The results of the experiment of FIG. 1 show measurable and statistically significant effect of the hypnotic treatment session 530 versus the normal awake state 530 regarding, the level of anxiety, more specifically a reduction of the level of anxiety; the level of pain, more specifically a pain reduction; and the level of dissociation, more specifically an increase in the level of dissociation as illustrated by means of Table 1 below. As will be described in further detail below the level of dissociation is an exemplary metric for quantifying a modified level of consciousness, such as for example resulting from a hypnotic treatment session, however it is clear that alternative embodiments are possible of a level of modified consciousness, such as for example a level of absorption, etc..

Table 1 below, shows a Wilcoxon signed-rank test of the self-reported data for subjects during the experiment of FIG. 1 during the normal awake state 530 and during the hypnotic treatment session 540:

| Self-reported data 534, 544 | Normal awake state 530 <br><br> *Mean ±SD* <br> *Median [IQR]* | Hypnotic treatment session 540 <br><br> *Mean ±SD* <br> *Median [IQR]* | p-value | Adjusted p-value (Bonferroni correction) |
|---|---|---|---|---|
| Self-reported level of Anxiety | 4.92 (1.74) <br> 5.55 [1.8] | 3.16 (1.77) <br> 3.1 [2.95] | 0.0004 | 0.0024 |
| Self-reported level of Pain | 4.21 (2.27) <br> 4.35 [2.22] | 1.39 (1.50) <br> 1.2 [1.65] | 0.0059 | 0.0296 |
| Self-reported level of Dissociation | 4.11 (3.13) <br> 4.95 [5.72] | 7.52 (1.43) <br> 8 [0.9] | 0.0032 | 0.0162 |

It should first be noted that, in reaction to the application of the pain stimuli during the normal awake state 530, the self-reported level of anxiety has increased with respect to the level of anxiety as reported before the application of the pain stimuli. It is thus clear that the pain stimuli thus also function as an anxiety inducing stimulus or an anxiety inducing event. This is confirmed by the measurement of a self-reported level of anxiety before the normal awake state 530, which had as mean +/- SD: 2,65 (2.06) and as Median [IQR]: 2.15 [3.55], with a p-value = 0.002 and adjusted p-value =0.008. In between the Normal awake state 530 and the Hypnotic treatment session 540, there was a pause or a break, and before the Hypnotic treatment session 540, also there was performed a measurement of the self-reported level of anxiety, which had as mean +/- SD: 2,79 (2.21) and as Median [IQR]: 2.65 [1.25], with a p-value = 0.53 and adjusted p-value = 1.

It is thus clear that, according to the embodiment described above, in which during both the normal awake state 530 and the hypnotic treatment session 540, pain stimuli of an identical intensity were applied, or in other words, identical pain conditions were experienced by the subjects during application of the pain stimuli of identical intensity, or in other words an identical anxiety inducing events and/or stimuli, the self-reported level of Anxiety is significantly different in both sessions 530, 540. As for example shown, the mean of the value for self-reported anxiety in the normal awake state 530 is 4.92, while in the hypnotic treatment session 540 it was reported to be 3.16. In other words, a reduction of more than 30% with respect to the normal awake state. It is thus clear that the self-reported level of anxiety corresponds to a level of anxiety as experienced by the subject, as clearly, the self-reported values for the level of anxiety triggered by an identical external pain stimulus are modulated by the modified state of consciousness caused by the hypnotic treatment session. In other words, the self-reported data for the level of anxiety are an indicator for the level of anxiety as experienced by the subject, or in other words the level of experienced anxiety. As the self-reported data relating to anxiety are triggered by the painful stimuli, it is clear that these painful stimuli can be considered as an anxiety inducing event, or in other words an adverse situation at a specific moment in time, and thus the self-reported data relates to the level of anxiety of the subject as experienced with respect to the painful, anxiety inducing stimuli. Further it should be noted that the experiment was set up with a first phase in the normal awake state 530 with increased levels of anxiety as compared to prior to the experiment, and still during the subsequent second phase in during the hypnotic treatment session 540, again reduced levels of anxiety are reported. A predetermined rest time between the normal awake state 530 and the hypnotic treatment session 540 was included.

It is further clear that, according to the embodiment described above, in which during both the normal awake state 530 and the hypnotic treatment session 540, pain stimuli of an identical intensity were applied, or in other words, identical pain conditions were experienced by the subjects during application of the pain stimuli of identical intensity, the self-reported level of pain are significantly different in both sessions 530, 540. As for example shown, the mean of the value for self-reported pain in the normal awake state 530 is 4.21, while in the hypnotic treatment session 540 it was reported to be 1.39. In other words, a reduction of more than 50% with respect to the normal awake state. It is thus clear that the self-reported level of pain corresponds to a level of pain as perceived by the subject, as clearly the self-reported values for the level of pain correlated to an identical external pain stimulus are modulated by the modified state of consciousness caused by the hypnotic treatment session. In other words, the self-reported data for the level of pain are an indicator for the level of pain as consciously perceived by the subject, or in other words the level of perceived pain. It is further clear that this level of perceived pain is such that it is subject to modulation by means of a modification or change of the level of consciousness of a subject, such as for example by means of a change to the level of dissociation of the subject.

The change to the level of dissociation of the subject by means of the hypnotic treatment session versus the normal awake state is confirmed by means of the self-reported data in Table 1, which shows a mean self-reported value for the level of dissociation of 7.52 during the hypnotic treatment session 540 versus a value of 4.11 during the normal awake state 530. In other words, during the hypnotic treatment session 540, there is an increase in the level of dissociation of more than 80% with respect to the normal awake state 530.

Moreover, as shown in further detail with respect to table 2, a statistically relevant correlation within the self-reported data for both the hypnotic treatment session 540 and the normal awake state 530 was found, in that the value for the self-reported level of pain was found to be inversely correlated with the self-reported value for the level of dissociation. This thus means that, when the subject is in a state of consciousness associated with a higher value for the self-reported level of dissociation, this will lead to a lower value for the self-reported level of pain under identical pain conditions.

Additionally, as shown in further detail in table 2 below, there was found to be no statistically significant correlation between the self-reported level of anxiety and the stimulation intensity of the pain stimulus, or in other words the intensity of the applied transcutaneous electrical stimulus. As already mentioned above, the self-reported level of anxiety thus corresponds to the level of anxiety as experienced by the subject, and is thus not directly correlated to the intensity of the external pain stimulus, or in other words the intensity of the anxiety inducing event itself, but relates to the level of anxiety as cognitively experienced by the subject. In other words, the self-reported level of anxiety clearly relates to the experience of anxiety as it appears in the cognitive experience and optionally the autonomic physiological experience of the subject.

Additionally, as shown in further detail in table 2 below, there was found to be no statistically significant correlation between the self-reported level of pain and the stimulation intensity of the pain stimulus, or in other words the intensity of the applied transcutaneous electrical stimulus. As already mentioned above, the self-reported level of pain thus corresponds to the consciously perceived level of pain perception of the subject, and is thus not directly correlated to the intensity of the external pain stimulus itself, but relates to the conscious level of pain perception. In other words the perception of pain as it appears in the conscious perception consciousness of the subject, typically associated with the cortex, after potential modulation and/or suppression by for example the thalamus and/or other downstream and/or upstream neural pathways.

Additionally, there is a certain level of statistically relevant correlation between the stimulation intensity of the pain stimulus and the self-reported level of dissociation. This means that subjects that reported a higher value for the level of dissociation during the experiment, correlated a higher intensity of the applied electrical stimulus to the value of 8 on the pain scale during the calibration step.

Table 2 below shows the Spearman correlation for both hypnotic treatment session 540 and normal awake state 530:

| | Self-reported level of Dissociation | Self-reported level of Pain | Self-reported level of Anxiety |
|---|---|---|---|
| Stimulation Intensity of the electrical pain stimulus | rs = 0.471 p = 0.019 | rs = -0.150 p = 0.483 | rs = -0.205 p = 0.334 |
| Self-reported level of Dissociation | - | rs = -0.717 p = <0.0001 | r = -0.49 p = 0.014 |
| Self-reported level of Pain | - | - | r = 0.8 p < 0.001 |

In Table 2, the r is the general correlation coefficient. The rs is the spearman correlation coefficient. It can take a range of values from +1 to -1. A value of 0 indicates that there is no association between the two variables. A value greater than 0 indicates a positive association; that is, as the value of one variable increases, so does the value of the other variable. A value less than 0 indicates a negative association; that is, as the value of one variable increases, the value of the other variable decreases. The p is the p-value associated with the statistical test to evaluate whether the correlation is statistically significant and not just a coincidence. Generally, a p-value smaller than 0.05 is considered as statistically significant. When a p-value is closer to zero, this shows a higher statistical relevance, or in other words a better correlation.

Results from the analysis of the EEG measurement data generated during experiment 500 in combination with the self-reported data allowed associations to made between localized brain structures/networks and the experience of anxiety, the perception of pain and/or a modified state of consciousness, such as for example dissociation. Identification of those localized structures/networks associated with the experience of anxiety, perception of pain and modified state of consciousness allows for an objective combined measurement of the level of anxiety, the level of pain as perceived by the subject, or in other words the level of pain perception of the subject and/or of the modifications to the state of consciousness of the subject, for example during a hypnotic treatment session or any other suitable treatment session. It also allows to determine a link between a level of anxiety experienced by a subject, a level of pain as perceived by the subject, when undergoing a calibrated external pain stimulus; which also serves as a calibrated anxiety inducing stimulus, and optionally the modification, suppression and/or modulation thereof by a modified state of consciousness of the subject, such as for example characterized by means of a level of dissociation has now been determined by means of the experiment 500.

In the brain, several types of brain activity occur at the same time, which is also referred to as parallel processing. Specific types of brain activity can be correlated to a specific frequency band in the measured EEG data during the experiment. In order to explore the different types of brain activity, use can be made of a suitable processing of the EEG measurement data generated during the experiment 500, by transforming the time domain signals into the frequency domain, for example by means of a fast Fourier transform or FFT operation. From such frequency domain signals, then for example the power of a specific signal frequency band can be determined, where power for example refers to a square of the amplitude of the frequency domain signals within a specified in a frequency range associated with one or more particular brain waves, such as described in further detail below. Such frequency bands could for example comprise a combination of at least two, or exactly two types of brainwaves, such as for example a delta-theta frequency band, or a theta-alpha frequency band. This means that such theta-alpha frequency band for example consists of a combination of at least part of a theta frequency band and at least part of an alpha frequency band. This means that such a theta-alpha frequency band for example consists of a combination of at least part of a theta frequency band and at least part of an alpha frequency band. This means that such delta-theta frequency band for example consists of a combination of at least part of a delta frequency band and at least part of a theta frequency band. This means that such a delta-theta frequency band for example consists of a combination of at least part of a delta frequency band and at least part of a theta frequency band. According to the embodiments described below, the frequency bands could for example comprise a combination of at least two, or exactly two types of brainwaves, such as for example the delta-theta frequency band comprising a combination of the two brainwaves delta and theta and the theta-alpha frequency band comprising a combination of the two brainwaves theta and alpha.

In the EEG data of the measured data during the experiment 500, there were found significant power differences in a specific delta-theta (dt) frequency band, comprising a combination of two different types of brain waves, when comparing the measured data of the subject during the hypnotic treatment session 540, which is an embodiment of a non-pharmacologically induced modified state of consciousness, as compared to a normal awake state 530, and which relates to two different self-reported levels of anxiety as experienced by the subject and two different self-reported levels of pain as perceived by the subject:

- A P-power(dt) which is decreased during the hypnosis treatment session 540 with respect to the normal awake state 530 in the 1-6 Hz frequency band, which is an embodiment of a delta-theta, dt, frequency band within the delta-theta frequency range, located around the Parietal lobe (P), which is also referred to as the early component. As will be explained in further detail below, such EEG measurement data can be used as part of a group 8 indicator for the level of anxiety of a subject, and more particularly the experienced level of anxiety of a subject. It is clear from the above-mentioned description that also self-reported level of anxiety is also significantly different during the hypnosis treatment session 540 with respect to the normal awake state 530.

- A power(dt) which is decreased during the hypnosis treatment session 540 with respect to the normal awake state 530 in the 1-6 Hz frequency band, which is an embodiment of a delta-theta, dt, frequency band within the delta-theta frequency range, located around the frontal lobe (F), which is also referred to as the early component. As will be explained in further detail below, such EEG measurement data can be used as part of a group 1 indicator for the state of consciousness of a subject, and more particularly to a level of dissociation of a subject. It is clear from the above-mentioned description that also both the self-reported level of dissociation and the self-reported level of pain are also significantly different during the hypnosis treatment session 540 with respect to the normal awake state 530.

- A power(ta) which is increased during the hypnosis treatment session 540 with respect to the normal awake state 530 in the 6-12 Hz frequency band, which is an embodiment of a theta-alpha, ta, frequency band within the theta-alpha frequency range), located around frontal lobe (F), central lobe (C) and parietal lobe (P), which is also referred to as a late component. It is however clear that power(ta) could also be determined at any other suitable scalp location. As will be explained in further detail below, such EEG measurement data can be used as part of a group 4 indicator for the level of pain perception of a subject.

It is clear from the above-mentioned description that any suitable combination of at least two of the self-reported level of anxiety, the self-reported level of dissociation and/or the self-reported level of pain are significantly different during the hypnosis treatment session 540 with respect to the normal awake state 530.

The early component refers to the power(dt) decrease that was measured in a time period from 0.1 to 0.5 sec after the pain stimulus onset and/or the onset of any other suitable anxiety inducing event. Late component refers to the power(ta) increase that was measured in a time period from 0.38 to 0.78 sec after the pain stimulus onset. According to this specific embodiment, in which there is an external pain stimulus, the power was calculated from -0.5s to 1.5s from with reference to the stimulus onset, or in other words the power was calculated in a time window of 2s including the stimulus onset. It is however clear that alternative embodiments are possible, in which for example a time window of at least 0.1s, for example a time window in the range of 0,5s up to and including 60s, or any other suitable time window is used, at any other suitable point in time with respect to the stimulus onset, as long as the time window includes at least a portion at a point in time after the stimulus onset. According to still further embodiments, such as for example during continuous power measurements, not related to a specific external stimulus, the power can be continuously calculated based on overlapping time windows of for example 2s, or of any other suitable time period, such as for example at least 0.1s, or in the range of 0.5s up to and including 60s, ...

The abovementioned results are the first identifying location-specific brain frequency band differences between an automated hypnosis treatment session and a normal awake state, especially for a brain frequency band comprising and/or consisting of a combination of at least part of two brain waves such as for example theta and alpha brainwaves, or delta and theta brain waves. It is clear, that these results are also the first at identifying this for any other suitable treatment sessions, for example pharmacological and/or non-pharmacological treatment sessions, and/or in which no use is made of a treatment session. It is clear that this is also the first identification of brain frequency band differences, especially a brain frequency band comprising a combination of two brain waves, with respect to different levels of anxiety as experienced by the subject and/or levels of pain as perceived by the subject. As already mentioned above, it is clear that such a frequency band comprising and/or consisting of a combination of two brain wave frequencies, or at least a part thereof, is different from a frequency band only comprising a single brain wave frequency band and different from a frequency band comprising more than two brain wave frequency bands.

From the measured EEG data during the experiment 500, there was extracted power(dt)) and/or power(ta) at EEG electrodes F, C and/or P, and the following results were obtained:

- The self-reported level of anxiety was correlated (spearman) with computed group 8 indicator, or in other words P-power (dt) at EEG electrode P (rs = 0.51, p = 0.022). FIG. 2 shows the correlation between the self-reported level of anxiety and the computed P-power (dt) at EEG electrode P from the measured data during the experiment 500, for the subjects that took part in experiment 500 as described above. It is thus clear that the specific group 8 indicator, P-power (dt) is correlated to the self-reported value for the level of anxiety;

- The group 8 indicator, or in other words, the P-power(dt) was significantly lowered during the hypnosis treatment session 540 as compared to the normal awake state 530, as calculated from the EEG measurement data at the EEG electrode location P (0.84 mV during normal awake state 530 vs 0.44 mV during the hypnosis treatment session 540, p = 0.027). This is consistent with the above-mentioned correlation between the group 8 indicator, P-power (dt) and the self-reported value for the level of anxiety. An embodiment of this experimental data is for example shown in FIG. 2.

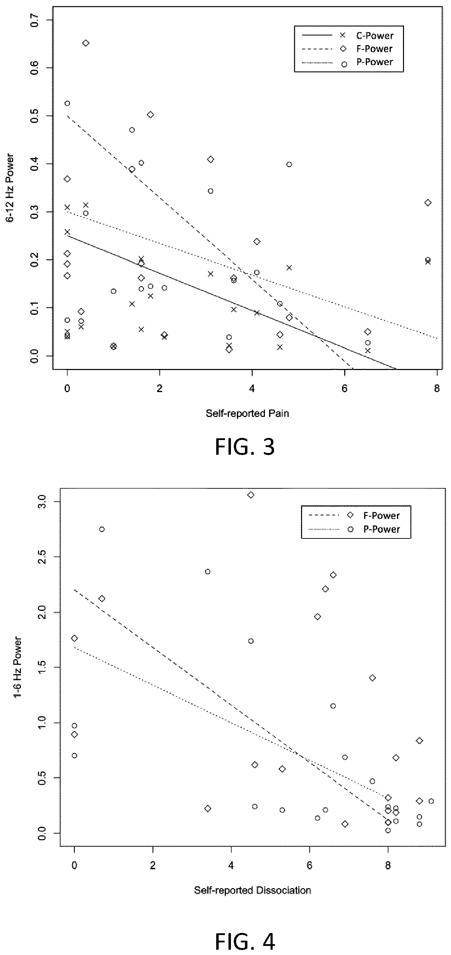

- The self-reported level of pain was correlated (spearman) with the computed group 4 indicator, or in other words power(ta) at EEG electrodes F (rs = -0.29, p = 0.10), P (rs = -0.33, p = 0.15) and/or C (rs = - 0.49, p = 0.02). Fig. 3 shows the correlation between self-reported level of pain as perceived by the subject and the computed power (ta) at EEG electrodes F, P and/or C from the measured data during the experiment 500 in more detail, for the subjects that took part in experiment 500 as described above. It is thus clear that the group 4 indicator, power (ta) is inversely correlated to the self-reported value for the level of pain;

- The group 4 indicator, or in other words power(ta) was significantly higher during the hypnosis treatment session 540 as compared to the normal awake state 530, as calculated from the EEG measurement data at the 3 EEG electrodes locations F (0.13 during normal awake state 530 vs 0.29 during the hypnosis treatment session 540, p = 0.009), P (0.16 during the normal awake state 530 vs 0.29 during the hypnosis treatment session 540, p = 0.009) and C (0.089 during normal awake state 530 vs 0.147 during the hypnosis treatment session 540, p = 0.005); This is consistent with the above-mentioned inverse correlation between the group 4 indicator, power (ta) and the self-reported value for the level of pain;

- The self-reported measure of dissociation was correlated (spearman) with computed group 1 indicator, or in other words power (dt) at EEG electrodes P (rs = -0.37, p = 0.011) and F (rs = -0.55, p = 0.010). FIG. 4 shows the correlation between the self-reported level of dissociation and the computed power (dt) at EEG electrodes F, P from the measured data during the experiment 500, for the subjects that took part in experiment 500 as described above. It is thus clear that the group 1 indicator, F-power (dt) is inversely correlated to the self-reported value for the level of dissociation. It is further also clear that other scalp locations such as the parietal scalp location don't have a correlation that is as high as the frontal scalp location, which is selected for the F-power(dt) of the group 1 indicator.

- The group 1 indicator, or in other words, the F-power(dt) was significantly lowered during the hypnosis treatment session 540 as compared to the normal awake state 530, as calculated from the EEG measurement data at the EEG electrode location P (0.84 during normal awake state 530 vs 0.44 during the hypnosis treatment session 540, p = 0.027) and F (1.01 during normal awake state 530 vs 0.43 during the hypnosis treatment session 540, p = 0.048); This is consistent with the above-mentioned inverse correlation between the group 1 indicator, F-power (dt) and the self-reported value for the level of dissociation. An embodiment of this experimental data is for example shown in FIG. 4. According to a preferred embodiment, the group 1 indicator is only based on the F-power(dt), and not on any EEG data from other scalp locations such as for, as this provides for the best correlation. Optionally the group 1 indicator could be based on F-Power(dt) combined with P-power(dt), however, it should be clear that according to embodiments an evaluation protocol will assign a larger weight to the F-Power(dt) component than to the P-power(dt) component when determining DoS.

It should be clear that the P-power (dt) calculated from the combined brainwave frequency band delta-theta, surprisingly provides for a substantially higher correlation to the self-reported level of anxiety of the each of its composing individual brainwave frequency bands, i.e. delta and theta. The self-reported level of anxiety was correlated (spearman) with P-power (delta), i.e. the same power calculation for the individual delta brain wave frequency band, at the same EEG electrode P, with an rs = 0.09 and a p-value = 0.80. The self-reported level of anxiety was correlated (spearman) with P-power (theta), i.e. the same power calculation for the individual theta brain wave frequency band, at the same EEG electrode P, with an rs = 0.24 and a p-value = 0.4. It is thus clear that the P-power of the specific delta-theta combined frequency band provides for a surprising and unexpectedly high level of correlation, which could not have been foreseen based on either of the individual frequency band components delta and theta.

The above experimental data generated during the experiment 500, which comprises both measurement data, such as EEG data, as well as self-reported data, and which could also be referred to as response data, confirms the existence of a correlation between self-reported level of anxiety and a power of a specific combined brain wave frequency band at a specific location, P-power (dt), associated with a delta-theta frequency band, dt, which according to this embodiment extends from 1Hz up to and including 6Hz, also referred to as a group 8 indicator. However, as already mentioned above, it is clear that alternative embodiments are possible in which the frequency band comprises a combination of both delta brainwaves and theta brainwaves.

It was determined from the experimental data above that, surprisingly, only measured data received from the P EEG electrodes show a certain level of correlation of power (dt) with the self-reported level of anxiety. In the current experiment there was found no correlation at different electrode locations such as for example the C and F electrode location. The experimental data for the C-EEG electrode, provided a C-power(dt) with an rs=-0.09 with a p-value=0.69 for the correlation with the self-reported level of anxiety. Similarly, the experimental data for the F-EEG electrode, provided for a F-power(dt) with an rs=0.18 with a p-value=0.44 for the correlation with the self-reported level of anxiety. It is thus clear, that only the EEG measurement data resulting from electrodes at a parietal location provided for a sufficiently high level of correlation with the level of anxiety, while the EEG measurement data from electrodes at other locations, such as the central and frontal location, did not provide for such a sufficiently high level of correlation, or in other words, there was no or a level of correlation that was very low. According to the embodiment described here the absolute value of the rs value at the parietal location was greater than or equal to 0.45 and the absolute value of the rs value at other locations was lower than or equal to 0.20 .

It is clear that according to alternative embodiments, an alternative external pain and/or anxiety stimulus could be provided. It is clear that instead of and/or in addition to measurement of the level of anxiety in reaction to an external pain and/or anxiety stimulus, the experimental data can also be used for measurement of a level of anxiety caused by internal events or states of the subject, which for example could relate to anxiety experienced, which is not caused by an external event, such as an external pain and/or anxiety stimulus, being applied. It is clear that alternative embodiments of the experiment are possible, in which the P-power(dt) is determined for any other suitable frequency band comprising a combination of both delta brain waves and theta brainwaves, or at least part thereof, such as for example any suitable frequency band extending in the frequency range of 0Hz up to and including 8 Hz.

The above experimental data generated during the experiment 500, which comprises both measurement data, such as EEG data, as well as self-reported data, and which could be referred to as response data, confirms the existence of a correlation between self-reported pain and a power, power (ta) associated with a theta-alpha frequency band, ta, which according to this embodiment extends from 6Hz up to and including 12Hz, also referred to as a group 4 indicator. However it is clear that alternative embodiments are possible in which the frequency band comprises a combination of both theta brainwaves and alpha brainwaves. It is clear from the experimental data above that measured data received from the C, P and F EEG electrodes all show a certain level of correlation of power (ta) with the self-reported measure of pain. Although, in the current experiment the correlation is highest at the C EEG electrode, it is clear that according to alternative embodiments, the distribution of the level of the correlations amongst different EEG electrodes might be different. However, it is clear that according to alternative embodiments an alternative external pain stimulus could be provided. It is clear that instead of and/or in addition to measurement of the level of pain in reaction to an external pain stimulus, the experimental data can also be used for measurement of a level of pain caused by internal events or states of the subject, which for example could relate to pain experienced, which is not caused by an external event, such as an external pain stimulus, being applied. It is clear that alternative embodiments of the experiment are possible, in which the power(ta) is determined for any other suitable frequency band comprising a combination of both theta brain waves and alpha brainwaves, such as for example any suitable band extending in the frequency range of 4Hz up to and including 12 Hz.

These findings have been advantageously applied in a computer-implemented method for measurement of a level of anxiety, a level of modified consciousness and/or a level of pain. Based on the insight that there exists a correlation between P-power(dt) and self-reported measurements of the level of anxiety, and that there exists a correlation between power(ta) and self-reported measurements of the level of pain, it now becomes possible to more objectively measure a level of anxiety and/or a level of pain, even in cases where self-reported anxiety and/or pain measures are not possible or available, such as for example when the subjects are in a state in which they are unable or experience difficulties to communicate, when the subjects are young children, ... It is clear that the Level of Anxiety, or LoA is an indicator of the strength of the psychophysiological response to an anxiety inducing event or situation. It is clear that such a level of anxiety as experienced by the subject can be modulated by for example its emotional, psychological, cognitive state, such as for example the state of consciousness of the subject. The LoA is thus an indicator for the level of anxiety as it is experienced of the subject and can thus be used as an indicator of the degree to which the subject experiences anxiety, for example on a cognitive, emotional and/or physical level.

It is clear that the Level of Pain, or LoP is an indicator of the level or degree to which a subject is aware and receptive to pain. In other words, it refers to the level of pain as consciously perceived by the subject. It is clear that such a level of pain perception by the subject can be modulated by for example its emotional, psychological, cognitive state, such as for example the state of consciousness of the subject. The LoP is thus an indicator for the subjective pain perception of the subject and can thus be used as an indicator of the degree to which the subject experiences pain, for example on an emotional, cognitive and/or physical level.

It is thus clear that anxiety, and more specifically anxiety as defined above, includes a cognitive experience and optionally also a physiological experience by the subject. In other words, the level of anxiety as experienced by the subject is a subjective, psychophysiological experience that is influenced to varying degrees by for example biological, psychological, cognitive, physiological, neurophysiological and social factors. The experience that something causes anxiety includes for example cognitive features, such as for example intensity, quality, ... of an anxiety stimulus and/or affective emotional aspects. It should further be noted that in this way anxiety as experienced and purely autonomic physiological stress responses are different, and can for example occur independently of each other.

As explained in further detail below, in the context of this application a Level of Anxiety Index, or LoAI, or Anxiety index, refers to a standardized index indicating the level of anxiety of which the subject is at least cognitively experiencing and optionally physiologically experiencing. In other words, the degree to which the subject experiences anxiety at a cognitive dimension, optionally supplemented by for example an autonomic sensory and/or an emotional level as described in further detail below. This LoAI for example varies systematically with the intensity of anxiety as experienced by the subject, independently of factors not affecting the experience of anxiety. A first limit of the LoAI, for example a lower limit of the LoAI, may for example indicate that the subject experiences no anxiety. A second limit of the LoAI, for example an upper limit of the LoAI, may for example indicate that the subject experiences the strongest/worst possible experience of anxiety.

It is thus clear that pain in the context of this description, as for example defined by the International Association for the Study of Pain or IASP is an aversive sensory and emotional experience typically caused by, or resembling that caused by, actual or potential tissue injury. It is thus clear that pain is experienced as a conscious perception by the subject. In other words, the level of pain as experienced by the subject is a subjective experience that is influenced to varying degrees by for example biological, psychological and social factors. The conscious perception that something causes pain includes for example sensory-discriminative features, such as for example intensity, quality and location, ... and/or affective emotional aspects. It should further be noted that in this way pain as consciously perceived and nociception are different phenomena, which can occur independently of each other. The conscious experience or perception of pain cannot always be reduced to activity in sensory pathways. Nociception as defined by IASP is the neural process of encoding noxious and/or painful stimuli. The consequences of such encoding may be automatic such as for example an increased blood pressure, or behavioral, such as for example a motor reflex. However, it is clear that a conscious pain experience is not necessarily implied. As explained in further detail below, in the context of this application a Level of Pain Index, or LoPI, or Pain index, refers to a standardized index indicating the level pain of which the subject is consciously aware. In other words, the degree to which the subject consciously perceives pain at for example a sensory and/or an emotional level. This LoPI for example varies systematically with pain intensity as perceived by the subject, independently of factors not affecting pain perception. A first limit of the LoPI, for example a lower limit of the LoPI, may for example indicate that the subject experiences no conscious perception of pain. A second limit of the LoPI, for example an upper limit of the LoPI, may for example indicate that the subject experiences the strongest possible conscious perception of pain.

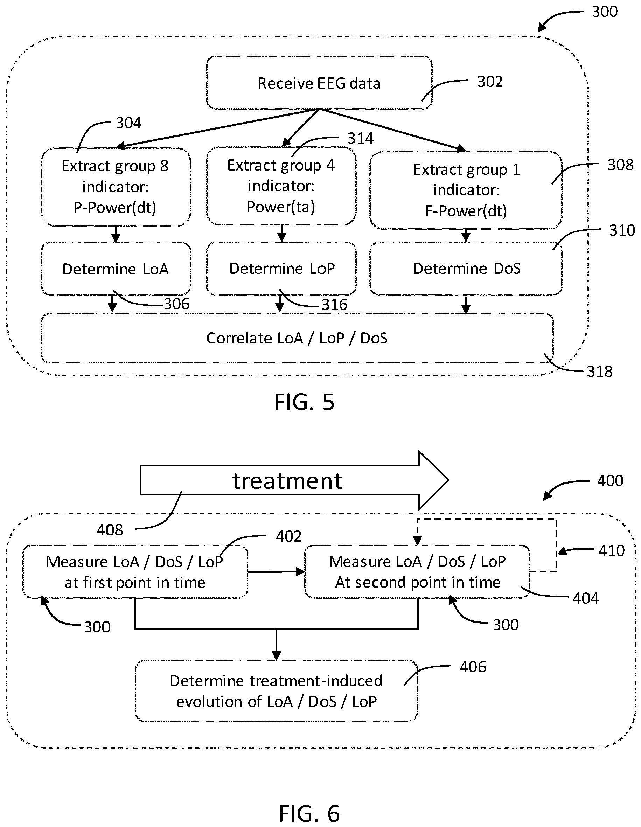

According to the embodiment shown in FIG. 5, such a computer-implemented method for an objective measurement of a level of anxiety experienced by a subject, a level of modified consciousness in a subject and/or a level of pain 300 as consciously perceived by a subject.

According to the embodiment shown in FIG. 5, such a computer-implemented method 300 for an objective measurement of a level of anxiety or LoA as experienced by a subject, level of pain or LoP as perceived by a subject and a level of modified state of consciousness or DoS in a subject, for example comprises the steps of receiving EEG data 302, extracting P-power(dt) 304 and determining a level of anxiety, LoA, 306. At step 302 there is received measured data comprising electroencephalogram, EEG, data collected from one or more P-EEG electrodes. At step 304, there is then extracted from the EEG data, a group 8 indicator corresponding to: a power, P-power (dt), associated with a delta-theta frequency band, dt, within a delta-theta frequency range. At step 306 there is then determined, based on said group 8 indicator, a level of anxiety, LoA, which is a value indicative for the level of anxiety as experienced by the subject. It is clear that, according to alternative embodiments, optionally, in addition to said group 8 indicator, further elements, such as other group indicators, measurements, parameters, reference measurements, ... could be taken into account for determining the LoA. It is clear that according to particular embodiments the method could for example comprise additional steps, such as for example after the step of receiving EEG data or any other suitable data, pre-processing the EEG data or other data, subsequently extracting relevant features, thereby allowing the determination of for example group 8 indicators or other suitable group indicators, and subsequently normalization and/or classification, thereby for example determining LoA or an indexed and/or scaled version of LoA, such as for example LoAI as will be explained in further detail below. It is however clear that still further alternative embodiments are possible.

According to the embodiment of the experimental data generated above, it was found that for a population with a mean self-reported anxiety value of 4.92, there was measured a mean P-power(dt) of 0.84 mV$^2$, when a calibrated pain stimulus of value 8 on the pain scale was applied in normal awake state 530. For a population with a mean self-reported anxiety value of 3.16, there was measured a mean P-power (dt) of 0.44 mV$^2$, when a calibrated pain stimulus of value 8 on the pain scale was applied during the hypnotic treatment session 540. As already mentioned above, from the experimental data above, the LoA is correlated to the group 8 indicator, P-power(dt), or in other words an increase in P-power(dt) corresponds to an increase in the LoA, and a decrease in P-power(dt) corresponds to an decrease in the LoA as experienced by the subject. As already mentioned above, it is clear that the P-power(dt) values mentioned above, were calculated from - 0.5s to 1.5s from with reference to the stimulus onset, or in other words the power was calculated in a time window of 2s including the stimulus onset, according to an embodiment where there was an external pain and/or anxiety stimulus. As further mentioned above, it is however clear that alternative embodiments are possible, in which alternative suitable time windows are being used, and/or in which the time window is not related to a specific external stimulus, such that for example the power can be continuously calculated based on overlapping time windows.

Further, according to the embodiment of the method shown in FIG. 5, which for example comprises the steps of receiving EEG data 302, extracting power(ta) 314 and determining a level of pain, LoP, 316. At step 302 there is received measured data comprising electroencephalogram, EEG, data collected from one or more EEG electrodes. At step 314, there is then extracted from the EEG data, a group 4 indicator corresponding to: a power, power (ta), associated with a theta-alpha frequency band, ta, within a theta-alpha frequency range. At step 306 there is then determined, based on said group 4 indicator, a level of pain, LoP, which is a value indicative for the level of pain in the subject. It is clear that, according to alternative embodiments, optionally, in addition to said group 4 indicator, further elements, such as other group indicators, measurements, parameters, reference measurements, ... could be taken into account for determining the LoP. It is clear that according to particular embodiments the method could for example comprise additional steps, such as for example after the step of receiving EEG data or any other suitable data, pre-processing the EEG data or other data, subsequently extracting relevant features, thereby allowing the determination of for example group 4 indicators or other suitable group indicators, and subsequently normalization and/or classification, thereby for example determining LoP or an indexed and/or scaled version of LoP, such as for example LoPI as will be explained in further detail below. It is however clear that still further alternative embodiments are possible.

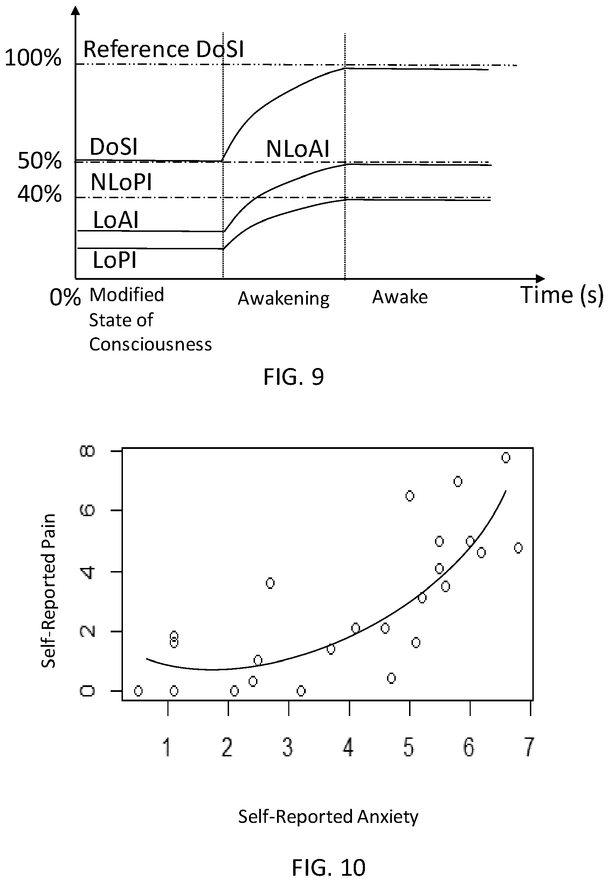

Optionally and preferably according to the embodiment of FIG. 5 in step 318 there could be correlated a plurality of LoA, LoP and/or DoS measurements and/or determining a correlation between a plurality of LoA, LoP and/or DoS measurements as will be explained in further detail below, for example with reference to the embodiments of FIG. 10 - FIG. 16, etc..

According to the embodiment of the experimental data generated above, it was found that for a population with a mean self-reported pain value of 4.2, there was measured a mean power(ta) of 0.16 mV$^2$, when a calibrated pain stimulus of value 8 on the pain scale was applied in normal awake state 530. For a population with a mean self-reported pain value of 1.4, there was measured a mean power (ta) of 0.29 mV$^2$, when a calibrated pain stimulus of value 8 on the pain scale was applied during the hypnotic treatment session 540. As already mentioned above, from the experimental data above, the LoP is inversely correlated to the group 4 indicator, power(ta), or in other words an increase in power(ta) corresponds to a decrease in the LoP, and a decrease in power(ta) corresponds to an increase in the LoP as consciously perceived by the subject.

It is further clear, that the response data, for example representing a subject's response to the treatment session can also be used to determine the state of consciousness of the subject, in particular a level of a modified state of consciousness.

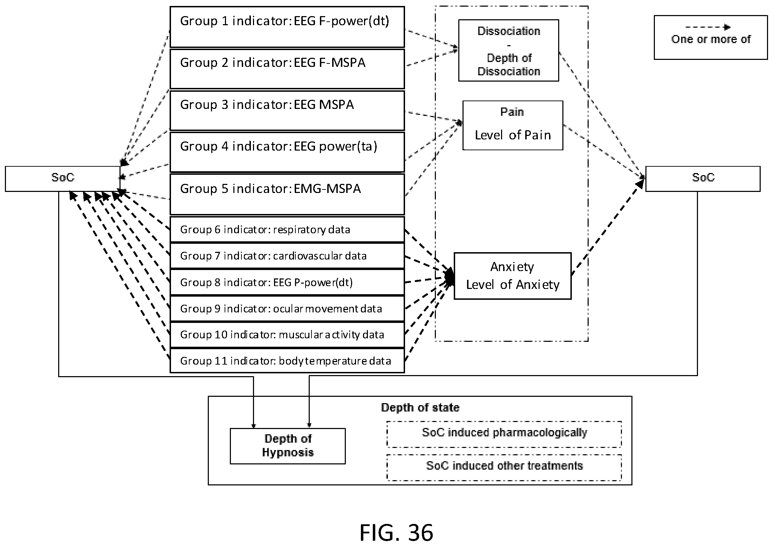

According to a further aspect, as schematically shown in FIG. 36, as described in more detail in co-pending patent application PCT/EP2020/053136, which is incorporated herein by reference, the method comprises further steps for measuring a depth of state, DoS by means of a group 1 indicator and optionally a group 2 indicator.

It is clear that according to such an embodiment, similar as explained above, there is receiving measured data at step 302 comprising EEG data comprising data collected from at least one frontal (F) EEG electrode located on the scalp anatomical region corresponding to a frontal lobe of the subject. This then allows the extraction of a group 1 indicator at step 308 based on, a power, F-power(dt), associated with a delta-theta frequency band, dt, within a delta-theta frequency range extracted from the at least one F- EEG electrode data. Based on this group 1 indicator, there can then be determined a DoS. Based on the group 1 indicator, there can then be determined a depth of state, DoS, which is a value indicative for the level of a modified state of consciousness.

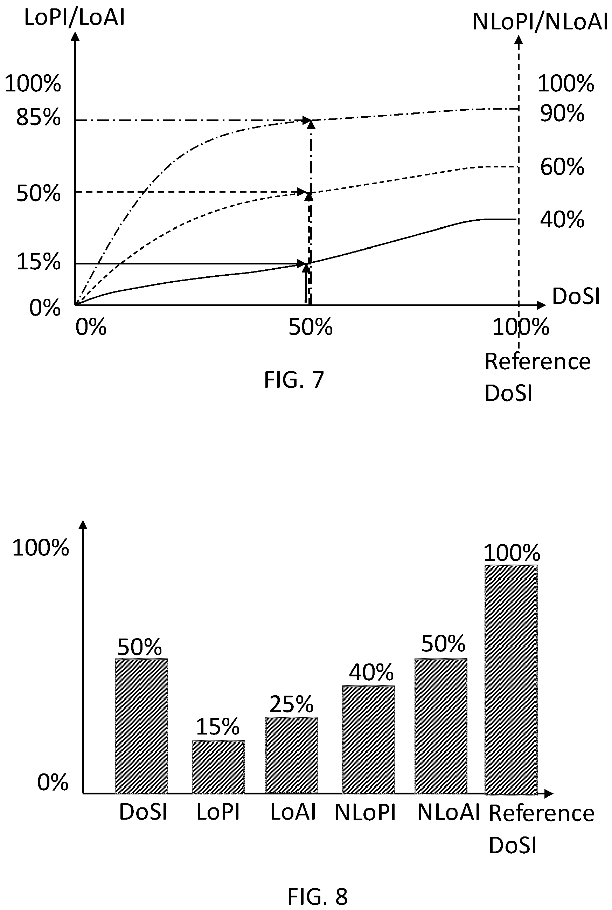

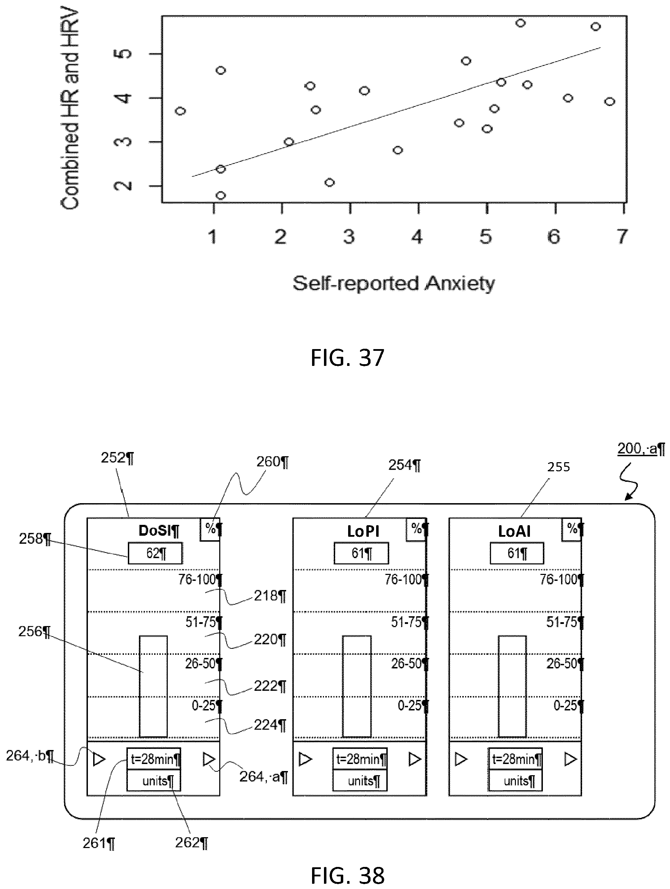

When according to a preferred embodiment, there are determining a plurality of LoA and/or LoP at different DoS; and/or a plurality of LoP at different LoA; and/or a plurality of LoA at different LoP; etc. from measurement data and/or measurement data comprising simultaneous measurement of LoA and/or LoP and DoS obtained, preferably, but not necessarily under identical pain conditions and/or under identical anxiety inducing events. It should be clear that identical pain conditions, do not necessarily mean constant pain conditions, it only means that at the time of measurement of the LoA and/or LoP and DoS are measured, the anxiety and/or pain conditions are identical, substantially identical and/or similar to. It is clear that alternative embodiments are possible in which the measurement of LoA, LoP and/or DoS is not performed simultaneous. Based on such measurements there can then for example be determined the modulation of LoA and/or LoP by DoS, by evaluating the evolution of the LoA and/or LoP at different DoS and/or the evolution of the LoA and/or LoP with respect to the evolution of DoS from the measurement data obtained, preferably, but not necessarily under identical pain and/or anxiety conditions. According to an alternative embodiment the LoP can be adjusted based on said DoS and/or said LoA to a level of normalized pain, NLoP, which is a value indicative for the level of pain in the subject at a predetermined reference DoS and/or a predetermined reference LoA. According to an alternative embodiment the LoA can be adjusted based on said DoS and/or said LoP to a level of normalized anxiety, NLoA, which is a value indicative for the level of anxiety in the subject at a predetermined reference DoS and/or at a predetermined reference LoP. The reference DoS could for example be that of a normal awake state, the reference LoP could for example be a level of pain where no or a minimal amount of pain is perceived and/or the reference LoA could for example be a level of anxiety where no or a minimal level of anxiety is experienced, such as for example shown in FIG. 7 - FIG. 9, in which DoS, LoA, NLoA, LoP and NLoP have been scaled and indexed as DoSI, LoAI, NLoAI, LoPI and NLoPI as explained in further detail below, and similar as explained for example with reference to FIG. 38. However it should be clear that in the context of this description, with respect to calculation, displaying, etc. of values related to DoS, LoA, NLoA, LoP, NLoP, and any other parameters, where examples are given with respect to the indexed and/or scaled parameters such as DoSI, LoAI, NLoAI, LoPI, NLoPI, etc. according to alternative embodiments these can be replaced by the non-indexed and/or scaled values such as DoS, LoA, NLoA, LoP, NLoP, etc. or vice versa. In this way the value for NLoP would provide for a value of LoP at a predetermined reference DoS of interest, independently of the current DoS and/or any changes to the current DoS, thereby for example providing an indicator for the equivalent amount of pain that would be consciously perceived when the subject would be, or return to a normal awake state of consciousness, when this reference DoS is the normal awake state such as for example shown in Fig. 7-9. It is however clear that alternative embodiments are possible, in which for example NLoP is based on a predetermined reference LoA, and/or a combination of a predetermined reference LoA and a predetermined reference DoS, etc.. In Fig. 7 NLoP or NLoAI, or according to the embodiment shown NLoPI or NLoAI can be calculated as follows. When for example a DoSI of 50% is measured and a LoPI or LoAI of 15%, this refers to a particular point which is part of a predetermined correlation between DoS and LoP or DoS and LoA, for example determined by measuring LoPI or LoAI at a plurality of different DoSI under identical pain and/or anxiety conditions, however it is clear that alternative embodiments are possible. As shown by means of the relevant predetermined correlation function between LoPI and DoSI or LoAI and DoSI represented by the full line, it is possible to determine the NLoPI or NLoAI to be 40% at the reference DoSI, which according to this embodiment is set to 100%. This means that when the DoSI would be changed from the measured DoSI of 50% to the reference DoSI of 100%, the level of pain perceived by the subject or the level of anxiety experienced by the subject, for example under the current pain and/or anxiety conditions or in other words when the pain and/or anxiety conditions would remain identical or unchanged, would rise from the measured 15% to 40%. Similarly, as shown in FIG. 7, when at DoSI=50% and there would be measured a LoPI or LoAI=50% or 85%, this would result in a corresponding NLoPI=60% or 90% respectively, by means of the dashed or dot-dashed correlation functions respectively. It is clear that alternative embodiments are possible for the calculation of NLoP or NLoAI based on a predetermined reference DoS and based on measurements of DoS and LoP or DoS and LoA than the schematic predetermined correlations illustrated in Fig. 7, such as suitable look-up tables, self-learning systems making use of individual and/or population based historical data, etc. It is clear that still further embodiments are possible in which for example the LoA based on said DoS is adjusted to a level of normalized anxiety, NLoA, which is a value indicative for the level of anxiety in the subject at a predetermined reference DoS. Similarly as explained above, it is clear, as will be explained in further detail below that the LoA is also influenced by the LoP, and therefor similarly the LoA could be adjusted, based on said LoP, instead of and/or in addition to the adjustment based on the DoS, to a level of normalized anxiety, NLoA, which is a value indicative for the level of anxiety in the subject at a predetermined reference LoP, and optionally at a predetermined reference DoS.

According to still further alternative embodiments, based on the insight that the LoP may be modulated by the LoA and the DoS as shown in further detail below, according to still further embodiments it might be useful to determine a plurality of LoP at different DoS and/or LoA from measurement data and/or measurement data comprising simultaneous measurement of LoA and DoS. According to a specific embodiment, this will for example help to determine the modulation of LoP by DoS and/or LoA, by evaluating the evolution of the LoP at different DoS and/or LoA and/or the evolution of the LoP with respect to the evolution of DoS and/or LoA from the measurement data.

FIG. 10 shows an exemplary embodiment of a method in which a plurality of LoA and LoP are correlated, based on the measurement data from the embodiment of the experiment of FIG. 1. The calculated correlation between the level of pain, LoP, and the level of anxiety, LoA, was found to fit a good correlation with r = 0.8, p-value < 0.001. This means that a higher level of anxiety is correlated and/or associated with a simultaneous higher level of pain. This correlation thus functions in such a way that the LoA increases as LoP increases and can even trigger pain perception. A combined measurement of LoA and LoP and the calculation of any corresponding correlations thus allows to determine which part of the LoP is associated with the LoA and could for example be treated by an anxiety management treatment. Alternatively, a combined measurement of LoA and LoP and the calculation of any corresponding correlations allows to determine which part of the LoA is associated with the LoP and could for example be treated by a pain management treatment.

It is thus clear from the above that pain can be a causes of anxiety and vice versa, and that the combined measurement of LoA and LoP and optionally the calculation of any correlations therebetween make it possible to determine the root cause, modulator, aggravating factor, etc. of anxiety and/or pain and to treat it effectively by means of a suitable treatment, for example an anxiety management treatment when the LoA is the main contributing component, or a pain management treatment when the LoP is the main contributing component, and/or a suitable weighted mix of an anxiety and pain management treatment, depending on the respective weights in the contribution of the LoA and/or the LoP and their respective correlation. It is clear that such correlations can most easily be determined between simultaneous measurements of LoA and LoP, however it is clear that alternative embodiments are possible in which the LoA and LoP that are correlated are not measured simultaneously, but during similar time periods and/or events, such as for example before, during and/or after a treatment. According to still further embodiments, when subject-based and/or population-based correlations are available, for example based on prior measurements of LoA, LoP and/or DOS, then these correlations could be stored and retrieved from for example a suitable database, for use during measurement of LoA, LoP and/or DoS and/or when making use of any correlations therebetween.

According to a particular advantageous use, based on the fact that anxiety plays an important role in chronic pain, such combined measurement of LoA and LoP and/or any associated correlation, it may be possible to for example determine patients with a high LoA, that may benefit from an anxiety management treatment in order to reduce the LoP associated with their chronic pain.

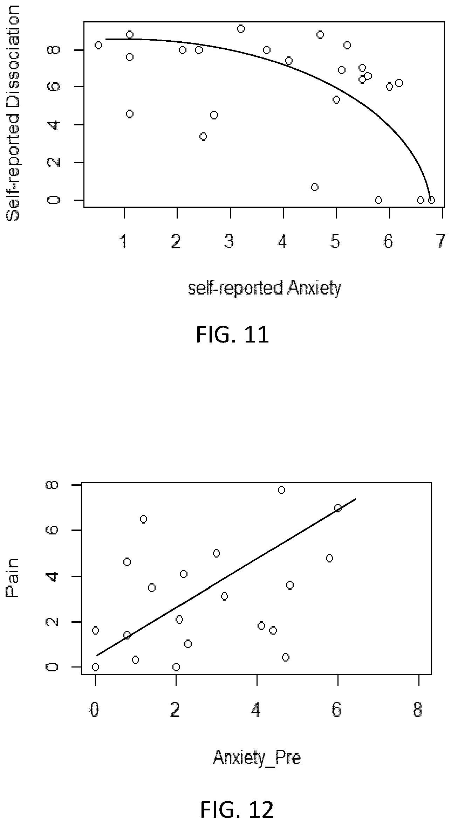

FIG. 11 shows an exemplary embodiment of a method in which a plurality of LoA and DoS are correlated, based on the measurement data from the embodiment of the experiment of FIG. 1. The calculated correlation between the level of anxiety, LoA, and the Depth of Dissociation, which is an embodiment of the Depth of State, DoS, was found to fit a good correlation with $r = -0.49$, p-value = 0.014 . In other words, there is an inverse correlation between the DoS and the LoA, which means that an increase in the depth of dissociation decreases the level of anxiety. Similar as explained above in this way, the combined measurement of LoA and DoS and optionally their correlation, for example enables to determine which part of the LoA could be managed by means of a treatment modifying the DoS, such as for example a treatment modifying the depth of dissociation, the level of absorption, etc. According to specific embodiments it might even be possible to determine and/or differentiate between short-term and long-term effects of for example treatments involving a modification of the DoS, for example by means of managing the depth of dissociation, the level of absorption, etc. for example by means of suitable pharmacological and/or non-pharmacological treatments, such as for example psychotherapy, etc. on a change, for example a reduction, in the level of anxiety (e.g. use in psychotherapies , ...).

FIG. 12 shows an alternative embodiment of a method in which a plurality of LoA and LoP are correlated, based on the measurement data from the embodiment of the experiment of FIG. 1. Different from the embodiment of FIG. 10 in which the level of anxiety and the level of pain were measured simultaneously, here the level of pain and the level of anxiety are not measured simultaneously. The LoA was measured before the test phase 520, for example at step 512 of the embodiment of FIG. 1, and the LoP was measure during the test session, preferably during and/or after painful, anxiety inducing stimulation, such as for example at step 534 and/or 544. However, it is clear that alternative embodiments are possible in which the LoA is for example measured before a treatment and the LoP during and/or after the treatment, and/or any other suitable embodiment in which the LoA and the LoP are measured at different moments in time. According to the embodiment shown in FIG. 12, the calculated correlation between the level of pain, LoP, during the test phase 520 and the level of anxiety, LoA, before the test phase was found to correlate with $r = 0.38$, p-value = 0.06. According to this embodiment of the correlation, it is found that a higher level of anxiety before the test phase 520, or any other treatment session, is associated with a higher level of pain during the test phase 520, or any other suitable treatment session. Such type of correlation, could be useful in measuring, managing and/or predicting, post-treatment levels of pain based on the LoA measured before the treatment and optionally combined with LoP measured before the treatment. According to a particular embodiment, this would for example enable to measure, manage and/or predict post-intervention pain. Alternatively, based on this correlation, the pre-treatment measurement of a LoA and optionally the LoP, could for example enable to determine subjects and/or a population for which an anxiety management treatment, before engaging in another treatment that comprises potential anxiety inducing stimuli, so that by managing the pre-treatment level of anxiety, the level of pain during and/or after the treatment could be reduced. As already mentioned above, anxiety is also a risk factor for developing persistent and/or chronic pain following an acute pain and/or anxiety inducing event. The combined, non-simultaneous measurement of LoA and LoP and any correlation, for example pre-treatment, could determine high risk subjects and/or a high risk group of subjects, with an increased risk of post-treatment chronic pain, and could potentially enable a reduction of this risk of developing chronic pain, for example by means of a suitable anxiety management treatment.

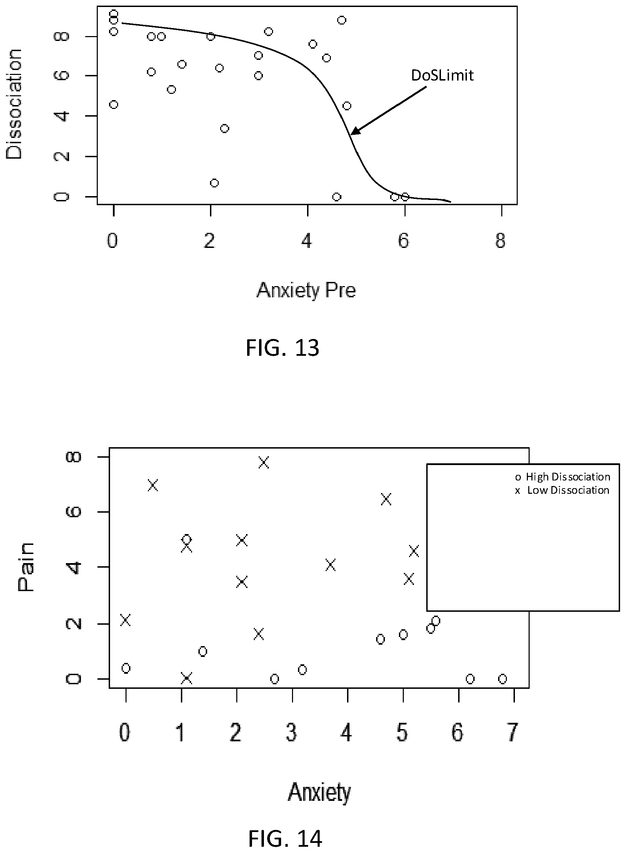

FIG. 13 shows an exemplary embodiment of a method in which a plurality of LoA and DoS are correlated, based on the measurement data from the embodiment of the experiment of FIG. 1. However different from the embodiment of FIG. 11, also here the LoA and DoS are not measure simultaneously. Similarly, as with the embodiment of FIG. 12, the LoA is measured before the treatment session, and the DoS is measured during the treatment session 540 of the test phase 520. The calculated correlation between DoS in the form of a depth of dissociation during digital treatment session and the level of anxiety before the test phase 520 has an r = -0.38, p-value = 0.06. In other words, a higher level of anxiety before the treatment session is associated by means of the correlation with a lower level of dissociation during the treatment session. In other words the level of anxiety before the treatment session limits the maximum level of and/or the maximum modification of the DoS that can subsequently be achieved during the treatment session involving a modified state of consciousness, such as for example the hypnotic treatment session 540 of the embodiment of Fig.1 or any other suitable embodiment of a treatment session. In this way the non-simultaneous combined measurement of the LoA and DoS and its associated correlation, enables for example to determine more objectively subjects with a too high anxiety level that could for example need of an anxiety treatment in order to reduce the LoA to within a predetermined desired range, before engaging in a further treatment session, for example a treatment involving a modified state of consciousness or any other suitable treatment.

As further, schematically shown in the embodiment of the correlation of FIG. 13, there can be calculated, based on the LoA, and according to this embodiment the corresponding correlation between the pre-treatment LoA and the in-treatment Dos, at least one limit and/or range for said DoS, which can be referred to as DoSLimit, which is indicative for the at least one limit and/or range of the modification of the level of consciousness attainable when the subject experiences said LoA. According to the embodiment shown, it is clear that according to this embodiment, which makes use of the correlation curve, that the DoSLimit slightly decreases from a Depth of Dissociation higher than 8 correlated with a pre-treatment LoA of 0, to a value of about 6 at a pre-treatment LoA of 4 and then steeply declines to a value of 0 correlated with pre-treatment LoA of 6 and higher. According to this particular embodiment, this thus means that when a pre-treatment LoA of for example 6 or higher is measured, it has no sense of initiating the treatment session involving a modified state of consciousness as it will not be able to modify the DoS, which according to this embodiment is embodied as the depth of dissociation. If the desired DoS to be reached during the treatment, is higher than the DoSLimit or in other words outside the attainable range as defined by the DoSLimit, then, according to this embodiment, first the pre-treatment LoA will need to be reduced, until a suitably high associated DoSLimit is reached, for example by means of a suitable anxiety management treatment. Alternatively, when a desired DoS is to be reached, for example by means of a suitable treatment, then, the LoA associated with the DoSLimit, will provide for a maximum level of the LoA that is acceptable when it is desired to reach his DoS. It is clear that alternative embodiments are possible in which the LoA and/or LoP associated with a DoSLimit, DoS and/or LoP associated with a LoALimit, LoA and/or Dos associated with a LoPLimit will define suitable limit values and/or ranges for a respectively desired LoA, LoP and/or DoS.

It is clear that alternative embodiments, are possible in which for example there is calculated, based on the LoA and/or the LoP, at least one limit and/or range for the DoS, DoSLimit, which is indicative for the at least one limit and/or range of the modification of the level of consciousness attainable when the subject experiences the LoA and/or the LoP. According to still further alternative embodiments, there can be calculated, based on the LoA, at least one limit and/or range for said LoP, LoPLimit, which is indicative for the at least one limit and/or range of the level of pain attainable when the subject experiences said LoA. Similarly as explained above when for example a LoA is measured associated with a LoPLimit that is higher than a desired LoP, then before for example engaging in a pain management treatment, it will be required to adjust the LoA by means of a suitable treatment such that a LoPLimit is reached for which the desired LoP does fall within its range and/or limits. According to still a further embodiment, there can for example be calculated, based on said LoP, and for example a suitable correlation with LoA, at least one limit and/or range for said LoA, LoALimit, which is indicative for the at least one limit and/or range of the level of pain attainable when the subject experiences said LoP. Similarly, as explained above if a desired LoA falls outside the range and/or limits of the LoALimit associated with a measured LoP, then first a treatment adjusting the LoP will need to be adjusted, such as for example a suitable pain management treatment, such that a suitable associated LoALimit can be attained, of which the desired LoA falls inside its range and/or limits.

Fig 14 shows still a further embodiment in which the level of pain and the level of anxiety are correlated. According to this embodiment, similar as with the embodiment of Figure 10, the LoA and the LoP are simultaneously measured. As shown, according to this embodiment the data is split into data points associated with a high level of dissociation indicated with a circle and data points associated with a low level of dissociation, which is an embodiment of DoS. Both the data with a low DoS as the data with a high DoS have a good correlation. LoA and LoP associated with a low DoS has a correlation with r=0.67 and a p-value=0.016. LoA and LoP associated with a high DoS has a correlation with r=0.68 and a p-value=0.018. It is thus clear that independently of the differences in the DoS, by means of a different depth of dissociation, the LoA and LoP are correlated, but as is clear from the embodiment of Fig. 14, equivalent LoA are associated with lower LoP when the depth of dissociation is high as compared to when the depth of dissociation is low. Similarly, equivalent LoP are associated with lower LoA when the depth of dissociation is high as compared to when the depth of dissociation is low. In other words, the impact of the depth of dissociation on the relation between the LoP and the LoA is such that the impact of LoA on the LoP is reduced. According to an embodiment this could for example be used for reducing the impact of the LoA on the LoP by for example increasing the depth of dissociation, or any other suitable DoS, or vice-versa, as the depth of dissociation then supports an enhanced effect of either LoP or LoA.

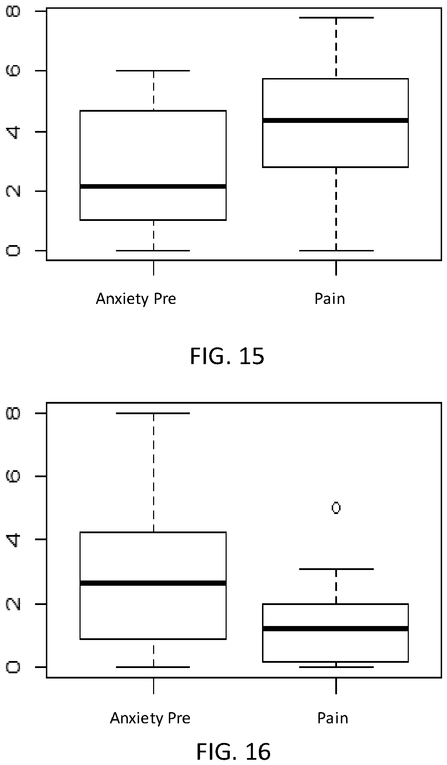

FIG. 15 show an embodiment of a non-simultaneous, combined measurement of LoA prior to the test session 520, and the LoP during painful stimuli during the normal awake state 530 which is associated with a low depth of dissociation with for example a mean = 3.11. FIG. 16 shows a similar embodiment of a non-simultaneous, combined measurement of LoA prior to the test session 520, and the LoP during painful stimuli during the hypnotic treatment session 540 which is associated with a higher depth of dissociation with for example a mean = 7.52. As shown the measured LoA doesn't change considerably in both states shown in FIG. 15 and FIG. 16. However, the LoP is clearly reduced at higher depth of dissociation such as shown in FIG. 16 as compared to FIG. 15. In other words, the negative impact of the LoA on the LoP is reduced at a higher depth of dissociation, or any other suitable embodiment of a DoS. This could for example be used in an advantageous way by determining subjects and/or populations that could benefit from a treatment aimed at raising the depth of dissociation in order to indirectly manage pain, by reducing the any negative impact of the LoA.

FIG. 8 for example shows an embodiment of a representation of the measured DoSI=50%, LoAI=25% and LoPI=15%, and a NLoAI calculated to be 50% and a NLoPI calculated to be 40% at a reference DoSI that was set to 100%. Such a representation could for example be integrated in an embodiment of a GUI, similar as will be described below, and will for example allow a practitioner to assess during a treatment which involves a modified level of consciousness, how the level of anxiety or the level of pain will evolve after the treatment when the subject returns to a normal awake state, and/or vice versa. In such a user interface the reference DoSI could for example be implemented as a suitable, modifiable input parameter.

This is for example illustrated by means of Fig. 9, in which in a modified state of consciousness, for example the result of non-pharmacological treatment for modifying the level of consciousness as explained with reference to FIG. 1, however it is clear that alternative embodiments might be possible, for example involving pharmacological treatments modifying the state of consciousness, the DoSI has been reduced to 50% and there is measured a LoA of 25% and a LoPI of 15%, similar as in the example above with respect to FIG. 8. In a subsequent phase of the treatment, as shown, the subject is allowed to awaken and the measured DoSI rises until in the normal awake state it reaches for example 100%. As shown, during this awakening the measured LoAI will also rise from 25% until it reaches 50% in the normal awake state and the measured LoPI will also rise from 15% until it reaches 40% in the normal awake state. As further shown in Fig. 9, in line with the example explained above, when the reference DoSI has been set to 100%, or the normal awake state, then already in the modified state of consciousness, there can be provided, by means of the NLoAI, for example calculated as described above to be 50%, or the NLoPI, for example calculated as described above to be 40%, an indication of the LoAI or the LoPI that will be present when the DoSI will subsequently change to the reference DoSI. In other words, as shown in FIG. 9, for example under identical pain or anxiety conditions, NLoAI will remain constant at 50% and NLoPI remains constant at 40% independent of the changes of any DoSI. Additionally, calculation of NLoA and/or NLoP based on a predetermined reference DoS may allow for a comparison or use of the NLoA and/or NLoP between subjects/populations independent of their DoS, for example in the context of developing and/or determining suitable scales and/or indexes for LoA, LoP and/or DoS. It is however clear that still further alternative embodiments and uses of NLoA and NLoP are possible. More specifically, as already mentioned above in which according to alternative embodiments the NLoA is a value indicative for the level of anxiety in the subject at a predetermined reference LoP and/or a predetermined reference DoS, or embodiments in which the NLoP is a value indicative for the level of pain in the subject at a predetermined reference DoS and/or a predetermined reference LoA.

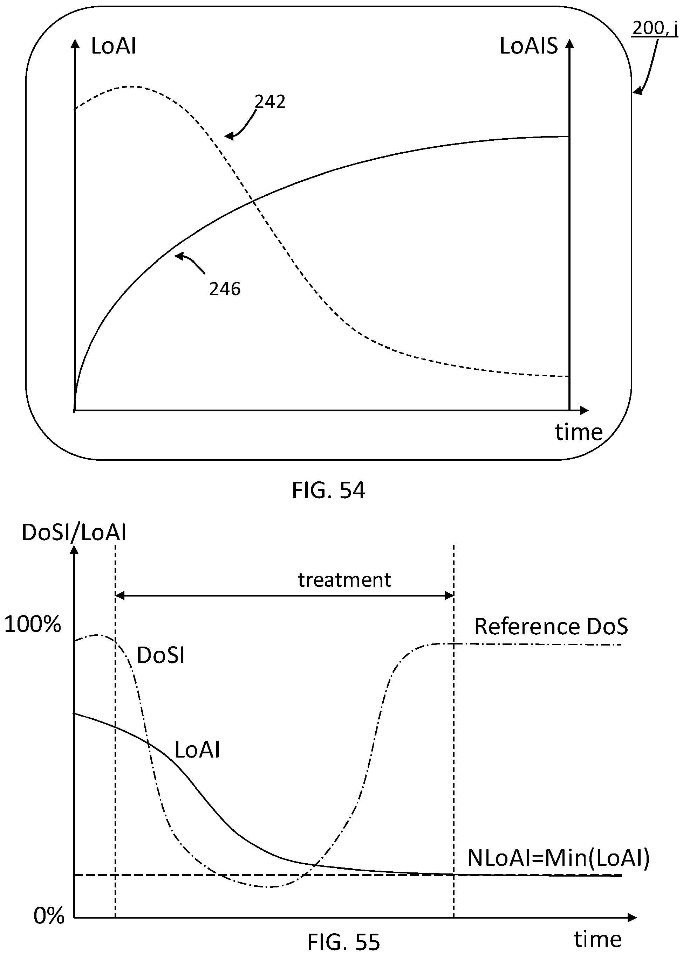

Specifically with respect to NLoA(I), alternative embodiments are possible with respect to the one shown in FIG. 9, where the LoAI raises again when the DoSI was raised, which is for example possible when a treatment modifying the state of consciousness only has a temporary effect on the LoA and/or when for example the anxiety inducing stimuli continue to be present after the treatment for modifying the state of consciousness. However, according to alternative embodiments, typically an anxiety management treatment will typically have a more lasting, long-term effect on the LoA, as for example shown in the embodiment of FIG. 55, and the NLoAI after the treatment might for example remain close to or equal the minimum LoA as attained during the treatment, which for example could be a treatment involving a modified state of consciousness, however alternative treatments, such as for example any suitable anxiety management treatments might be possible. As shown, LoA, when for example returning to a normal awake state of consciousness after the treatment might be lower than it was before the treatment. As shown, according to such an embodiment, in which the DoSI was sufficiently modified during the session, for example by a suitable level of absorption of the subject in a specific task, the LoAI, which is reduced during the treatment, would thus remain at such a lower level as compared to before the treatment. In this way the NLoAI after the treatment, for the reference DoSI could for example be calculated based on the LoAI before the treatment and the minimum value for DoSI and/or LoAI as attained during the treatment, or in any other suitable way.

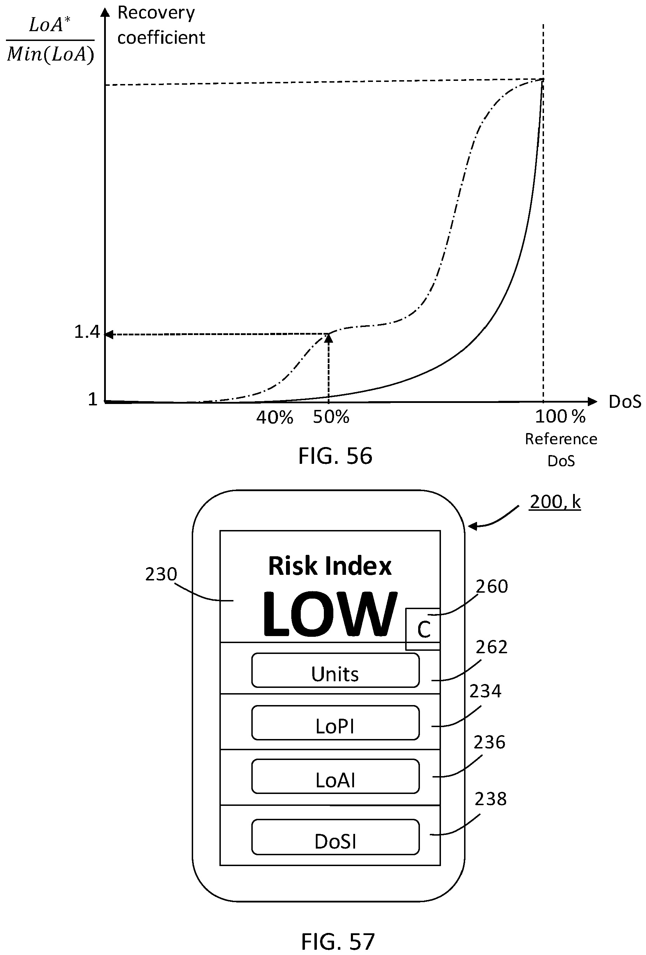

According to still a further embodiment, schematically illustrated by means of FIG. 56, especially in the embodiment of FIG. 55, there could further be created, for example a population based or subject based correlation between the DoS reached during a treatment session and a derived index, referred to as a LoA* indicator, which is configured as an indicator of how much LoA will increase after the treatment when returning to a predetermined reference DoS, which is for example associated with a normal awake state. A further derived index is referred to as the Recovery coefficient. As explained below, with reference to FIG. 56, this will for example allow for the calculation of the NLoA based on such a recovery coefficient. As for example shown by means of the dot-striped curve in FIG. 56, which shows an embodiment of such a recovery coefficient in the form of a predetermined ratio of LoA* / Min(LoA). This recovery coefficient has been correlated to the reference DoS for the NLoA. Min(LoA) is the minimum level of LoA reached during the treatment session. According to such an embodiment, as shown, a subject that reached a DoS of 50% during the treatment session will have a LoA after the session, which as shown in the embodiment of FIG. 54, at the reference DoS of 100% will be equal to the NLoA and which could be computed as Min(LoA) multiplied by the recovery coefficient correlated to this DoS of 50% during the treatment. According to the embodiment shown, NLoA will thus be Min(LoA) * 1.4. According to an alternative embodiment, the full line curve shows the such a correlation for an alternative treatment, and it is clear that for such a treatment, for a subject, that reached a DoS lower than 40% during the treatment session, the LoA after the session, or in other words the NLoA at the reference DoS could be computed as Min(LoA)*1, as the recovery coefficient is approximately 1. According to such an embodiment, similar as shown in FIG. 55, after the treatment, the LoA will not increase or remain substantially the same as the minimum LoA attained during the treatment.

It is further clear from the embodiment shown in FIG. 56, that when for example the treatment does not succeed at reducing DoS and/or reducing LoA during the treatment, then LoA after the treatment, or in other words the NLoA at the reference DoS will remain at the pre-treatment LoA*, as calculated from LoA* multiplied by LoA*/LoA* (as Min(LoA) = LoA*). In other words, according to such an embodiment the post-treatment LoA will remain at the pre-treatment LoA. It is clear that both embodiments of the correlations represented by the curves provide for a correlation between the DoS reached during the treatment session and a recovery coefficient indicating how the minimum LoA attained during the treatment session will evolve after the end of the treatment session. It is clear that numerous alternative embodiments of such correlations are possible, for example depending on treatment-based, subject-based and/or population-based parameters.

According to FIG. 6, this computer-implemented measurement of the LoA combined with DoS and/or LoP based on measurement data could be applied to measure, monitor and/or predict an evolution, such as for example a treatment induced evolution of the LoA, DoS and/or LoP. When for example a treatment is applied, such as for example an anxiety management treatment, a pain management treatment, or any other suitable treatment, the effect of this treatment on the LoA, DoS and/or LoP may be monitored by such an embodiment of the method. Such treatments might for example comprise any suitable treatment, such as non-pharmacological treatments, such as for example non-pharmacological anxiety treatments, non-pharmacological sedation, hypnosis, other evidence-based psychological and/or mind/body interventions, etc. , and/or pharmacological treatments, such as for example pharmacological anxiety treatments, pharmacological sedation, a treatment comprising administration of active pharmacological ingredient, administration of an analgesic, administration of an anxiolytic and/or any other suitable treatments such as for example medical or paramedical treatment such as surgery, physical therapy, electrical treatment, mechanical treatment, ... , and/or mind/body complementary or alternative medicines such as acupuncture, biofeedback, massage.... In this way FIG. 6 shows an embodiment of a computer-implemented method for measuring and/or monitoring a LoA, a DoS and/or LoP in a subject before, during and/or after a treatment 400. The embodiment of the method 400 shown in FIG. 6, comprises the steps of:

- measuring the LoA, DoS and/or LoP at a first point in time 402, for example before the treatment or at a first point in time during the treatment;
- measuring the LoA, DoS and/or LoP at a second point in time 404, for example after the treatment, or at a second point in time during the treatment later than the first point in time; and
- determining the treatment-induced evolution of the LoA, DoS and/or LoP 406.

The treatment, and its associated chronology, has been schematically illustrated by means of arrow 408 in the embodiment of FIG. 6, which could for example represent a singular treatment session with a well-defined start and end-point, however it is clear that alternative embodiments are possible in which the treatment for example has continuous and/or chronic nature without a well-defined start and end-point, or in which the treatment has an intermittent nature and comprises a sequence of a plurality of treatment sessions with optional breaks or pauses in between these treatment sessions, or any other suitable type of treatment comprising a chronological nature, for example comprising a sequence of treatment phases, steps, etc.

As shown at step 402 the LoA, DoS and/or LoP is measured, for example with the embodiment of the method for measurement of DoS and LoP 300 according to the embodiment shown in FIG. 5. This first measurement of LoA, DoS and/or LoP could for example be a measurement of the LoA, combined with DoS and/or LoP before the start of a treatment session and could, according to some embodiments be considered as a reference, initial, baseline, ... LoA, DoS and/or LoP respectively that serves as a predetermined reference level for subsequent measurements of LoA, DoS and/or LoP. As further shown in FIG. 6 at step 404, the second, subsequent measurement of LoA, DoS and/or LoP, is for example also measured with the method for measurement of LoA, DoS and/or LoP 300 shown in FIG. 5. This could for example be a measurement of the LoA, DoS and/or LoP during or after the treatment, for example an anxiety and/or a pain management treatment. At step 406 then there is determined the treatment-induced evolution of the LoA, DoS and/or LoP, based on a comparison of the first LoA, DoS and/or LoP and a respective second LoA, DoS and/or LoP, measured at steps 402 and 404. In this way the treatment-induced evolution of the LoA, DoS and/or LoP is be able to show a value representative for the effectiveness, intensity, desired effect, etc. of the treatment. It is thus clear that in the embodiment of FIG. 6, there is provided a computer-implemented method for measuring and/or monitoring and/or predicting and/or aggregating a level of anxiety, a level of pain and/or a level of a modified state of consciousness in a subject, for example before, during and/or after a treatment. In other words, there is measured and/or monitored and/or predicted and/or aggregated the LoA, the LoP and/or the DoS at at least two different points in time, for example before, during and/or after the treatment; and/or over a period of time. However, it is clear that alternative embodiments are possible in which the LoA, the LoP and/or the DoS are measured and/or monitored at at least two different points in time during any suitable time period.

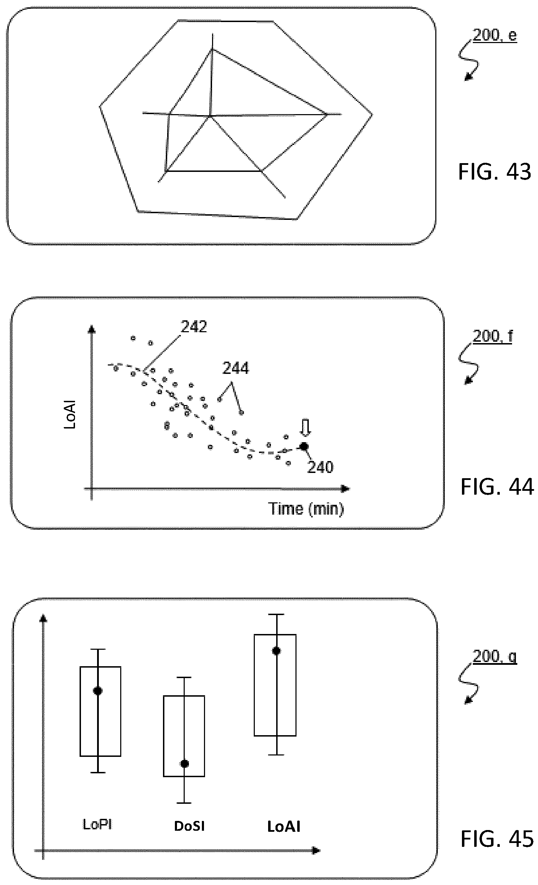

Based on a respective comparison of at least two LoA, at least two LoP and/or at least two DoS measured at at least two different points in time, there can then be measured and/or monitored a treatment-induced evolution of the LoA, the LoP and/or the DoS. According to a particularly simple embodiment the treatment-induced evolution of the LoA, the LoP and DoS is determined based on a respective comparison of the LoA, the LoP and DoS measured for example during and/or after the treatment with the LoA, the LoP and DoS respectively measured before the treatment. However, it is clear that such comparisons could be performed to determine any suitable evolution of the LoA, the LoP and DoS during any suitable time period. Additionally, and/or alternatively according to some embodiments the LoA, the LoP and/or the DoS could also be predicted. As for example described in further detail below with respect to Fig. 44 a suitable plurality of historical data points with respect to LoA, LoP and/or DoS could provide for a suitable trend analysis, for example based on a trend correlating to the a running average, mean, or any other suitable trend of the historical data points, thereby allowing prediction of future, expected or trending data points. It is clear that in this way a prediction of an evolution of the LoA, the LoP and the DoS can be accomplished. Predictive analysis of the historical data points, could be done by means of any suitable method, for example based on predetermined correlations between the evolution of LoA, LoP and/or DoS during different phases or time periods of a particular treatment, or by means of self-learning, self-optimizing algorithms, artificial intelligence algorithms, etc. which have been trained on historical data sets in order to detect repeating and/or predictable patterns and/or correlations, thereby allowing for an assessment of detectable patterns and/or correlations in the measured data, thereby allowing for a determination of current and predictive trends based on the measured data. It should be clear that alternative embodiments are possible of historical data points and/or any other suitable data resulting from LoA, LoP and/or DoS measurements and/or any other suitable measurement for enabling predictive analysis of current measurements. Such data on which the predictive analysis can be based can for example comprise historical data from individual subjects, from populations, predetermined treatment related patterns of an evolution of the LoA, LoP, DoS, etc.

According to still further embodiments, additionally and/or alternatively there may be aggregated a level of anxiety score or LoAS, a level of pain score or LoPS and/or a Depth of State Score or DoSS based on an aggregation of respectively at least two LoA, at least two LoP and/or at least two DoS measured at at least two different points in time. It is thus clear that according to such embodiments the aggregation of respectively at least two LoA, at least two LoP and/or at least two DoS measured could be performed over a predetermined period of time. Such aggregated parameters such as LoAS related to anxiety measurement could be useful in determining the impact of a chronic state of anxiety, which might be related to a personality trait and/or an anxiety disorder. An aggregated parameter such as a LoPS related to pain measurement could be useful in determining the impact of chronic pain, in which for example during a longer period, of for example several hours and/or days the level of pain is continuously measured, and the aggregated LoPS is able to provide for an indicator of the impact of a constant, chronic level of pain as perceived by a subject as opposed to an instantaneous peak of pain. This is useful as even moderate or low levels of anxiety and/or pain, when experienced chronically, may have a high impact on the quality of life of subjects, while for example short, infrequent bursts of anxiety and/or pain, even when they are intense can be tolerated more easily. Similarly, an aggregated DoSS may be useful in identifying the level of safety and stability associated with a modified level of consciousness during a particular time period. For example, during surgery, it is valuable to ascertain whether the modified level of consciousness has been established in a sufficiently stable way, and is not the result of a temporary, short term, evolution of the level of consciousness. It is clear that such aggregated forms could also be useful for any other parameters related to the level of pain and the state of consciousness as detailed in this description, and/or their scaled and/or indexed versions, such as for example LoA(I), LoP(I), DoS(I), NLoP(I), DoD(I), DoH(I), etc. thereby allowing for similar scores of these parameters that correspond to the aggregation of at least two values of these parameters. According to particular embodiments the LoA(I)S, LoP(I)S, DoS(I), NLoP(I)S, DoD(I)S, DoH(I)S, etc. could for example comprise any suitable embodiment of aggregation of the respective LoA(I),LoP(I), DoS(I), NLoP(I), DoD(I), DoH(I), etc. values over a predetermined time period, such as for example a sum, mean, average, etc. and the value could for example be presented in a GUI of a system similarly as described below, for example presenting the aggregated value, optionally in relation to the time period during which the LoA(I), LoP(I), DoS(I), NLoP(I), DoD(I), DoH(I), etc. was aggregated.

It is clear that still further alternative embodiments are possible of such methods for measuring and/or monitoring and/or predicting and/or aggregating a level of anxiety combined with and/or correlated with a level of a modified state of consciousness and/or a level of pain in a subject, preferably before, during and/or after a treatment, wherein the method comprises the steps of:

- measurement of the LoA combined with the DoS and/or the LoP at at least two different points in time, preferably before, during and/or after the treatment; and
- measuring and/or monitoring and/or predicting an evolution, preferably a treatment-induced evolution, of the LoA combined with the DoS and/or the LoP, based on a respective comparison of at least two LoA combined with and/or correlated with at least two DoS and/or at least two LoP measured at at least two different points in time; and/or
- aggregating a level of anxiety score or LoAS and/or a Depth of State Score or DoSS and/or a level of pain score or LoPS based on an aggregation of at least two LoA and/or at least two DoS and/or at least two LoP measured at at least two different points in time and/or over a period of time.

According to a specific embodiment the treatment-induced evolution of the LoA combined with DoS and/or LoP can be determined based on a respective comparison of the LoA combined with DoS and/or LoP determined during and/or after a treatment, with the LoA combined with DoS and/or LoP determined before a treatment. This means that the treatment induced evolution of the LoA is determined by a comparison of the LoA determined during and after the treatment according to this embodiment, and/or that the treatment induced evolution of the DoS, similarly is determined by a comparison of DoS determined during and after the treatment, and/or that the treatment induced evolution of the LoP is determined by a comparison of the LoP determined during and after the treatment.

According to a particular embodiment, similar as explained above with respect to the DoSLimit, LoALimit, and/or LoPLimit, when the treatment is a pain management treatment, the pain management treatment could be replaced and/or combined with an anxiety management treatment; and/or when the treatment is an anxiety management treatment, the anxiety management treatment could be replaced and/or combined with a pain management treatment, when the LoP and/or the LoA is outside the predetermined range and/or until the LoP and/or the LoA is inside the predetermined range. According to such an embodiment, when the LoA is too high, this might limit the effect of a pain management treatment, so when for example the LoA is outside a predetermined range, for example by exceeding one or more predetermined limits associated with the inability to further efficiently manage the LoP by means of a pain management treatment. In such a case, it is beneficial to replace and or combine the pain management treatment with an anxiety management treatment, at least until the anxiety management treatment was able to provide for a treatment induced evolution of the LoA, for example by suitably reducing the LoA to within predetermined limits, such that the LoA is again in the predetermined range in which a pain management treatment is known to be effective for managing the LoP upon which the pain management treatment can be continued and/or resumed and optionally the anxiety management treatment can be stabilized, reduced and/or ended. Similarly, as described above, according to an embodiment when the treatment is an anxiety management treatment, the anxiety management treatment can be replaced with a pain management treatment when the LoP and/or the LoA is outside a predetermined range and/or until the LoP and/or the LoA is inside the predetermined range. This will ensure that, when for example the LoP is exceeds a predetermined limit and/or threshold for any anxiety management treatment to be effective, for example first the LoP is reduced till the predetermined limit is no longer exceeded, or until it is within any suitable predetermined range for the anxiety management treatment to be effective in managing the LoA again. It is clear that still further embodiments are possible in which any suitable combination of an anxiety management treatment and/or a pain management treatment and/or a treatment involving a modification of the state of consciousness are used in order to ensure that LoP and/or LoA and/or DoS are respectively managed such that they become and/or remain within a respective predetermined range for enabling any further treatment to be efficient. According to still a further embodiment, when the treatment is a treatment modifying the state of consciousness it might for example be beneficial to replace and/or combine the treatment modifying the state of consciousness with an anxiety and/or pain reducing treatment when the LoP and/or LoA is outside a predetermined range and/or until the LoP and/or the LoA is inside the predetermined range.

According to a further particular embodiment, when the treatment is a pain management treatment, the pain management treatment can be replaced and/or combined with a treatment modifying the state of consciousness when the DoS is outside a predetermined range and/or until the DoS is inside the predetermined range.

Similarly, the treatment modifying the state of consciousness can be replaced and/or combined with a pain management treatment when the LoP is outside a predetermined range and/or until the LoP is inside the predetermined range. According to an alternative embodiment, the efficacy of a pain management treatment, such as for example a pain-reducing, pain-controlling, pain-preventing, or any other suitable pain management treatment can be determined and/or monitored based on the treatment-induced evolution of the NLoP. Similarly the efficacy of an anxiety-reducing treatment can be determined and/or monitored based on the treatment-induced evolution of the NLoA.

When the treatment is a treatment modifying the state of consciousness for reducing anxiety, such as for example a non-pharmacological treatment such as a hypnosis treatment session, a mind-body treatment, cognitive behavioral therapy, etc. , or a pharmacological treatment, for example a sedative treatment, by measuring the DoS at at least two different points in time, for example before, during and/or after the treatment modifying the state of consciousness; there can be determined a treatment-induced evolution of the DoS and/or the LoA, based on a comparison of for example the DoS and/or the LoA measured during and/or after the treatment modifying the state of consciousness with the DoS and/or the LoA measured before the treatment modifying the state of consciousness.

When the treatment is a treatment modifying the state of consciousness for reducing pain, such as for example a non-pharmacological treatment such as a hypnosis treatment session, or a pharmacological treatment, for example a sedation treatment, by measuring, the DoS at at least two different points in time, before, during and/or after the treatment modifying the state of consciousness; there can be determined the treatment-induced evolution of the DoS and/or the LoP, based on a comparison of the DoS and/or the LoP measured during and/or after the treatment modifying the state of consciousness with the DoS and/or the LoP measured before the treatment modifying the state of consciousness.

Alternatively, in order to determine the efficacy of treatment, independent of any changes to the level of consciousness, for example to allow for a comparison between subjects and/or populations, for example to determine/adjust titration or dosage regimen, or to determine/adjust the required depth of state during a hypnosis treatment session, there can be determined a treatment and/or determining an adjustment to a treatment and/or determining an intensity of a treatment based on the determined and/or monitored NLoA and/or NLoP and/or DoSLimit and/or LoPLimit and/or LoALimit respectively. This then allows for example for a determination of for example the efficacy of the treatment with respect to the level of anxiety and/or the level of pain respectively, independently of any changes to the state of consciousness of the subject. It for example allows an anesthetist to assess the LoA independent of any changes to the DoS for example prior to and/or during the initial phases of a sedative treatment, and/or to assess the LoP a sedated patient will experience when brought back to a normal awake state, prior to bringing this sedated patient back to the normal awake state. This then allows, to provide for a suitable anxiolytic, analgesic and/or sedative treatment plan to be set up and implemented prior to the sedative treatment and/or prior to awakening of the patient from the sedated state. It is clear that still further alternative embodiments of the use of the NLoA and/or the NLoP are possible. According to further embodiments this for example allows to determine a treatment and/or an adjustment to a treatment and/or an intensity of a treatment and/or the efficacy of a treatment and/or the choice of a treatment based on the measured and/or monitored and/or predicted and/or aggregated NLoA and/or NLoP and/or DoSLimit and/or LoPLimit and/or LoALimit.

If the treatment, for example is an anxiety management treatment, then a treatment-induced evolution of the LoA, which for example corresponds to a reduction of the second LoA measured at step 304 as compared to the first LoA measured at step 302 and treatment-induced evolution of the DoS that for example corresponds to a decrease of the second DoS measured at step 304 as compared to the first DoS measured at step 302, is able to confirm the effectiveness of the treatment. According to some embodiments the value of the treatment-induced evolution of the DoS and LoA also provides for an indication of the intensity of the treatment. For example, according to an embodiment, an anxiety management treatment reduces a first LoA measurement with a first value at step 402 to second value, that corresponds to a 50% reduction of the first value, at a second measurement at step 404, which thus corresponds to a treatment-induced evolution of the LoA of -50% or in other words a change in the LoA corresponding to a reduction of 50% with respect to the measurement at step 302 before the treatment. When this treatment is adapted or another treatment is applied, which only causes a treatment induced evolution of the LoA of -25%, it is clear that this latter treatment has an intensity that is lower than the former. When no change is detected with respect to the first LoA measurement, it can be concluded that the treatment-induced evolution of the LoA corresponds to no change to the LoA, or in other words, that the treatment at that point in time is for example not effective in reducing the LoA and might need adaptation or change to another type of treatment. Similarly, a treatment induced evolution of the DoS could also be quantified to indicate the intensity of the treatment, preferably in combination with the LoA. However, according to alternative embodiments, in which for example the treatment comprises the application of anxiety inducing stimuli, such as for example during a medical treatment involving an injection, administration of local sedation, etc. when the LoA as measured before the treatment, and/or the DoS attained during a particular phase of the treatment, does not change, does not increase and/or does not increase outside a predetermined desired range, during the treatment, especially during and/or after the application of the anxiety inducing stimuli, it is clear that the anxiety management treatment, for example in the form of pharmacological and/or non-pharmacological anxiety management treatment is performing effectively.

If the treatment, for example is a pain reducing treatment, then a treatment-induced evolution of the LoP that for example corresponds to a reduction and/or the absence of an undesired increase of the second LoP measured at step 304 as compared to the first LoP measured at step 302 and treatment-induced evolution of the DoS that for example corresponds to an decrease of the second DoS measured at step 304 as compared to the first DoS measured at step 302, is able to confirm the effectiveness of the treatment. According to some embodiments the value of the treatment-induced evolution of the DoS and LoP also provides for an indication of the intensity of the treatment. For example, according to an embodiment, a pain reducing treatment reduces a first LoP measurement with a first value at step 402 to second value, that corresponds to a 50% reduction of the first value, at a second measurement at step 404, which thus corresponds to a treatment-induced evolution of the LoP of -50% or in other words a change in the LoP corresponding to a reduction of 50% with respect to the measurement at step 302 before the treatment. When this treatment is adapted or another treatment is applied, which only causes a treatment induced evolution of the LoP of -25%, it is clear that this latter treatment has an intensity that is lower than the former. When no change is detected with respect to the first LoP measurement, it can be concluded that the treatment-induced evolution of the LoP corresponds to no change to the LoP, or in other words, that the treatment at that point in time is for example not effective in reducing the LoP and might need adaptation or change to another type of treatment. Similarly, a treatment induced evolution of the DoS could also be quantified to indicate the intensity of the treatment, preferably in combination with the LoP. However, according to alternative embodiments, in which for example the treatment comprises the application of painful stimuli, such as for example during a surgical treatment, when the LoP as measured before the treatment, and/or the DoS attained during a particular phase of the treatment, does not change, does not increase and/or does not increase outside a predetermined desired range, during the treatment, especially during and/or after the application of the painful stimuli, it is clear that the pain treatment, for example in the form of pharmacological and/or non-pharmacological sedation is performing effectively.

As schematically shown by means of striped line 410, according to an optional embodiment, the measurement of a further LoA combined with and/or correlated with a DoS and/or LoP at step 404, at a later point in time than at step 402, could be repeated or could be performed continuously, which allows for a repeated and/or continuous monitoring of the treatment-induced evolution of the LoA, DoS and/or LoP, or in other words the comparison of two LoA, DoS and/or LoP respectively measured at two different points in time. It is clear that alternative embodiments are possible in which any suitable plurality of LoA, DoS and/or LoP measurements at different points in time, before, during and/or after the treatment, are used in order to determine and/or monitor a treatment-induced evolution of the LoA, DoS and/or LoP, based on a comparison of these at least two LoA, DoS and/or LoP measured at at least two different points in time. It is thus clear that such a treatment induced evolution of the LoA, DoS and/or LoP is indicative of a treatment induced change, if any, to the LoA, DoS and/or LoP.

In addition to the group 4 indicator mentioned above with respect to the experiment 500 as shown in FIG. 1 and the application of the results of this experiment in the computer-implemented methods of Figures 5 and 6, the LoP can optionally be determined by means of additional indicators, such as for example a group 3 indicator or a group 5 indicator as described in further detail below.

According to such an embodiment, during the embodiment of the experiment as shown in FIG. 1, in addition to the group 4 indicator, there is for example extracted from the measured EEG data a group 3 indicator in the time domain, for example by means of a suitable event related potential, or ERP, in response to the calibrated pain stimulus.

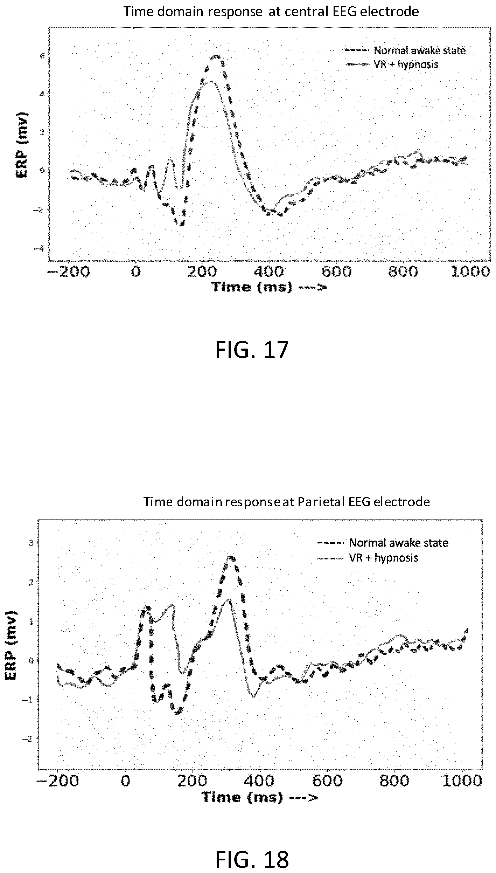

Such a time-domain EEG analysis showed an overall decreased in the ERP components during the hypnotic treatment session 540 as compare to the normal awake state 530. Based on the measured EEG data, there was found a difference in overall mean signal peak-to-peak amplitude, or MSPA, time-locked to the painful stimulus onset. According to a particular embodiment, as shown, in FIG. 17, 18 and 19, the time window could for example be set from 300ms to 350ms after the painful stimulus onset, which is represented as 0ms on the x-axis. However it is clear that alternative embodiments are possible in which the MSPA is measured on an ongoing basis, for example by means of a suitable, rolling time window, not linked or triggered by an external pain stimulus. In case there is only a single peak in the time window for measurement of the MSPA, the MSPA is equal to the peak-to-peak amplitude of the signal. In case there are multiple peaks in the signal in the time window for measurement of the MSPA, the MSPA will be calculated in function of the mean or average of the individual peak-to-peak amplitudes of the plurality of these peaks of the signals. The difference in this embodiment of the ERP amplitude between the 2 sessions 530, 540 of the first phase 520, in other words between the normal awake state 530 and the hypnotic treatment session 540 that induces a different level of pain as perceived by the subject, was found to be significant at least at the following 3 EEG measurement locations:

- at an EEG electrode positioned at the central lobe (C), for example along the midsagittal plane: there was found a decrease in overall MSPA during the hypnotic treatment session 540 as compared to the normal awake state 530, in other words the C-MSPA EEG measurement data shows a decreased involvement in the response to the calibrated pain stimulus;
- at an EEG electrode positioned at the parietal lobe (P), for example along the midsagittal plane: there was found a decrease in overall MSPA during the hypnotic treatment session 540 as compared to the normal awake state 530, in other words the P-MSPA EEG measurement data shows a decreased involvement in the response to the calibrated pain stimulus;
- at an EEG electrode positioned at frontal lobe (F), for example along the midsagittal plane: there was found a decrease in overall MSPA during the hypnotic treatment session 540 as compared to the normal awake state 530, in other words the P-MSPA EEG measurement data shows a decreased involvement in the response to the calibrated pain stimulus.

The study based on this experiment is thus showing statistically significant MSPA changes at those specific locations of the EEG measurement data, when comparing a modified state of consciousness for example during a non-pharmacologically, e.g. hypnotically induced treatment session, with a normal awake state 530, and which correlate to the different level of pain perceived in reaction to a calibrated painful stimulus.

The extraction of the mean signal peak-to-peak amplitude, or MSPA, for electrodes F, C and/or P, from the EEG data of the measurement data of the experiment 500, which can also be referred to as group 3 indicators, provided the following results:

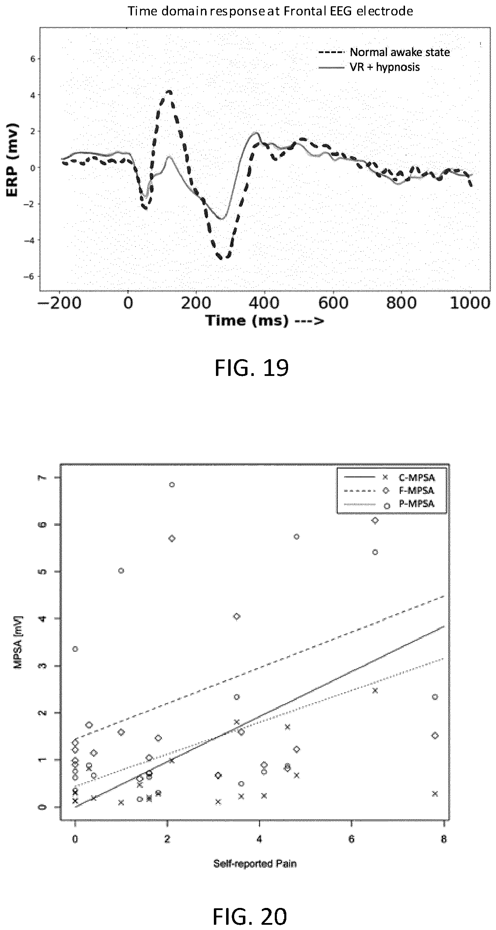

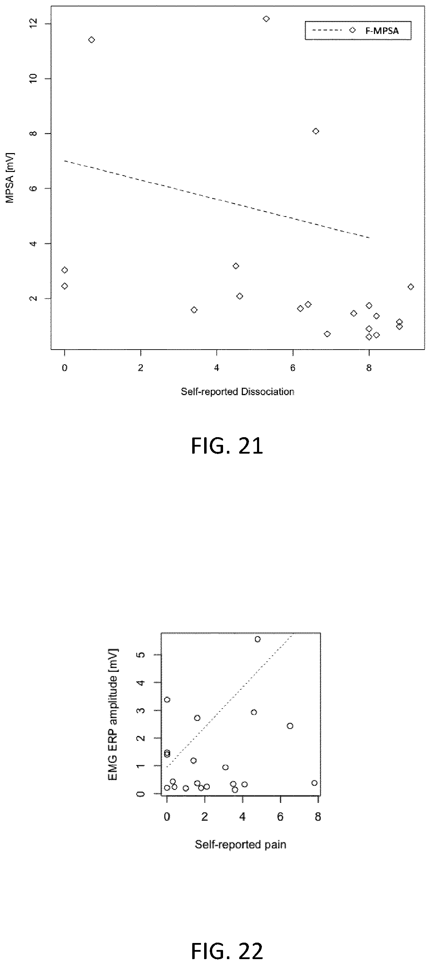

- The MSPA extracted from the EEG data, or the group 3 indicator, is significantly lower during the hypnotic treatment session 540 as compared to the normal awake state 530 at at least the following 3 EEG electrode locations: at EEG electrode location F there was extracted a F-MSPA value of 24.13 mV during the normal awake state 530 versus an extracted a F-MSPA value of 11.16 mV during the hypnotic treatment session, p-value = 0.019, at EEG electrode location C there was extracted a C-MSPA value of 8.67 mV during the normal awake state 530 versus an extracted a C-MSPA value of 2.94 mV during the hypnotic treatment session 540, p-value = 0.037, and at EEG electrode location P there was extracted a P-MSPA value of 5.159 mV during the normal awake state 530 versus an extracted a P-MSPA value of 2.635 mV during the hypnotic treatment session 540, p-value = 0.037. Similar as above, it is thus clear that the group 3 indicator is an indicator for the level of pain as consciously perceived by the subject, as the group 3 indicator is modified by a change of the state of consciousness under identical pain conditions of the experiment, in which an identical calibrated pain stimulus was applied as described above.
- The self-reported measure of pain is correlated (spearman) with an embodiment of the group 3 indicator, or in other words the MSPA extracted from the EEG measurement data, at at least the following 3 EEG electrode locations: F (rs = 0.38, p = 0.097), C (rs = 0.407, p = 0.074) and/or P (rs = 0.34, p = 0.095). FIG. 20 shows a graph indicating the correlation between the group 3 indicator, or in other words the MSPA extracted from the measured EEG data at the 3 EEG electrodes F, C and P and pain during the experiment 500.
- The self-reported measure of dissociation is correlated (spearman) with an embodiment of a group 2 indicator, that corresponds to the MSPA extracted from the measured EEG data at electrode location F (rs = -0.41, p = 0.07), or in other words the F-MSPA. FIG. 21 shows a graph indicating the correlation between MSPA measured at the EEG F-electrode and dissociation.

As already mentioned above in relation to the signals shown in FIG. 17, 18 and 19, in case there is only a single peak in the time window for measurement of the MSPA, the MSPA is equal to the peak-to-peak amplitude of the signal. In case there are multiple peaks in the signal in the time window for measurement of the MSPA, the MSPA will be calculated in function of the mean or average of the individual peak-to-peak amplitudes of the plurality of these peaks of the signals. The time window for measurement could for example be in the range of 0.01s to 10s, such as for example in the range of 0.1s to 5s.

The study of the experiment 500 mentioned above, thus shows significant MSPA changes in the measured EEG data at those specific EEG sensor locations when comparing a modified state of consciousness during a hypnotic treatment session 540, or any other suitable non-pharmacological or pharmacological treatment inducing a modified state of consciousness, with a normal awake state 530, or in other words when comparing different levels or depths of dissociation or DoD, which is an embodiment of different depth of states or DoS. It is clear that this also means that significant MSPA changes in the measured EEG data have been observed when comparing different levels of pain as perceived by the subject, as the level of pain perceived by the subject during the normal awake state 530 differed from the level of pain perceived by the subject during the hypnotic treatment session 540.

Similar as described above, the insights of the experiment 500 above allow for an alternative embodiment of a computer-implemented method 300 for measurement of a level of pain comprising the further step of extracting from the EEG data, a group 3 indicator corresponding to a mean signal peak-to-peak amplitude, MSPA. Then at a subsequent step the LoP is determined based on the group 4 indicator and at least the group 3 indicator. It is clear that similarly an alternative embodiment of the computer-implemented method 400 for determining and/or monitoring a level of anxiety, a level of modified consciousness and/or a level of pain of a subject, comprising this alternative method for measurement of a level of anxiety, a level of modified consciousness and/or a level of pain is possible.

According to still a further embodiment there is provided a computer-implemented method 300 for measurement of a level of pain comprising the further step of receiving measured data comprising muscular activity data, such as for example electromyography, EMG, data, for example collected from one or more EMG electrodes or any other suitable electrode. These EMG electrodes could for example be positioned at one or more of the following positions: a leg, face, arm, or any other suitable position, .... According to still further embodiments it is possible to derive EMG signals and/or EMG data from signals and/or data received from any other suitable electrodes, such as for example EEG electrodes, etc. During a subsequent step extracting from the EMG data, a group 5 indicator corresponding to a mean signal peak-to-peak amplitude, EMG-MSPA. Subsequently then determining the LoP based on the group 4 indicator and at least the group 5 indicator.

Such an embodiment is made possible by means of the following insights resulting from the embodiment of the experiment 500 mentioned above, in which EMG measurement data resulted from suitable measurement of EMG signals during the test phase 520. There was found a decreased MSPA of the ERP-EMG triggered by the pain stimulus during the hypnotic treatment session 540 as compared to the normal awake state 530. There was also found a correlation between the ERP-EMG triggered by the pain stimuli between the EMG-MSPA and the self-reported level of pain perception (r= 0.387, p-value = 0.09). It was further found that a wider ERP-EMG was associated with an increased self-reported level of pain perception. FIG.22 shows the correlation between the self-reported level of pain perception and the EMG-ERP MSPA extracted from the EMG measurement data during the experiment 500.

According to a preferred embodiment of the method 300 the LoP is determined based on the group 4 indicator, the group 3 indicator, and the group 5 indicator.

Similar as described above, according to a preferred embodiment, the method for measuring the LoA, DoS and/or LoP 300 comprises the step of receiving the measured data of the subject comprising the electroencephalogram, EEG, data collected from one or more of the following EEG electrodes:

- a frontal (F) EEG electrode configured for collection of F-EEG electrode data from the scalp anatomical region corresponding to a frontal lobe of the subject,
- a parietal (P) EEG electrode configured for collection of P-EEG electrode data from the scalp anatomical region corresponding to a parietal lobe of the subject,
- a central (C) EEG electrode configured for collection of C-EEG electrode data from the scalp anatomical region corresponding to a precentral and postcentral gyrus of the subject.

Such EEG data is related to brain waves generated by parts of the brain close to the scalp, including the cortex, and therefor are related to the conscious perception of the level of pain by the subject.

Electroencephalography (EEG) is a technique well known in the art for recording electrical activity of the brain. EEG Electrodes placed along the scalp at certain locations measure voltage fluctuations resulting from electrical impulses within the neurons of the brain, mainly in the region of the brain close to the scalp, including the cortex. EEG measurement data reflects how different neurons/ populations of neurons in the brain networks communicate with each other by means of electrical impulses. Electroencephalography produces an electroencephalogram. The electroencephalogram, which thus forms the EEG data, may be refined to a particular electrode location, such as the frontal, parietal or central electrodes mentioned above. From the EEG data there may for example be extracted a frequency-based parameter and/or a phase-based parameter, and/or amplitude-based parameter. Frequency-based parameters may for example be obtained by a frequency-based analysis of EEG measurement data. Such a frequency based analysis of EEG measurement data may for example be used to measure the power contained in a specific signal frequency band, where power refers to a square of amplitude of the frequency domain signals typically within a specified in a frequency band or range, such as for example a frequency band spanning or within a delta-theta frequency range, or a frequency band spanning or within a theta-alpha frequency range.

The "delta-theta" (dt) frequency band refers for example to a band of frequencies in a range encompassing both delta and theta brain waves. The delta-theta (dt) frequency band is typically greater than 0 Hz and equal to or less than 8 Hz. The theta brain waves are preferably slow theta brain wave, for example greater than 3 Hz and equal to or less than 6 Hz. The width of the delta-theta (dt) frequency band may be at least 2 Hz. Most preferably the delta-theta (dt) frequency band is greater than or equal to 1 Hz and less than or equal to 6 Hz. In other words, the delta-theta (dt) frequency band comprises and preferably consists of a combination of at least part of the delta frequency band and at least part of the theta frequency band.

The "theta-alpha" (ta) frequency band refers to a band of frequencies in a frequency range encompassing both theta and alpha brain waves. The theta-alpha (ta) frequency range is typically greater than or equal to 4 Hz and equal to or less than 12 Hz. The theta brain waves are preferably fast theta brain waves, for example greater than 6 Hz and equal to or less than 8 Hz. The width of the theta-alpha (ta) frequency band may be at least 2 Hz. Most preferably the theta-alpha (ta) frequency band is greater than 6 Hz and less than or equal to 12 Hz. In other words, the theta-alpha (ta) frequency band comprises and preferably consists of a combination of at least part of the theta frequency band and at least part of the alpha frequency band.

A phase-based parameter may be obtained by phase-based analysis of EEG measurement data. An amplitude-based parameter may be obtained by amplitude -based analysis of EEG measurement data, such as for example a mean signal peak-to-peak amplitude or MSPA in a time domain. The signal peak amplitude is the maximum excursion of the signal wave from the zero point. The signal peak-to-peak amplitude is the distance from a negative peak to a positive peak.

During the experiment 500 a subject may experience during the hypnotic treatment session 540 an increased activity in the frontal cortex and/or in the frequency range of delta waves and a decreased activity in the frontal and/or occipital network and/or in the frequency range of alpha waves, as compared to the activity during the normal awake state 530.

The EEG data may be acquired with one or more EEG electrodes placed on the scalp in the anatomical regions corresponding to the position of the frontal lobe, the parietal lobe and/or both precentral and postcentral gyrus.

A frontal EEG electrode or F-EEG electrode is located on the scalp anatomical region corresponding to the frontal lobe. It is configured for attachment to or contact with the scalp anatomical region corresponding to the frontal lobe. It records F-EEG electrode data. The frontal lobe refers to the anatomical part of the brain. The region of the frontal lobe may be along the sagittal plane, more preferable to in middle of the scalp anatomical region corresponding to the frontal lobe, along the midsagittal plane.

A parietal EEG electrode or P-EEG electrode is located on the scalp anatomical region corresponding to the parietal lobe. It is configured for attachment to or contact with the scalp anatomical region corresponding to the parietal lobe. It records P-EEG electrode data. The parietal lobe refers to the anatomical part of the brain. The region of the parietal lobe may be along the sagittal plane, more preferable to in middle of the scalp anatomical region corresponding to the parietal lobe, along the midsagittal plane.

A central EEG electrode or C-EEG electrode is located on the scalp anatomical region corresponding to the precentral and postcentral gyrus. It is configured for attachment to or contact with the scalp anatomical region corresponding to the precentral and postcentral gyrus. It records C-EEG electrode data. The precentral and postcentral gyrus refers to the anatomical part of the brain. The region of the precentral and postcentral gyrus may be along the sagittal plane, more preferable to in middle of the scalp anatomical region corresponding to the precentral and postcentral gyrus, along the midsagittal plane.

It is clear that alternative embodiments are possible in which in one or more EEG electrodes are arranged at the above positions, and or additional suitable scalp anatomical regions. As a minimum one localized EEG electrode may be used, such as for example positioned at the scalp anatomical region of the frontal lobe. However, it is understood that a second electrode, or ground electrode may be employed for common mode rejection, i.e. to dissociate relevant information from noise; this can be placed anywhere on the scalp, for instance in another region of the scalp, in a bilateral position. A third electrode, or reference electrode, may be employed to compute voltage differences. This third electrode can be placed anywhere on the scalp, for instance at an electrically neutral location.

It is thus clear that the measured data may comprise one or more measured data components, such as for example EEG data, which is a measured data component, such as for example also EMG data, ECG data, EOG data, ....

In addition to the measured data, the embodiments of the experiment 500, method for measurement of the LoA combined with DoS and LoP 300, etc. may in addition to measured data optionally also take into account observational data and/or self-reported data. In other words, these methods may make use of response data that may comprise measured data and optionally observational data and/or self-reported data. The response data may comprise measured data and/or observational data and/or self-reported data. The response data contains one or more data components, each component being derived from a single measurement (e.g. EEG, EMG, EDA, ECG, EOG), single observation (e.g. movement, skin color), or single self-reported event such as for example a self-reported level of anxiety, a self-reported level of pain or a self-reported level of dissociation as mentioned above.

It is clear that the measured data is data obtained from the subject using one or more devices such as an electrode and/or sensor, such as for example a transducer, camera, motion sensor, ... disposed in relation to the subject. Observational data is then data obtained from a non-self, or hetero, observation of the subject. Self-reported data is data or information reported by the subject, for example before, during or after the measurement of the LoA, LoP, DoS and/or the treatment.

According to particular embodiments the response data may comprise electrical activity data. Electrical activity data is measured data. Electrical activity data is any data derived from a measurement obtained by an electrical electrode. The electrode is typically placed on the skin. Usually the data is captured by at least 2 electrodes (e.g. 2, 3, 4, 5 or more). Examples of electrode-captured data include electroencephalogram (EEG) data, electromyography (EMG) data, electrodermal activity (EDA) data, galvanic skin response (GSR), electrocardiogram (ECG) data, electrooculogram (EOG) data. It is preferred that the response data comprises at least EEG data. Any electrical data component (e.g. EEG data) may or may not be processed prior to being used as measured data and/or response data.

According to particular embodiments the response data may comprise physiological data. Physiological data is measured data. Physiological data is data derived from a measurement on the subject obtained by a device usually containing a transducer, camera, motion sensor. Physiological data excludes the above-mentioned electrical activity data. Examples of physiological data include pulse rate data, cardiovascular data (e.g. heart rate, heart rate variability data), blood pressure data, respiratory (rate) data (e.g. respiratory rate, respiratory rate variability data), brain oxygenation data, blood $O_2$ saturation data, regional and/or central blood $O_2$ saturation data, body temperature data, photoplethysmogram (PPG) data. Blood oxygen saturation may be, for instance SpO2, SvO2, and the like. Any physiological data component (e.g. pulse rate data) may or may not be processed prior to being used as response data. It is clear that, according to some embodiments, physiological data could be derived from electrical activity data, such as for example cardiovascular data could for example be derived from ECG data, etc.

According to exemplary embodiments in which the response data further comprises cardiovascular data such as for example ECG, heart rate, heart rate variability, etc. and/or respiratory data, such as for example breathing rate, breathing rate variability, etc. , the LoP can further be determined in function of such response data, as during the experiment it was detected that:

- Heart rate variability was significantly higher during painful stimulation (mean 137 ms, standard deviation 64) as compared to rest (mean 97 ms, standard deviation 10) (p-value = 0.017); and
- Breathing rate was significantly lower during painful stimulation (mean 9.8 cycles/sec, standard dev 1.2) as compared to rest (mean 11.06 cycles/sec, standard deviation 1.16) (p-value = 0.032).

According to particular embodiments the response data may comprise motion-tracking data. Motion-tracking data is measured data. The motion tracking data is data derived from a movement of the body or of a part of the body. Typically, motion tracking data is measured by one or more motion sensors (e.g. a 2- or 3-axis accelerometer, gyroscope, one or more cameras). The motion-tracking data may comprise body one or more of body tracking data (e.g. head, limb (arms legs hands), bodily shaking) and/or eye tracking data. The motion may be spontaneous and/or as a result of a stimulation.

According to particular embodiments the response data may comprise facial expression data. Facial-expression data is measured data. The facial expression data is data relating an emotion and/or pain perception. Typically, facial expression data is measured by one or more cameras directed at the face (or EMG sensors).

According to particular embodiments the response data may comprise observational data. The observational data is data observed about the subject by another person. The observational data is data observed by the user (e.g. care provider such as a physician, or medical assistant, or non-medical assistant (e.g. friend, relative, helper)), or by a stakeholder associated with the user (e.g. hospital), or obtained from a database (e.g. medical records). The observed data may comprise one or more observed data components (e.g. a subject's movements is an observed data component). Observational data may include one or more of subject's movements/ lack of movement; procedural events; clinical observation (skin color, groaning or verbalization of discomfort...); age, type of surgery, ethnic origin, language; dosages of sedation drugs.

According to particular embodiments the response data may comprise self-reported data. The self-reported data is data reported by the subject. The self-reported data may for example be provided during a treatment session. Preferably, the self-reported data is provided before, during and/or after the treatment session. The self-reported data may comprise one or more self-reported data components (e.g. level of pain during measurement, or during treatment is a self-reported data component). Self-reported data may include one or more of a self-reported level of pain, for example before, during and/or after treatment, a self-reported level of dissociation before, during and/or after treatment, estimated duration of the procedure, recall of the events during the session, ....

According to embodiments the muscular activity data comprises EMG measured data, EMG data may be acquired using two or more EMG electrodes placed on the skin, preferably on limbs or face; or derived from another sensor like EEG. It represents electrical activity data from the subject muscle tissue. It may comprise a frequency-based parameter and/or a phase-based parameter and/or an amplitude-based parameter. However, it is clear that according to alternative embodiments EMG measured data, might for example be extracted from any suitable electrodes, such as for example from signals measured by EEG electrodes.

According to further embodiments the following measured data my optionally and/or additionally be captured and processed during alternative embodiments of the methods described above and/or below. Cardiovascular measurement data may be acquired using two or more electrocardiogram (ECG) electrodes placed on the skin using peripheral or precordial placement, or using any other placement where ECG data can be derived. ECG detects electrical activity of the heart. Alternatively or in addition, cardiovascular data may be acquired using a photoplethysmogram (PPG) sensor placed on the skin. PPG detects blood volume changes vasculature below the skin. cardiovascular data may comprise heart rate, heart rate variability, derived metrics, phase-based parameters, frequency-based parameters. However, it is clear that according to alternative embodiments ECG measured data, might for example be extracted from any suitable electrodes, such as for example from signals measured by EEG electrodes.

Respiratory measurement data may be acquired using one or more force/movement transducer (e.g. chest band) or air pressure sensor, or derived from a PPG sensor or from ECG electrodes placed at location where respiratory rate is detectable. The respiratory data may comprise one or more of respiration rate, respiration rate variability (also known as respiration variability), inhalation pressure, exhalation pressure, respiratory rhythm, respiratory depth, respiratory pattern and derived indexes. Changes of respiration patterns may for example also indicate changes to the LoP.

Respiratory data may be known herein as respiratory rate data.

The eye movement measurement data or eye tracking measurement data may include one or more of pupil dilation, eye fixation, blink rate or frequency, eye-EOG, eye movement, blink closing duration, blink flurries, eye closure rate, eyelid distance change (with respect to the normal eyelid distance). Pupil dilation can give information about the activity in the coeruleus, the main site of norepinephrine (stress hormones) synthesis in the brain, as well as the state of sleepiness/ alertness.

Eye movement data, such as for example electrooculogram (EOG) measurement data may be acquired using two or more EOG electrodes placed on the skin around the eye (*e.g.* above and below, or to the left and right of the eye). EOG records eye movements based on the corneo-retinal standing potential that exists between the front and the back of the human eye. EOG data may comprise eye movements/fixation, eye blinks, synchronization of both eye's movements.

Skin galvanic measurement data may be acquired using two or more conductance-measuring electrodes placed on the skin. The data represents skin conductivity and is linked to changes in sweat glands activity that are reflective of sympathetic activity. Skin galvanic data may comprise conductivity variability, peak amplitude/latency, trends.

SpO2 measurement data is a measure of blood oxygen saturation. It may be measured using a number of techniques such as pulse oximetry. SpO2 data may comprise timeline values. It is a key parameter for anesthesiologists since it is linked to respiratory function and it reflects tissue oxygenation.

The body motion tracking measurement data (e.g. head, limb (arms legs hands)) may include an indication of increased or reduced body movement. A change in motion of the head may be a sign of lack of concentration in the tasks/ low immersiveness/ distraction during passive "deepening" phase of the treatment session. It is clear that alternative embodiments are possible, for example when instructions and/or a treatment session request for interaction resulting in body motion, then the lack of detection of such body motion could be an indicator of the lack of absorption in this task.

The response data according to a particular embodiment may comprise measured data that comprises EEG data, and optionally muscular activity data, such as for example EMG data, and cardiovascular data, such as for example ECG data, and eye movement data, such as for example EOG or eye tracking data, observational data that comprises patient's observed movement or lack of movement and dosages of received medication and/or self-reported data that comprises self-reported consciously perceived level of pain ratings, level of dissociation ratings, optionally amnesia ratings, .... Preferably the response data comprises EEG data. Preferably the self-reported data (if any) comprises ratings for a level of anxiety, a level of pain and optionally a depth of state, such as for example a level of dissociation., a level of absorption, etc.

According to some embodiments cardiovascular data and/or respiratory data could also be derived from motion tracking data and/or data generated by one or more cameras directed at the face.

According to exemplary embodiments in the response data further comprises ECG and/or cardiovascular data and/or respiratory data and/or muscular activity data and/or EOG data and/or body temperature data, from which the LoA can be determined.

Preferably the measured data comprises EEG data that comprises data collected from the F-EEG electrode and/or P-EEG electrode and/or C-EEG electrode, and the group 4 indicator is determined by:

- extracting from the F- EEG electrode data, a power associated with a band in the theta-alpha, ta, frequency range (F-power (ta)); and/or
- extracting from the P- EEG electrode data, a power associated with a band in the theta-alpha, ta, frequency range (P-power (ta)); and/or
- extracting from the C- EEG electrode data, a power associated with a band in the theta-alpha, ta, frequency range (C-power (ta));

wherein the F-power (ta) and/or P-power (ta) and/or C-power (ta) (Group 4 indicators) are together further also indicative of the state of consciousness of a subject. For example when subjected to an identical constant pain stimulus, any measured changes to LoP are also an indicator for changes to the state of consciousness of a subject.

The embodiments of the Group 4 indicators:

- one or more of F-power (ta), P-power (ta), C-power (ta)

are also indicators of the state of consciousness of a subject. In particular, they are indicative of the level of pain the subject consciously perceives. In other words, for example when subjected to an identical constant pain stimulus, any measured changes to LoP are also an indicator for changes to the state of consciousness of a subject.

Preferably the EEG data comprises data collected from the C-EEG electrode and optionally from P-EEG electrode and optionally from the F-EEG electrode, and the determining comprises:

- extracting from the C-EEG electrode data, the C-power (ta),
- optionally extracting from the P- EEG electrode data, the P-power (ta), and
- optionally extracting from the F-EEG electrode data, the F-power (ta)

wherein the C-power (ta), and optionally P- power (ta) and optionally F- power (ta) are indicative of the LoP and optionally the state of consciousness of a subject.

Preferably the EEG data comprises data collected from the C-EEG electrode and from P-EEG electrode and optionally from the F-EEG electrode, and the determining comprises:

- extracting from the C-EEG electrode data, the C-power (ta),
- extracting from the P- EEG electrode data, the P-power (ta), and
- optionally extracting from the F-EEG electrode data, the F-power (ta)

wherein the C-power (ta) and P- power (ta) and optionally F- power (ta) are indicative of the LoP and optionally the state of consciousness of a subject.

The EEG data may comprise data collected from the C-EEG electrode and the P-EEG electrode and the F-EEG electrode, and the determining comprises:

- extracting from the C-EEG electrode data, the C- power (ta), and
- extracting from the P- EEG electrode data, the P- power (ta), and
- extracting from the F-EEG electrode data, the F- power (ta),

wherein the combination of C- power (ta) and P-power (ta) and F- power (ta) are indicative of the LoP and optionally the state of consciousness of a subject.

It is clear that alternative embodiments of the EEG data collected from any suitable combination of one or more of the above-mentioned EEG electrodes, and/or at least one other EEG electrode are possible. It is thus clear that still further embodiments of the group 4 indicator are possible in which EEG electrode data from one or more electrodes at any suitable scalp location is used to extract a power (ta) or in other words a power associated with a theta-alpha frequency band that is indicative for the LoP consciously experienced by the subject.

According to a particular embodiment a value of power (ta) compared with a reference value is further indicative of the LoP experienced by a subject. For example, a value of said F-power (ta) compared with a reference value is further indicative of the LoP experienced by a subject, a value of said P-power (ta) compared with a reference value is further indicative of the LoP experienced by a subject, and/or a value of said C-power (ta) compared with a reference value is further indicative of the LoP experienced by a subject.

According to such an embodiment an increase of power(ta), for example of F-power (ta) and/or P-power (ta) and/or C-power (ta) compared with a respective reference value(s) is indicative of a reduced LoP and vice versa.

According to such embodiments an increase of power(ta), for example F-power (ta), P-power (ta), and/or C-power (ta), compared with a reference value is further also indicative of a lower state of consciousness, for example associated with a higher level of dissociation, of a subject and/or vice versa.

As will be explained in further detail below such reference values, or reference data can be used to scale and/or index the measured data, such as for example power(ta) or any other measured data and/or the parameters determined from it, such as for example the LoP. According to such embodiments reference data comprising measured data of a subject and/or a population correlated to at least one predetermined reference LoP is received. This could for example be reference data generated by the subject itself, such as a process similar as the calibration phase or the normal awake state of the experiment, for example prior to a treatment session. According to alternative embodiments the reference data and the correlations could result from separate and distinct subject or population-based reference measurements. There can then be determined at least one correlation of at least one predetermined reference LoP and at least one group indicator extracted from the reference data. In this way, similar as described above, for example one or more particular values of the group 4 indicator power(ta) can be correlated with one or more corresponding particular reference values for LoP. These correlations then allow to scale and/or index any subsequently measurements of such a group indicator, such as for example the group 4 indicator power(ta). It is clear that similarly for other group indicators there can be scaled and/or indexed a subsequently measured LoP with respect to the at least one predetermined reference LoP by means of said at least one correlation.

According to a particular embodiment, similar as already mentioned above, the LoP is also based on a group 3 indicator. According to such an embodiment, preferably the EEG data comprises data collected from the C-EEG electrode, and/or P-EEG electrode and/or F-EEG electrode, and the determining comprises:

- extracting from the C- EEG electrode data, a mean signal peak-to-peak amplitude (C-MSPA), and/or

- extracting from the P-EEG electrode data, a mean signal peak-to-peak amplitude (P-MSPA), and/or
- extracting from the F-EEG electrode data, a mean signal peak-to-peak amplitude (F-MSPA),

wherein the C-MSPA, and/or P-MSPA and/or F-MSPA, embodiments of Group 3 indicators, are indicative of the LoP of a subject. In particular, they are indicative of how much pain the subject consciously perceives. Similar as explained above, pain perception, for example under identical pain conditions, can also be an indicator of the state of consciousness of a subject.

The embodiments of Group 3 indicators:

- one or more of C-MSPA, P-MSPA, F-MSPA

are indicators of the LoP of a subject.

Similar as explained above with reference to the group 4 indicator, also for the group 3 indicator there can be made use of reference data to scale and/or index the measured data for determining the LoP. For example, a change of said C-MSPA and/or P-MSPA and/or F-MSPA compared with a respective reference value(s) is indicative of a change to the LoP of the subject. In particular a reduction of said C-MSPA and/or of said P-MSPA and/or of said F-MSPA is indicative of a decrease in the LoP of the subject and an increase with respect to the reference value is thus indicative for an increase in the LoP. Similar as explained above with respect to the group 4 indicator, for example under identical pain conditions, a change to the LoP can also be indicative for a corresponding change to the state of consciousness of the subject. It is clear that similar as described above with respect to group 4 indicator 4, also for group 3 indicator, and/or any suitable combination of group indicators and/or other group indicators, there can be scaled and/or indexed a subsequently measured LoP with respect to the at least one predetermined reference LoP by means of said at least one correlation, which could be subject based or population based.

According to a preferred embodiment the EEG data comprises data collected from the C-EEG electrode and optionally from P-EEG electrode and optionally from the F-EEG electrode, and the determining comprises:

- extracting from the C-EEG electrode data, the C-MSPA,
- optionally extracting from the P- EEG electrode data, the P-MSPA, and
- optionally extracting from the F-EEG electrode data, the F-MSPA

wherein the C-MSPA, and optionally P-MSPA and optionally F-MSPA are indicative of the LoP of a subject.

According a further embodiment the EEG data may comprise data collected from the C-EEG electrode and from P-EEG electrode and optionally from the F-EEG electrode, and the determining comprises:

- extracting from the C-EEG electrode data, the C-MSPA, and
- extracting from the P- EEG electrode data, the P-MSPA, and
- optionally extracting from the F-EEG electrode data, the F-MSPA

wherein the C-MSPA, and P-MSPA and optionally F-MSPA are indicative of the LoP of a subject.

According to a further embodiment the EEG data may comprise data collected from the C-EEG electrode and the P-EEG electrode and the F-EEG electrode, and the determining comprises:

- extracting from the C-EEG electrode data, the C-MSPA, and
- extracting from the P- EEG electrode data, the P-MSPA, and
- extracting from the F-EEG electrode data, the F-MSPA

wherein the combination of C-MSPA, and P-MSPA and F-MSPA are indicative of the LoP of a subject.

According to a particular embodiment, similar as already mentioned above, the LoP is also based on a group 5 indicator. According to such an embodiment, preferably the measured data may further comprise electromyography, EMG, data, for example collected from one or more EMG electrodes, or derived from any other suitable electrode, such as for example EEG electrodes. There can be extracted from the EMG data, a group 5 indicator, which is for example a mean signal peak-to-peak amplitude, or EMG-MSPA, wherein the EMG-MSPA, or Group 5 indicator, is indicative of the level of pain the subject consciously perceives. Similar as explained above, pain perception, for example under identical pain conditions, can also be an indicator of the state of consciousness of a subject.

The Group 5 indicator:

- EMG-MSPA

Is an indicator for the level of pain a subject consciously perceives, or in other words the LoP.

Similar as explained above with reference to the group 4 indicator, also for the group 5 indicator there can be made use of reference data to scale and/or index the measured data for determining the LoP. For example, a change of said EMG-MSPA compared with a reference value is further indicative of a change to the LoP of the subject. A reduction of said EMG-MSPA compared with a reference value is further indicative of a reduced LoP of the subject. It is clear that an increase compared to a reference value, is thus indicative of an increase of the LoP. Similar as explained above with respect to the group 4 indicator, for example under identical pain conditions, a change to the LoP can also be indicative for a corresponding change to the state of consciousness of the subject. It is clear that similar as described above with respect to group 4 indicator, also for a group 5 indicator, and/or any suitable combination of group indicators and/or other group indicators, there can be scaled and/or indexed a subsequently measured LoP with respect to the at least one predetermined reference LoP by means of said at least one correlation, which could be subject based or population based.

As already mentioned above, a level of pain or LoP of the subject may be determined from a Group 4 indicator and optionally, a Group 3 indicator and/or a Group 5 indicator.

In particular, the level of pain, LoP may be determined from:

- power(ta), such as for example one or more of the C-power (ta), the P-power (ta), and the F-power (ta) (Group 4 indicator), and optionally
- the MSPA, such as for example C-MSPA, and/or P-MSPA and/or F-MSPA (Group 3 indicator), and/or
- the EMG-MSPA (Group 5 indicator).

In particular, the level of pain LoP may be determined from:

- the power(ta), for example one or more of the C-power (ta), the P-power (ta), the F-power (ta) (Group 4 indicator),
- optionally the MSPA, for example the C-MSPA, and optionally P-MSPA and optionally F-MSPA (Group 3 indicator), and
- optionally the EMG-MSPA (Group 5 indicator).

In particular, the level of pain (LoP) may be determined from:

- the power(ta), for example the C-power (ta), and optionally the P-power (ta), and optionally the F-power (ta) (Group 4 indicator), and
- the MSPA, for example the C-MSPA, and optionally P-MSPA and optionally F-MSPA (Group 3 indicator), and
- optionally the EMG-MSPA (Group 5 indicator).

In addition to the group 8 indicator mentioned above with respect to the experiment 500 as shown in FIG. 1 and the application of the results of this experiment in the computer-implemented methods of FIG. 5 and FIG. 6, the LoA can optionally be determined by means of additional indicators, such as for example a group 6, 7, 9, 10 and/or 11 indicator as described in further detail below.

According to such embodiments, during the embodiment of the experiment as shown in FIG.1, in addition to the group 8 indicator, there is for example extracted from the measured data any other suitable group indicator in the time domain, for example by means of a suitable event related potential, or ERP, in response to the calibrated pain and/or anxiety inducing stimulus and/or any other suitable group indicator in the frequency domain, similar as described above.

As will be described in further detail below, and already mentioned above the group 8 indicator is based on measured data comprising EEG data, which can be referred to as a cognitive, electrophysiological signal, and is related to corticoidal signals, and is related to the cognitive dimension of the level of anxiety as experienced by the subject. The group 6, 7, 9, 10 and/or 11 indicators are all related to non-cognitive physiological and/or non-cognitive electrophysiological signals for generating measured data, such as for example cardiovascular data, ECG data, respiratory data, ocular movement data, EOG data, muscular activity data, EMG data, body temperature data, etc. It is thus clear that, a combination of the group 8 indicator for measuring the cognitive dimension of the level of anxiety and optionally, additionally and/or preferably at least one non-cognitive physiological group indicator such as for example group 6, 7, 9, 10 and/or 11 as described in further detail below provides for measuring a combination of this cognitive dimension of anxiety with the autonomic dimension, thereby allowing for measurement of the multi-dimensional aspect of the level of anxiety as experienced by a subject.

According to an exemplary embodiment the measured data may comprise cardiovascular data. Such cardiovascular data may for example be acquired using two or more electrocardiogram (ECG) electrodes placed on the skin using peripheral or precordial placement, or using any other placement where ECG data can be derived. ECG detects electrical activity of the heart. Alternatively, or in addition, cardiovascular data may be acquired using a photoplethysmogram (PPG) sensor placed on the skin. PPG detects blood volume changes vasculature below the skin, from which for example heart rate, heart rate variability, etc. can be derived. Cardiovascular data may alternatively, or in addition be acquired from at least one other suitable electrode, such as for example one or more EEG electrodes, etc., configured to detect a signal from which cardiovascular data, such as heart rate, heart rate variability, ... can be detected and/or extracted. Cardiovascular data may comprise heart rate, heart rate variability, derived metrics, phase-based parameters, frequency-based parameters. However, it is clear that, according to alternative embodiments, ECG measured data, might for example be extracted from any suitable electrodes, such as for example from signals measured by EEG electrodes. In general, and as described in further detail below such cardiovascular data may be received from any suitable cardiovascular data capturing unit.

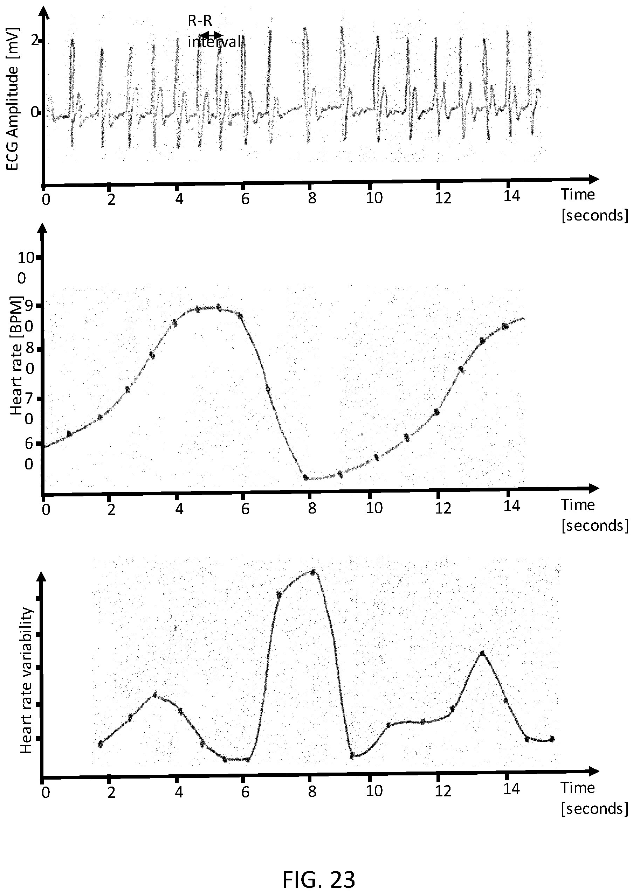

According to such an embodiment, based on the received measured data further comprising the cardiovascular data in addition to the P-EEG data, there can be extracted from the cardiovascular data a group 7 indicator based on a heart rate and/or heart rate variability. Preferably, as will be described in further detail with respect to the embodiments shown in FIG. 23 and/or FIG. 24 - FIG. 26, preferably a specific combination of heart rate and heart rate variability is used to extract the group 7 indicator. Subsequently the LoA is determined based on said group 8 indicator and at least the group 7 indicator.

The correlation between a specific combination of heart rate or HR and heart rate variability HRV providing for a group 7 indicator is shown in Fig. 37 for the measurement data according to the embodiment explained above with reference to Fig. 1. As shown, for this specific combination of HR and HRV, as will be explained in further detail below, there was a strong correlation with the self-reported LoA with rs=0.5 and a p-value=0.02. Fig. 23 details an embodiment, in which use is made of ECG data to obtain the HR and HRV. However, it is clear that alternative embodiments are possible in which the HR and HRV are determined by alternative means, and/or are present in the measured data obtained from the experiment directly. According to the embodiment of Fig. 23, there is shown an example of the amplitude of an ECG signal, subsequently, by means of peak detection in this ECG signal the heart rate can be detected, which is shown as the number of beats per minute or bpm. The variability of the detected heart rate then forms the basis for calculating the heart rate variability. According to the specific embodiment, as shown in Fig. 23, the ECG signal is for example first pre-processed to remove any noise and artefacts. Subsequently, any useful characteristic features of the ECG signal can be extracted such as for example, the P wave, which represents the depolarization of the atria; the QRS complex, which represents the depolarization of the ventricles; and/or the T wave, which represents the repolarization of the ventricles, as known to the man skilled in the art. According to the embodiment shown, the R peaks of each ECG signal are detected, and the R-R interval, i.e. the interval between the R peaks of two consecutive heart-beat ECG signals is determined, and based on this R-R interval the heart rate is calculated. Subsequently based on the R-R interval and/or the derived heart rate, there is performed a time-domain analysis to determine the HRV, based on the difference and/or variability between two adjacent R-R intervals, and/or the heart rate associated with two adjacent heart beats. According to a particular embodiment the HRV could for example be determined based on the standard deviation of the normal R-R interval in a particular time period.

One subject of the embodiment of the experiment above, for example provided a self-reported level of anxiety of 4.1 during the normal awake phase 530, during which a corresponding HR = 58 bpm, which was for example the mean, average, weighted mean, ... of the HR during the normal awake phase. Additionally a HRV = 53 ms was measured, as the standard deviation of the normal R-R interval during the normal awake phase 530. Subsequently during the second phase 540 with the hypnotic treatment session a lower self-reported level of anxiety of 1.1 was provided by the subject and this corresponded to a measured HR = 51bpm and a HRV = 169 ms.

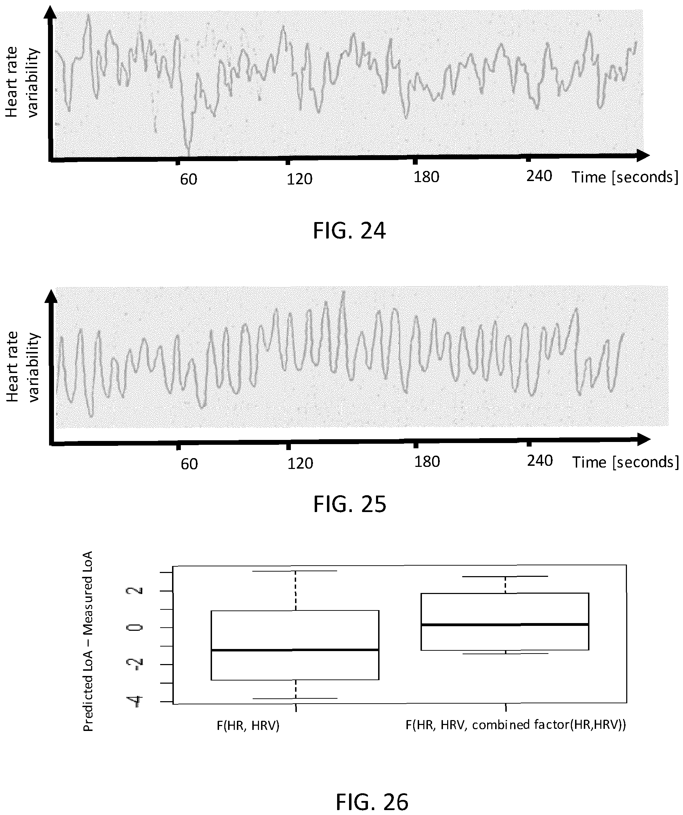

In order to further visualize the effect on the HRV FIG. 24 shows an exemplary heart rate variability for a subject with a higher level of anxiety than in FIG. 25, and FIG. 25 shows a heart rate variability for a subject with a lower level of anxiety than in FIG. 24.

According to a particular advantageous embodiment, in which a combination of the group 8 indicator and the group 7 indicator is used for determining the LoA, there is made use of the following specific formula for computing the LoA:

$$LoA_{s,t} \sim f_4 \left( group\ 8\ indicator_{s,t}, \alpha_1 f_1 \left( HR_{s,[t',t]} \right) + \alpha_2 f_2 \left( HRV_{s,[t',t]} \right) + \alpha_3 f_3 \left( HR_{s,[t',t]}, HRV_{s,[t',t]} \right) \right)$$

Where:

- $LoA_{s,t}$ is the anxiety level of subject s at time t;
- $group\ 8\ indicator_{s,t}$ is an embodiment of the group 8 indicator based on P-power(dt) as described above of subject s at time t;
- $\alpha_1 f_1 (HR_{s,[t',t]}) + \alpha_2 f_2(HRV_{s,[t',t]}) + \alpha_3 f_3(HR_{s,[t',t]}, HRV_{s,[t',t]})$ is a specific advantageous embodiment of the group 7

indicator.

- $\alpha_i$ is a parameter that is determined in function of for example subject characteristics, population characteristics, population size, ... and that is for example determined using an automated machine learning processes, in which parameters are selected which provide for the best correlation with a predetermined set of measured data and/or reference data, such as for example population based reference data;
- $f_i$ is a mathematical function determined using a similar automated machine learning processes, in which functions are selected that provide for the best correlation with a predetermined set of measured data and/or reference data, such as for example population based reference data;
- $HR_{s,[t',t]}$ is a single value representing the heart rate of subject s during a time period from time t' to time t, e.g. mean value, however alternative embodiments are possible such as an average value, weighted mean, etc.,
- $HRV_{s,[t',t]}$ is a single value representing the HRV of subject s during a time period from time t' to time t, e.g. standard deviation of the normal R-R interval, or any other suitable quantification of the HRV.

This specific embodiment of the group 7 indicator is particularly advantageous, as it not only combines the HR and HRV, but also comprises what can be referred to as a combined HR-HRV factor: $\alpha_3 f_3 (HR_{s,[t',t]}, HRV_{s,[t',t]})$. To demonstrate the surprising, advantageous effect of this combined HR-HRV, FIG. 26 shows a boxplot of the difference between a predicted LoA and an actually measured LoA based on a model only making use of a function comprising the combination of HR and HRV, in other words $\alpha_1 f_1 (HR_{s,[t',t]}) + \alpha_2 f_2 (HRV_{s,[t',t]})$, for the calculation of the group 7 indicator, which was labelled "f(HR, HRV)". For comparison, a further boxplot is shown labelled "f(HR, HRV, combined HR-HRV factor)", in which for the calculation of the group 7 indicator use was also made of the combined HR-HRV factor, or in other words $\alpha_1 f_1 (HR_{s,[t',t]}) + \alpha_2 f_2 (HRV_{s,[t',t]}) + \alpha_3 f_3 (HR_{s,[t',t]}, HRV_{s,[t',t]})$. We observe that the prediction accuracy of the model with the combined HR-HRV factor is better as the mean difference = 0.36 and standard deviation = 1.55, which is better than the mean difference = -1.14 and std dev = 2.20 of the model without the combined HR-HRV factor.

Optionally a time-frequency analysis of the HRV could be performed in order to extract from the HRV signal a signal correlated to the breathing rate, which could also be used for determining a group indicator, as will be described in further detail below. It is however clear that alternative embodiments are possible in which the HR and HRV can be extracted from the measured data. As shown in FIG. 24 and FIG. 25, there is a clear effect of the breathing frequency that is recognizable as a sinusoidal pattern with peaks and valleys, of which one cycle corresponds to a breathing cycle, in the HRV signal. It is clear, that when a subject is experiencing a higher level of anxiety as in FIG. 24, the breathing rate is less stable or comprises a higher level of variability then at a lower level of anxiety as shown in FIG. 25.

According to still a further embodiment there is provided a computer-implemented method 300 for measurement of a level of anxiety comprising the further step of receiving measured data further comprising respiratory data. From this respiratory data there can then be extracted a group 6 indicator based on respiration rate and/or a respiration variability and/or a predetermined respiration pattern and/or oxygen saturation. This then allows to determine the LoA based on the group 8 indicator and at least the group 6 indicator, which similar as described above provides for a multi-dimensional measurement of the LoA.

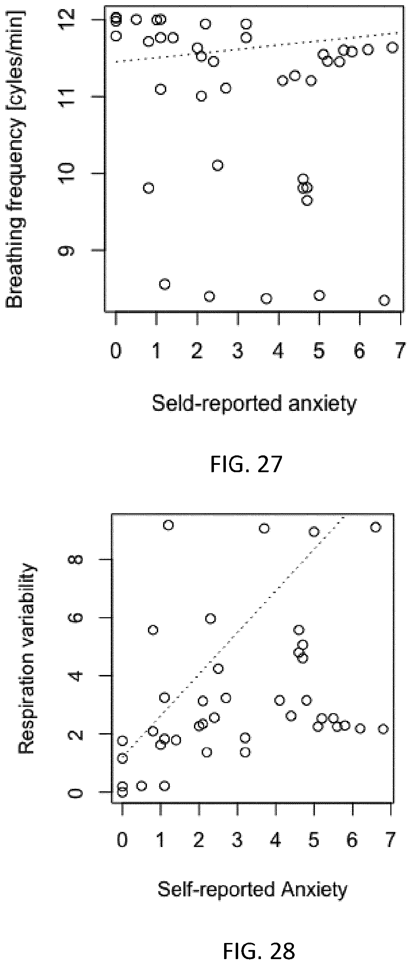

From the measured data from the experiment of Fig. 1, there was found to be a correlation of the respiration rate with the self-reported level of anxiety, with rs= 0.458 and a p-value = 0.002. In other words, as already mentioned above, an increased level of anxiety as experienced by the subject leads to a higher respiration rate. FIG. 27 shows correlation between self-reported anxiety and respiration rate also referred to as breathing frequency for the measured data from the experiment of Fig. 1. It is clear that the respiration rate or the breathing frequency, typically refers to the number of breathing cycles per minute.

Further there was also found a correlation between the respiration rate variability, such as for example the standard deviation of the Inter-cycles interval or ICI, was found to be significantly higher during a period with a higher LoA. There was thus found to be a correlation with the self-reported level of anxiety, with an rs= 0.434, and p-value = 0.005, i.e. an increased level of anxiety leads to a less regular respiration pattern. FIG. 28 shows such correlation between the self-reported level of anxiety and respiration rate variability, also referred to as respiration variability, for the measured data from the experiment of Fig. 1. It is thus clear that the respiration rate variability, such as for example determined as the standard deviation of the ICI, is a measure for the regularity and/or irregularity of successive breathing cycles over a certain period of time.

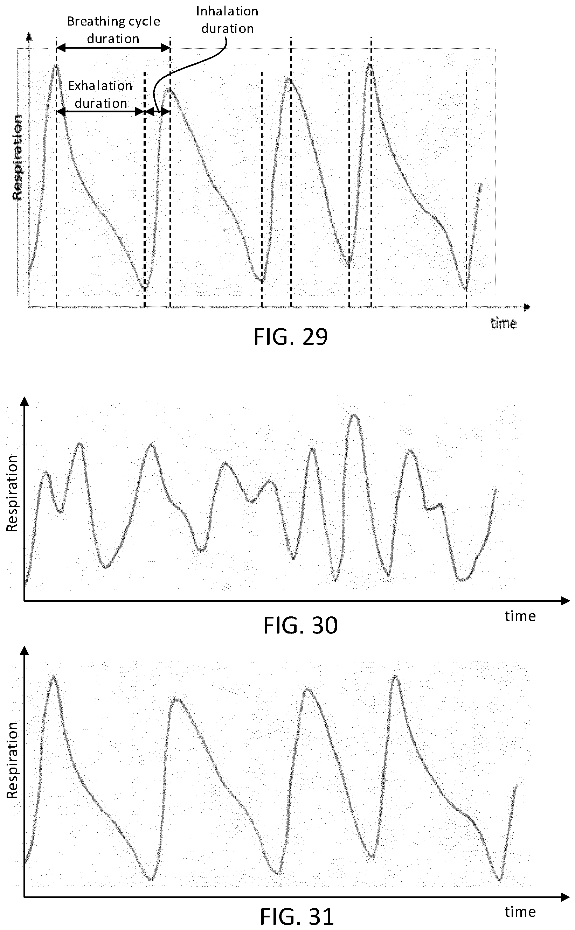

Further FIG. 29 shows an exemplary embodiment of a time domain respiratory data signal, which for example shows flow and/or pressure variations during a few successive breathing cycles, in which a breathing cycle comprises a combination of a successive exhalation and inhalation. FIG. 29 shows such respiratory data during a time period of about 1 minute. As shown, from such respiratory data there could be extracted parameters such as the breathing cycle duration, which is the time period associated with one complete breathing cycle; the exhalation duration, which is the time period associated with the part of the breathing cycle during which the exhalation takes place; the inhalation duration, which is the time period associated with the part of the breathing cycle during which the inhalation takes place; and/or any ratio's and/or any combination of such durations; and/or any other parameters derivable from such time domain respiratory data signal, such as for example the peak to peak amplitude of the signal, the minima and maxima of the signal, and/or variability of such signals, etc. FIG. 30 and FIG. 31 show the respiratory data signals of an exemplary subject that participated in the experiment of FIG. 1. FIG. 30 shows the respiratory data signal of the subject during the normal awake state 530 during which the subject experienced a self-reported level of anxiety = 4.6 . FIG. 31 shows the respiratory data signal of the same subject during the hypnotic treatment session 540 during which the subject experienced a self-reported level of anxiety = 2.5. It is clear from a comparison of FIG. 30 and FIG. 31 that when the level of anxiety is lower, this correlates for example to a lower respiration rate, or in other words a longer breathing cycle duration. Further, as shown a lower level of anxiety also correlates to a more regular breathing cycle, in other words there is less variability regarding the duration and/or amplitude of successive breathing cycles, the duration and/or amplitude of successive inhalation periods, the duration and/or amplitude of successive exhalation periods, etc.. Further, as for example shown, at a lower level of anxiety the exhalation duration is longer than the inhalation duration, while at a higher level of anxiety this is no longer always the case. Any of these parameters, and/or any suitable combination, ratio, etc. thereof thus allows for further embodiments of a suitable group 6 indicator based on respiratory data for determining the LoA. It is however clear that still further alternative embodiments are possible. According to such alternative embodiments, there could for example be taken into account the occurrence of any suitable predetermined respiration pattern, such as for example the occurrence of erratic breathing cycles in which there cannot be made a clear distinction between an exhalation and an inhalation, the occurrence, frequency, and/or duration of pauses between and/or during an inhalation and/or exhalation, etc. It is however clear that alternative embodiments of alternative predetermined respiration patterns are possible, which could be taken into account for embodiments of the group 6 indicator.

According to a particular advantageous embodiment, the LoA is calculated based on the group 8 indicator, in combination with an embodiment the group 6 indicator and the group 7 indicator, or in other words: *LoA~f* (*group* 8 *indicator ,group 7 indicator, group* 6 *indicator*)

According to a particular advantageous embodiment, similar as explained above, with respect to the combined HR-HRV factor, preferable there is additionally made use of a combined factor taking into account the influence of the respiration rate or BR on the heart rate and/or the heart rate variability, so similar as explained above such a combined HR-BR factor, such as for example could be included in the formula for calculation of the LoA, such as for example $\alpha_i f_i$ ($HR_{s,[t',t]}$,$BR_{s,[t',t]}$) and/or $\alpha_i f_i$ ($HRV_{s,[t',t]}$, $BR_{s,[t',t]}$), for example as part of the group 7 indicator and/or the group 6 indicator.

It is clear that still further alternative embodiments for determining a group 6 indicator from the respiratory data, for example it is possible to take into account data with respect to the volume and/or depth of breathing, which could for example be directly measured, and/or indirectly derived from data relating to oxygen saturation, such as for example by means an SpO2 sensor, or from the amount of belly and/or chest movement during breathing. A person with a higher level of anxiety has been detected to have a shallower breath, with a reduced volume of breathing, then when a subject is experiencing a lower level of anxiety. This is for example also illustrated in the embodiment of FIG. 30 and FIG. 31 as the peak to peak amplitude of the signal of FIG. 31 is larger than that of FIG. 30. It is however clear that still further alternative embodiments are possible, such as for example the detection of a reduced flow, volume and/or pressure of air during an inhalation and/or an exhalation, etc.

According to still a further embodiments there is provided a computer-implemented method 300 for measurement of a level of anxiety comprising the further step of receiving measured data relating to a autonomic physiological parameter for providing a multi-dimensional measurement of the LoA similar as described above and as will be explained in more detail below. According to these embodiments the LoA can be determined based on the group 8 indicator and at least one or more of a group 6, 7, 9, 10 and/or 11 indicator. Such as for example

$$LoA \sim f \begin{pmatrix} group\ 8\ indicator\ \ ,group\ 7\ indicator,\ group\ 6\ indicator, \\ group\ 9\ indicator,\ group\ 10\ indicator,\ \ group\ 11\ indicator \end{pmatrix}.$$

According to such an embodiment the received measured data further comprises ocular movement data, and there can be extracted a group 9 indicator based on an ocular movement rate and/or ocular movement amplitude and/or predetermined ocular movement patterns from the ocular movement data. This then allows to determine the LoA based on the group 8 indicator and at least the group 9 indicator, and optionally any other suitable autonomic physiological group indicator.

According to a particular embodiment the ocular movement data was captured as EOG data and the group 9 indicator is based on the detection of saccadic eye movements from the ocular movement data.

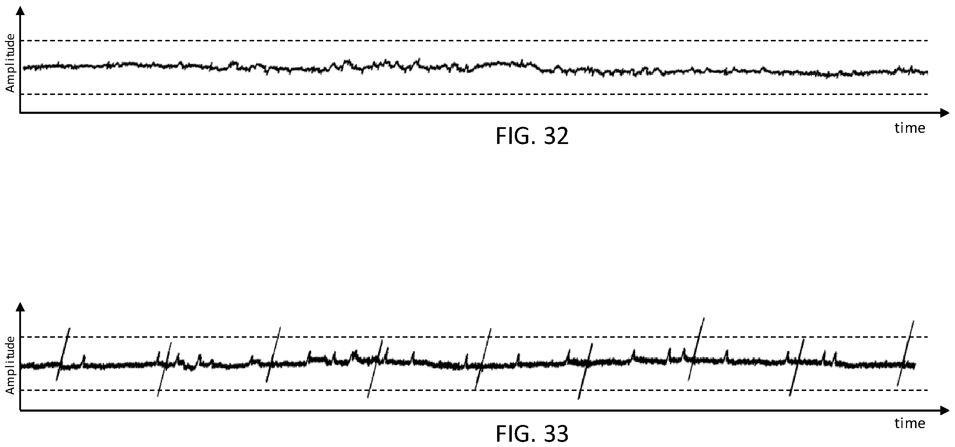

FIG. 32 and FIG. 33 show an embodiment of ocular movement data of a single subject that was captured during the experiment of FIG. 1 in the form of EOG data signals. FIG. 32 shows the EOG data signal of the subject during the hypnotic treatment session 540 during which the subject experienced a self-reported level of anxiety = 2.1. FIG. 33 shows the EOG data signal of the subject during the normal awake state 530 during which the subject experienced a self-reported level of anxiety = 6.8 . The time period of the signal shown is about 30 seconds. In order to determine the frequency of saccadic eye movements, and/or the number of saccadic eye movements during a particular time period, according to the embodiment shown, there can be made use of one or more thresholds, as for example schematically represented by means of the striped lines in FIG. 32 and FIG. 33. According to such an embodiment, for example every time the EOG signal crosses one of these thresholds a saccadic eye movement is detected. As shown in FIG. 32, no saccadic eye movements are detected in the EOG signal for this subject at a self-reported level of anxiety = 2.1 . As shown in FIG. 33 9 saccadic eye movements are detected in the time period of about 30 seconds in the EOG signal for this subject at a self-reported level of anxiety = 6.8 . In other words, the frequency of saccadic eye movements is higher when there is a higher level of anxiety. It is however clear that alternative embodiments are possible in which the number and/or frequency of saccadic eye movements, and or any other suitable parameter related to saccadic eye movements, such as for example the amplitude, direction, predetermined pattern, etc., is extracted from ocular movement data, such as for example EOG data, on which a suitable embodiment of a group 9 indicator can be based for determining the LoA.

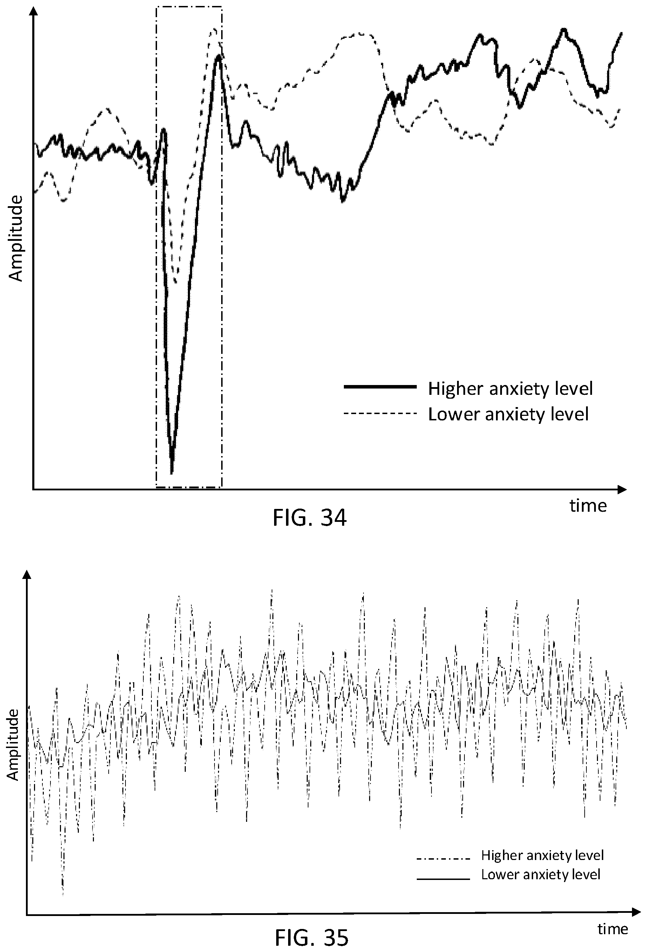

FIG. 34 shows an alternative, exemplary embodiment of time domain ocular movement data, in the form of an EOG data signal, averaged for a plurality of subjects, which is time-locked to a painful stimulus, which serves as an anxiety inducing stimulus. In other words, there is shown an event-related potential or ERP for the EOG data signal which is time-locked to the painful stimuli during the experiment of FIG. 1. According to the exemplary embodiment shown, the time-domain signal can for example be analyzed, during a specific time window, as schematically shown by means of the dot striped box, which is for example approximately 2 seconds, during which for example a peak amplitude, a peak to peak amplitude, and/or any other suitable amplitude based parameter with respect to the ocular movement data signal can be determined. The full line graph shows the averaged EOG signal of all subjects during the experiment of FIG. 1 during the normal awake state 530, and the striped graph shows the averaged EOG signal for all subjects during the hypnotic treatment session 540. It is clear that in the time window, the peak to peak amplitude of the eye movement data signal is larger for the signal with a higher level of anxiety. Further, according to this specific embodiment, specifically the negative peak amplitude of the EOG signal, or in other words the minimum value of the EOG signal in the time window shows a clear difference at different levels of anxiety. For the experiment of FIG. 1 the average value of the amplitude of the EOG data signal during the time window was for example -17.8 mV during the normal awake state 530 which corresponds to a higher level of anxiety, while it was only -7.6 mV during the hypnotic treatment session 540. It is however clear that alternative embodiments are possible in which, for example based on the amplitude of the ocular movement data, there can be determined a suitable embodiment of a group 9 indicator for determining the LoA. It is however clear that alternative embodiments are possible in which the amplitude of the EOG data, and or any other suitable parameter related to ocular movement is extracted from ocular movement data, such as for example EOG data, on which a suitable embodiment of a group 9 indicator can be based for determining the LoA, such as for example ocular movement rate, ocular movement amplitude, predetermined ocular movement patterns, detection of saccadic eye movements, one or more characteristics of saccadic eye movement patterns, such as for example frequency, amplitude, direction and/or predetermined saccadic eye movement patterns.

The EOG data could be captured by a suitable sensor for electrooculography, which is a technique for measuring the corneo-retinal standing potential that exists between the front and the back of the human eye. The resulting signal is called the electrooculogram or EOG. An EOG allows to record eye movements.

There are four basic types of eye movements: saccadic eye movements, smooth pursuit eye movements, vergence eye movements, and vestibulo-ocular eye movements. Saccadic eye movements are rapid, ballistic movements of the eyes that abruptly change the point of fixation. It has been found that the frequency and/or number in predetermined time frame of saccadic eye movements increases when a higher level of anxiety is experienced by a subject.

According to such a further embodiment the received measured data further comprises muscular activity data, such as for example electromyography, EMG, data from which a group 10 indicator based on muscular activity, such as for example electromyographic, EMG, activity can be extracted, which allows to determine the LoA based on said group 8 indicator and at least the group 10 indicator, and optionally any other suitable group indicator.

FIG. 35 shows an embodiment of muscular activity data of a single subject that was captured during the experiment of FIG. 1 in the form of EMG data signals, of for example mastoid muscles or any other suitable muscles; in the time domain. The signal shown in dot striped line is an EMG signal captured for this subject during the normal awake state 530, during which the subject reported a level of anxiety = 6.8 . The signal shown in the full line is an EMG signal captured for this subject during the hypnotic treatment session 540, during which the subject reported a level of anxiety = 2.1 . In other words, the EMG signal in the dot striped line is associated with a higher level of anxiety, while the EMG signal in the full line is associated with a lower level of anxiety. It is clear from the embodiment shown, that for example the peak to peak amplitude, of the EMG signal is higher at higher levels of anxiety, or in other words, there is more muscular activity, and/or a higher level of muscular contraction when the subject experiences higher levels of anxiety. It is thus clear that, for example based on such differences in the amplitude of the EMG signal, a suitable embodiment of the group 10 indicator could be determined for determining the LoA. It is however clear that further embodiments are possible in which a group 10 indicator is based on any suitable parameters of muscular activity data, for example extracted from an EMG signal, such as for example the amplitude, frequency, variability, predetermined patterns, etc.

According to such a further embodiment the received measured data further comprises body temperature data from which a group 11 indicator based on body temperature can be extracted so that the LoA based on said group 8 indicator and at least the group 11 indicator or any other suitable indicator.

Similar as described above, according to a preferred embodiment, the method for measuring the LoA 300 comprises the step of receiving the measured data of the subject comprising the electroencephalogram, EEG, data collected from one or more of the following EEG electrodes:

- a frontal (F) EEG electrode configured for collection of F-EEG electrode data from the scalp anatomical region corresponding to a frontal lobe of the subject,
- a parietal (P) EEG electrode configured for collection of P-EEG electrode data from the scalp anatomical region corresponding to a parietal lobe of the subject,
- a central (C) EEG electrode configured for collection of C-EEG electrode data from the scalp anatomical region corresponding to a precentral and postcentral gyrus of the subject.

Such EEG data is related to brain waves generated by parts of the brain close to the scalp, including the cortex, and therefor are related to the level of anxiety as cognitively experienced and optionally physiologically experienced by the subject.

According to a particular embodiment a value of P-power (dt) compared with a reference value is further indicative of the LoA experienced by a subject. For example, a value of said P-power (dt) compared with a reference value is further indicative of the LoA experienced by a subject. According to such an embodiment an increase of P-power (dt) compared with a respective reference value(s) is indicative of an increased LoA and vice versa.

As will be explained in further detail below such reference values, or reference data can be used to scale and/or index the measured data, such as for example P-power(dt) or any other measured data and/or the parameters determined from it, such as for example the LoA. According to such embodiments reference data comprising measured data of a subject and/or a population correlated to at least one predetermined reference LoA is received. This could for example be reference data generated by the subject itself, such as a process similar as the calibration phase or the normal awake state of the experiment, for example prior to a treatment session. According to alternative embodiments the reference data and the correlations could result from separate and distinct subject or population-based reference measurements. There can then be determined at least one correlation of at least one predetermined reference LoA and at least one group indicator extracted from the reference data. In this way, similar as described above, for example one or more particular values of the group 8 indicator P-power(dt) can be correlated with one or more corresponding particular reference values for LoA. These correlations then allow to scale and/or index any subsequently measurements of such a group indicator, such as for example the group 8 indicator P-power(dt). It is clear that similarly for other group indicators there can be scaled and/or indexed a subsequently measured LoA with respect to the at least one predetermined reference LoA by means of said at least one correlation.

Similar as explained above with reference to the group 8 indicator, also for any of the other group indicators, such as the group 6, 7, 9, 10, and/or 11 indicator there can be made use of reference data to scale and/or index the measured data for determining the LoA. It is clear that similar as described above with respect to group 8 indicator, also for any the group 6, 7, 9, 10, and/or 11 indicators, and/or any suitable combination of group indicators and/or other group indicators, there can be scaled and/or indexed a subsequently measured LoA with respect to the at least one predetermined reference LoA by means of said at least one correlation, which could be subject based or population based.

According to a preferred embodiment the reference data is received comprising measured data of a subject and/or a population correlated to at least one predetermined reference LoA, combined with DoS and/or LoP respectively. From the reference data there is determined at least one correlation of at least one predetermined reference LoA, combined with DoS and/or LoP respectively and at least one respective group indicator extracted. A subsequently measured LoA can then be scaled and/or indexed combined with DoS and/or LoP with respect to the at least one predetermined reference LoA, combined with DoS and/or LoP respectively by means of said at least one respective correlation, and optionally determining a level of anxiety index or LoAI, and a Depth of State index or DoSI and/or a level of pain index or LoPI therefrom.

Described herein are one or more indexes, which are standardized indexes indicating the level of anxiety as experienced by the subject. Typically, an index is standardized or normalized as compared to a reference. As mentioned above such a reference could for example be subject-based or population based.

Indexes described herein include a level of anxiety index (LoAI).

A level of anxiety index (LoAI) is a standardized index of the level of anxiety (LoA) indicating the level of anxiety as experienced by the subject. Such an index also allows to determine how much the LoA has been modified, for example by a treatment session, in particular by a pharmacological, and/or non-pharmacological treatment session as compared to a reference state.

An index, for example one or more of LoAI, may have a scale with a first and a second limit. The first limit may represent a lowest level of anxiety, or alternatively a lowest level of anxiety associated with no anxiety and the second limit may represent the highest level of anxiety when compared to a reference state. The first limit may represent an initial state or a reference state of the subject and/or population. The first limit may represent a normal level of anxiety and the second limit may represent a highest level of anxiety. The first limit may represent a normal level of anxiety, and the second limit may represent a significantly increased level of anxiety. The first and second limits may be numerically represented e.g. 0 to 20, 20 to 0, 0 to 60, 60 to 0, 0 to 100, 100 to 0, 0 to 1, 1 to 0 respectively. The second limit may be higher than the first limit, or the first limit may be higher than the second limit. The choice of a higher number for the second limit may depend on the subject or user perspective. An anesthetist may have a preference that the first limit is higher (e.g. 1, 60, 100, 100%) than the second limit (e.g. 0, 0%).

A subject with an index, for example a LoAI which remains close to the first limit, for instance, can be safely approached for further treatments, for example as long as the level of anxiety remains sufficiently low.

The index may have a scale between the first and second limits; the scale may be linear, logarithmic, or other. The scale may be continuous, categorical, discrete, percentage, ratio.

The index, e.g. LoAI, may be indicative of a number of different levels of anxiety or different LoA, for the subject. The number of levels may be any, for instance 3 to 10, preferably 4 to 8 levels. There may be 4, 6, or 8 levels. The levels may be divided within the first and second limits. The division may be even (linear), logarithmic, or according to another scheme. The lowest level (e.g. 1st level) may correlate with or contain the first limit, the highest level (e.g. 4th level) may correlate with or contain the second limit. When an index is a value between 100 (first limit) and 0 (second limit) and the number of levels is 4, the 4 levels may be 100-76 (1st level), 75 to 51 (2nd level), 50 to 26 (3rd level), 25 to 0 (4th level). It is appreciated that the end points of the levels may allow a continuous numerical scale (not interrupted) into the next level. When the index is a value between 100 (first limit) and 0 (second limit) and the number of levels is 8, the 8 levels may be 100-87.5 (1st level), 87.5 to 75 (2nd level), 75 to 62.5 (3rd level), 62.5 to 50 (4th level), 50 to 37.5 (5th level), 37.5 to 25 (6th level), 25 to 12.5 (7th level), 12.5 to 0 to (8th level). The skilled person would be able to determine the extent of the scale (e.g. 100 to 0, or 60 to 0), the number of levels within the scale (e.g. 4, 6 or 8), the type of scale (e.g. linear, logarithmic) and the boundaries between the levels according to the situation.

The index may be a subject-based index in which state of each subject is compared to a reference state of the subject itself or of a group of subjects, or one or more particular populations. The index may be a population-based index in which the state of each subject is compared to a population-based reference. It is clear that alternatively, the index may also be scaled according to an absolute scale. It is clear that any suitable combination of relative, absolute, individual and/or population-based could be used to scale the index.

With respect to the subject-based index, a reference state e.g. a reference value for LoA, is for example measured for the subject before the treatment session. During or after the treatment session, based on response data comprising measured data, real-time values for one or more of LoA is measured for the subject. During the treatment session, based on a comparison between the reference value and the real-time LoA values, real-time values for one or more of LoAI is calculated.

With respect to the population-based index, a reference state e.g. a reference value for one or more of LoA is for example drawn from a database. The database for example comprises reference values that are based on population studies. The reference value may vary according to certain factors include age, gender, ethnicity, intervention characteristics hence, the reference value may be adapted according to the subject and/or the user. During, or after the treatment session, based on response data comprising measured data, real-time values for one or more of LoA is measured for the subject. During, or after the treatment session, based on a comparison between the database comprising the population-based reference value and the real-time LoA values, real time values for one or more of LoAI is calculated.

It is clear that, alternatively, for example the LoA could be scaled according to a subject-based or population-based absolute scale, such that no further reference measurement is required, and that for example measurement of LoA to determine real-time values could be performed without the need for any treatment session. In other words, with a subject-based or population-based absolute scale it is possible to measure LoA and/or LoAI in any suitable context.

The response data may be transformed into an index e.g. into a LoAI using an evaluation protocol. The evaluation protocol may comprise use of one or more of a mathematical (e.g. statistical) model, trained machine-leaning model, mathematical index, reference data. The evaluation protocol takes as input the response data, and outputs the index.

The evaluation protocol may include a step of extracting from the response data of the group 8 indicator and optionally one or more of the Group 6, 7, 9, 10 and/or 11 indicator.

The measured data may comprise one or more data components. A data component is attributed to a single measurement data, for example EEG data, ECG data, ... , a single observation data, for example movement, skin color, ..., or a single self-reported event data, for example a self-reported level of anxiety, ....

The evaluation protocol may weight the data components of the response data equally or differently. The weighting given to a data component depends, amongst other things, on relevance and precision of the component. For instance, the evaluation protocol may give EEG data a higher rating, reflecting its high relevance and precision. The evaluation protocol may use data components which have less relevance and precision, that are still indicative enough for the situation. In some situations, the response data may comprise a smaller number of data components that have high relevance and precision that are indicative enough for the situation. In some situations, the response data may comprise a larger number of data components that have lower relevance and precision that are indicative enough for the situation. It is clear that various embodiments are possible in which for example according to some embodiments the weighting given to the different group indicators mentioned throughout this description for calculation of the LoA could be adapted in order to adjust and/or fine-tune the calculation of the LoA depending on for example the desired weight and/or effect of the different dimensions of the LoA. According to some embodiments the weighting could be configured to provide a greater weight to the group 8 indicator, when the LoA is measured in a context where the cognitive dimension of LoA outweighs that of the other dimensions of LoA. However it is clear that according to alternative embodiments the group 8 indicator could be provided with a lower weight, and/or be outweighed by other group indicators in a context in which the combination of the cognitive dimension of the LoA with other dimensions of the LoA is more important.

The evaluation protocol may be refined as more subjects are evaluated using the method. A method of refining the evaluation protocol may comprise:

- receiving response data of a subject, for example representing a subject's response to a treatment session,
- receiving independently measured data of LoA(I),
- using the response data and independently measured data to refine the evaluation protocol.

A method of refining the evaluation protocol may comprise:

- Receiving measured response data of a subject, for example representing a subject's response to a treatment session,
- Receiving self-reported and/or observational measure of LoA(I);
- Receiving independently measured data of LoA(I);
- Using the response data, the self-reported and/or observational data, and the independently measured data to refine the evaluation protocol.

The evaluation protocol may additionally or alternatively be refined using analytics. The data collected before, during and/or after one or more previous measurements, and/or one or more previous treatment sessions may contribute a refinement of the evaluation protocol.

As the evaluation protocol is created and/or refined, it may be apparent that one or more data components of the response data become redundant in measuring, or in other words determining, and/or monitoring the level of Anxiety, or for example the modification of the Level of Anxiety by means of a pharmacological and/or non-pharmacological treatment, for example involving an anxiety treatment, a treatment involving the modification of the state of consciousness of a subject, etc.. According to a preferred embodiment at least one, preferably all, of the measured data, observation data and response data are used to create and/or refine the evaluation protocol. According to a preferred embodiment only measured data is used in the method for measuring the LoA.

An embodiment of an evaluation protocol for determining a LoAI is set out below. Generally speaking, it may for example comprise 5 principal steps:

Step 1: receive and digitize response data comprising EEG data, for example during a treatment session. The EEG data could for example be provided from a P-EEG electrode, and optionally one or more of C-EEG and F-EEG electrodes or any other suitable EEG electrodes and optionally from one or more ECG electrodes. According to still further embodiments, optionally there could be provided suitable capturing units, where applicable comprising suitable sensors and/or electrodes to capture one or more of the following: cardiovascular data, respiratory data, ocular movement data, muscular activity data, body temperature data, etc.

Step 2: generate:
Group 8 indicators P-power(dt) from the digitized response data, and optionally Group 6, 7, 9, 10 and/or 11 indicators.

Step 3: generate one or more indicator values, which means values for any of the group indicators, such as for example group 8 indicator, group 6, 7, 9, 10, 11 indicator, or any other suitable group indicators, from step 2), using for instance, integrative analysis.

Step 4: comparing the value of Step 3) with reference values, for example a population-based index or subject-based index such as mentioned above.

Step 5: obtain from Step 4) a LoAI index e.g. a number of a scale 0 to 20, or a word, or category. It is clear, as explained in further detail in this description that such a LoAI index according to some embodiments could be provided by means of a suitable graphical visualization.

In step 2, the digitized response data may undergo a pre-processing protocol including one or more of re-referencing to average reference, bandpass and/or notch filtering to exclude low and high frequency artifacts, signal segmentation into so called epochs (= sets of x seconds time windows), artifact rejection (ocular, muscular, ...), and baseline adjustment. For time domain analysis of EEG, ECG, EMG, EOG, body temperature, ... signals, there is typically peak detection to for example determine the time period between peaks, peak amplitude, latency (in form of a time series if multiple epochs), and mathematical transformations performed (e.g.: mean or change variable if multiple epochs. The time window for measurement could for example be in the range of 0.01s to 10s, such as for example in the range of 0.1s to 5s.

For a frequency domain analysis of for example EEG signals: there is typically a time to frequency transform (e.g. Fourier transform), relevant frequency bands (e.g. delta-theta (dt)) are extracted, amplitude/power and phase for each frequency band (and each epoch) are determined, and mathematical transformations performed (e.g.: mean if multiple epochs). For signals analyzed in the time domain, peaks can be detected, time interval between peaks, mathematical transformations performed (e.g.: mean if multiple epochs).

From this, one obtains at least one of the following features for at least one electrode: delta-theta frequency band related amplitude/power, delta-theta frequency band related phase, ECG R-R peak interval, .... There may be a normalization step for instance to correct for the time of intervention to ensure that every subject contributes similarly.

In step 3, one or more group indicator values is generated using integrative analysis, for instance, using the equation:

$$LoA_{s,t} = \sum_{i=1}^{n} (\alpha_i f_i(F_{i,s,[t',t]}))$$

Where $\alpha_i$ is an optional parameter that evolves with the population size and characteristics, and may be determined using learning processes based on response data and/or independently measured data of the LoA, $f_i$ is a function determined using learning processes based on response data and/or independently measured data of the LoA, $F_{i,s,[t',t]}$ is the value(s) of the feature obtained from point 2 from time t' to time t (t'<t) and for the subjects. The above-mentioned [t' - t] interval means that the value of LoA at time t may be computed based on features extracted from several $F_{i,s,t}$ values. As example, taking the mean and the standard deviation from the past x milli-seconds leads to a more sensitive and more robust determination of LoA. It is clear that the [t' - t] interval thus refers to the time window mentioned above.

In step 4, there is a comparison of values with reference values

If the index is a subject-based index (each subject is its own control),

$$\Delta_r LoA_{s,t} = S\left(\beta\left(LoA_{s,p}^* - LoA_{s,[t',t]}\right) + K\right)$$

If the index is population-based (absolute) and that subject s is part of population p,

$$\Delta_a LoA_{s,t} = S\left(\beta\left(LoA_p^* - LoA_{s,[t',t]}\right) + K\right)$$

Where S is a scale-up parameter, $\beta$ is a parameter accounting for the population characteristics (age, sex, ethnicity, ...) determined using learning processes based on response data and/or independently measured data of the LoA, $LoA_{s,p}^*$ is the individual/subject-based reference state, $LoA_p^*$ is the absolute/population-based reference state using learning processes based on response data and/or independently measured data of the LoA, and K is a "translation" parameter allowing to adjust thresholds based on user preference.

In step 5 the LoAI index is generated. The value obtained in step 4 is compared with a predefined scale depending on subject/intervention characteristics determined following process described below in the section "refinement of the evaluation protocol", for example using trained machine-learning models and/or artificial intelligence, based on response data and/or independently measured data and/or reference data.

In LoAI, the index may be a population-based index, or in other words an absolute scale, in which a subject is compared with a population and/or any other suitable absolute reference. The first limit may indicate that the level of anxiety corresponds to no anxiety being experienced or being free from any anxiety as determined by the population. The second limit may indicate that the level of anxiety corresponds to experiencing the highest possible level of anxiety as determined by the population. Intermediate values represent intermediate levels of anxiety.

In LoAI, the index may be a subject-based index, or in other words a relative scale, in which each subject is compared with itself. The first limit may indicate that the level of anxiety, corresponds to no anxiety being experienced or being free from any anxiety as determined by the subject itself. The second limit may indicate that the level of anxiety corresponds to experiencing the highest possible level of anxiety as determined by the subject itself.

The method and system may determine an expected LoAI and/or ratio, for example for a point in time before, during and/or after the treatment session, for example based on the particular phase, acts of treatment already performed, etc. before, during and/or after the treatment session. For instance, if the treatment comprises a hypnotic session, an expected LoAI may be determined from the phase, for example one of phases (i) to (iv), as will be described in further detail below, of the hypnotic session, and for example from the position within such a phase. For instance, if the treatment comprises a pharmacological treatment session, an expected LoAI may be determined from the dose, titration, and/or timing of the administration of anxiolytic. The expected LoAI may for example be determined from population data or subject data, namely from historical and/or precedence data.

Because an administered treatment does not always lead to the expected LoA a difference between the measured LoA and the expected LoA provides the user with guidance for corrective measures with respect to the treatment.

It is clear that still further alternative embodiments are possible, in which there is provided a computer-implemented method for measuring, or in other words determining, and/or monitoring a level of anxiety of a subject, which comprises the method for measurement of a level of anxiety, according to one or more of the embodiments as described above, applied on a subject by receiving the measured data of a subject.

According to a further aspect, similar as described above, there is thus also provided a method for determining and/or monitoring a level of anxiety in a subject at at least two different points in time, for example before, during and/or after a treatment. According to such a method, there is measured a LoA at at least two different points in time, for example before, during and/or after the treatment. Based on a comparison of at least two LoA measured at at least two different points in time, there is then determined and/or monitored an evolution, for example a treatment-induced evolution of the LoA. However it is clear that such comparisons could be performed to determine any suitable evolution of the LoA during any suitable time period. Additionally and/or alternatively according to some embodiments the LoA could also be predicted. As for example described in further detail below with respect to Fig. 12 a suitable plurality of historical data points with respect to LoA could provide for a suitable trend analysis, for example based on a trend correlating to the a running average, mean, or any other suitable trend of the historical data points, thereby allowing prediction of future, expected or trending data points. It is clear that in this way a prediction of an evolution of the LoA can be accomplished. Predictive analysis of the historical data points, could be done by means of any suitable method, for example based on predetermined correlations between the evolution of LoA during different phases or time periods of a particular treatment, or by means of self-learning, self-optimizing algorithms, artificial intelligence algorithms, etc. which have been trained on historical data sets in order to detect repeating and/or predictable patterns and/or correlations, thereby allowing for an assessment of detectable patterns and/or correlations in the measured data, thereby allowing for a determination of current and predictive trends based on the measured data. It should be clear that alternative embodiments are possible of historical data points and/or any other suitable data resulting from LoA measurements and/or any other suitable measurement for enabling predictive analysis of current measurements. Such data on which the predictive analysis can be based can for example comprise historical data from individual subjects, from populations, predetermined treatment related patterns of an evolution of LoA, etc. According to still further embodiments, additionally and/or alternatively there may be aggregated a level of anxiety score or LoAS based on an aggregation of at least two LoA determined at at least two different points in time. Such aggregated parameters such as LoAS related to anxiety measurement could be useful in determining the impact of chronic anxiety, in which for example during a longer period, of for example several hours and/or days, the level of anxiety is continuously measured, and the aggregated LoAS is able to provide for an indicator of the impact of a constant, chronic level of anxiety as experienced by a subject as opposed to an instantaneous peak of anxiety. This is useful as even moderate or low levels of anxiety, when experienced chronically, may have a high impact on the quality of life of subjects, while for example short, infrequent bursts of anxiety, even when they are intense can be tolerated more easily. It is clear that such aggregated forms could also be useful for any parameters related to the level of anxiety as detailed in this description, and/or their scaled and/or indexed versions, such as for example LoA(I), etc. thereby allowing for similar scores of these parameters that correspond to the aggregation of at least two values of these parameters. According to particular embodiments the LoA(I)S could for example comprise any suitable embodiment of aggregation of the LoA(I) values over a predetermined time period, such as for example a sum, mean, average, etc. and the value could for example be presented in a GUI of a system similarly as described below, for example presenting the aggregated value, optionally in relation to the time period during which the LoA(I) was aggregated.

According to some treatments, such as for example anxiety treatments, in which for example a pharmacological and/or non-pharmacological treatment is used to reduce a particular level of anxiety the subject is experiencing for example by means of an anxiolytic, it is clear that the goal of the treatment is to reduce the LoA, and that such a reduction in the LoA, for example during and/or after the treatment, corresponds to a desired treatment-induced evolution of the LoA. However according to certain other treatments, for example in a context of a sedative treatment, for example prior to a chirurgical treatment, a dental treatment, etc. , it could be desired to retain the LoA as experienced in the normal awake state in which the subject does not undergo the treatment, also during the treatment. In this way, it is clear that the desired treatment induced evolution of the LoA is the prevention of an increase of the LoA, or the prevention of an increase of the LoA beyond an undesirable threshold. It is clear that according to such embodiments, the treatment-induced evolution of the LoA can for example be based on a comparison of the LoA measured before and during the treatment, or measured before and after the treatment, etc.. It is thus clear that, according to an embodiment in which the treatment is an anxiety reducing treatment, then the efficacy of such an anxiety treatment, such as for example an anxiety-reducing, anxiety-controlling, anxiety-preventing, or any other suitable anxiety treatment, can be determining and/or monitored based on the treatment-induced evolution of the LoA. In other words, when the LoA is sufficiently reduced, for example to below a desired threshold, or when the LoA does not undesirably increase, for example to above an undesired threshold. Preferably, this treatment-induced evolution of the LoA allows to optimizing and/or adapting an anxiety treatment, such as for example an anxiety-reducing, anxiety-controlling, anxiety-preventing, or any other suitable anxiety treatment.

It is further also clear that based on the measured LoA, there could be determining a treatment and/or determining an adjustment to a treatment and/or determining an intensity of a treatment. For example, a higher value for the LoA could lead to a higher dosage of a sedative or anxiolytics. According to another example, if a particular anxiety treatment doesn't result in a decrease of the LoA, doesn't prevent an increase of the LoA, then an alternative treatment could be initiated. The desired level of titration of a pharmacological anxiety treatment, or the desired intensity of a non-pharmacological treatment, such as for example the depth of hypnosis, the intensity of a non-pharmacological anxiety management treatment, could for example be increased or decreased based on a desired LoA or treatment induced change thereto. According to still further embodiments, there based on a measured and/or desired LoA, there could be made a decision to choose and/or adapt and/or replace specific anxiety management treatment and/or any combination and/or mix of anxiety management treatments, such as for example one or more pharmacological and/or non-pharmacological anxiety management treatments.

Described herein are one or more indexes, which are standardized indexes indicating the level or degree to which subject's level of pain or state of consciousness has been modified by the treatment session. Typically an index is standardized or normalized as compared to a reference. As mentioned above such a reference could for example be subject-based or population based.

Indexes described herein include depth of hypnosis index (DoHI), depth of state index (DoSI), depth of dissociation index (DoDI), level of pain index (LoPI), a level of anxiety index (LoAI)

A level of anxiety index (LoAI) is a standardized index of the level of anxiety (LoA) indicating the level of anxiety as experienced by the subject. Such an index also allows to determine how much the LoA has been modified, for example by a treatment session, in particular by a pharmacological, and/or non-pharmacological treatment session as compared to a reference state.

A level of pain index (LoPI) is a standardized index of the level of pain (LoP) indicating the level of pain as consciously perceived by the subject. Such an index also allows to determine how much the LoP has been modified, for example by a treatment session, in particular by a pharmacological, and/or non-pharmacological treatment session as compared to a reference state.

A depth of state index (DoSI) is a standardized index of the depth of state (DoS) indicating the level or degree to which the state of consciousness of the subject has been modified, for example by means of a treatment session that is non-pharmacological (e.g. hypnotic) treatment session and/or by a pharmacological treatment session, as compared to a reference state.

A depth of hypnosis index (DoHI) is a standardized index of the depth of hypnosis (DoH) indicating the level or degree to which the state of consciousness of the subject has been modified by a treatment session that is non-pharmacological (e.g. hypnotic), as compared to a reference state.

A depth of dissociation index (DoDI) is a standardized index of the depth of dissociation (DoD) indicating the level or degree to which dissociation of the subject has been modified by the treatment session, in particular by a non-pharmacological treatment session as compared to a reference state.

An index, for example one or more of DoHI, DoSI, DoDI, LoPI, LoAI may have a scale with a first and a second limit. The first limit may represent a lowest level of pain, or alternatively a lowest level of pain associated with no pain, or a normal awake state of consciousness and the second limit may represent the highest level of pain, or the deepest modified state of consciousness when compared to a reference state. The first limit may represent an initial state or a reference state of the subject and/or population. The first limit may represent a normal level of pain or a normal awake state or only a lightly modified state of consciousness and the second limit may represent a highest level of pain or a fully or deeply modified state of consciousness. The first limit may represent a normal level of pain, and the second limit may represent a significantly increased level of pain. The first limit may represent a light level of trance and the second limit may represent a deep level of trance or vice versa. The first and second limits may be numerically represented e.g. 0 to 20, 20 to 0, 0 to 60, 60 to 0, 0 to 100, 100 to 0, 0 to 1, 1 to 0 respectively. The second limit may be higher than the first limit, or the first limit may be higher than the second limit. The choice of a higher number for the second limit may depend on the subject or user perspective. An anesthetist may have a preference that the first limit is higher (e.g. 1, 60, 100, 100%) than the second limit (e.g. 0, 0%). The latter is for example illustrated in some of the embodiments in this description with respect to DoS, in which a normal awake state or a state with a high / increased / maximum level of consciousness, corresponds to a DoS of for example 100%, which is then an embodiment of the first limit and in which a deeply dissociated state / immersive state or a state with a low / decreased / minimum level of consciousness corresponds to a DoS of for example 0%, which is then an embodiment of the second limit.

A subject with an index, for example DoSI, DoHI, DoDI towards the second limit and a LoPI and/or LoAI which remains closer to the first limit, for instance, can be safely operated on by surgery when the perception of the level of pain, the level anxiety and/or the state of consciousness has been sufficiently modified in order to attain the desired analgesic, anxiolytic and/or anesthetic effect.

The index may have a scale between the first and second limits; the scale may be linear, logarithmic, or other. The scale may be continuous, categorical, discrete, percentage, ratio.

The index, e.g. one or more of DoHI, DoSI, DoDI, LoPI, LoAI may be indicative of a number of different levels of consciousness, such as for example a depth of state (DoSI), a depth of dissociation (DoDI), or may be indicative of a number of different levels of pain (LoPI), or may be indicative of a number of different levels of anxiety (LoAI) for the subject. The number of levels may be any, for instance 3 to 10, preferably 4 to 8 levels. There may be 4, 6, or 8 levels. The levels may divided be within the first and second limits. The division may be even (linear), logarithmic, or according to another scheme. The lowest level (e.g. 1st level) may correlate with or contain the first limit, the highest level (e.g. 4th level) may correlate with or contain the second limit. When an index is a value between 100 (first limit) and 0 (second limit) and the number of levels is 4, the 4 levels may be 100-76 (1st level), 75 to 51 (2nd level), 50 to 26 (3rd level), 25 to 0 (4th level). It is appreciated that the end points of the levels may allow a continuous numerical scale (not interrupted) into the next level. When the index is a value between 100 (first limit) and 0 (second limit) and the number of levels is 8, the 8 levels may be 100-87.5 (1st level), 87.5 to 75 (2nd level), 75 to 62.5 (3rd level), 62.5 to 50 (4th level), 50 to 37.5 (5th level), 37.5 to 25 (6th level), 25 to 12.5 (7th level), 12.5 to 0 to (8th level). The skilled person would be able to determine the extent of the scale (e.g. 100 to 0, or 60 to 0), the number of levels within the scale (e.g. 4, 6 or 8), the type of scale (e.g. linear, logarithmic) and the boundaries between the levels according to the situation.

The index may be a subject-based index in which state of each subject is compared to a reference state of the subject itself, or of a group of subjects, or one or more particular populations. The index may be a population-based index in which the state of each subject is compared to a population-based reference. It is clear that alternatively, the index may also be scaled according to an absolute scale. It is clear that any suitable combination of relative, absolute, individual and/or population-based could be used to scale the index.

With respect to the subject-based index, a reference state e.g. a reference value for one or more of LoA, LoP, DoS, DoD, DoH is for example measured for the subject before the treatment session. During or after the treatment session, based on response data comprising measured data, real-time values for one or more of LoA, LoP, DoS, DoD, DoH is measured for the subject. During the treatment session, based on a comparison between the reference value and the real-time LoA, LoP, DoS, DoD, DoH values, real-time values for one or more of LoAI, LoPI, DoSI, DoDI, DoHI is calculated.

With respect to the population-based index, a reference state e.g. a reference value for one or more of LoA, LoP, DoS, DoD, DoH is for example drawn from a database. The database for example comprises reference values that are based on population studies. The reference value may vary according to certain factors include age, gender, ethnicity, intervention characteristics hence, the reference value may be adapted according to the subject and/or the user. During, or after the treatment session, based on response data comprising measured data, real-time values for one or more of LoA, LoP, DoS, DoD, DoH is measured for the subject. During, or after the treatment session, based on a comparison between the database comprising the population based reference value and the real-time LoA, LoP, DoS, DoD, DoH values, real time values for one or more of LoAI, LoPI, DoSI, DoDI, DoHI is calculated.

It is clear that, alternatively, for example the LoP and/or the LoA could be scaled according to a subject-based of population-based absolute scale, such that no further reference measurement is required, and that for example measurement of LoP and/or LoA to determine real-time values could be performed without the need for any treatment session. In other words, with a subject-based or absolute and/or population-based scale it is possible to measure LoP and/or LoA in any suitable context.

The response data may be transformed into an index e.g. into a LoAI, LoPI, DoDI, DoHI, DoSI using an evaluation protocol. The evaluation protocol may comprise use of one or more of a mathematical (e.g. statistical) model, trained machine-leaning model, mathematical index, reference data. The evaluation protocol takes as input the response data, and outputs the index.

The evaluation protocol may include a step of extracting from the response data one or more of:

- the group 1 indicator and optionally the group 2 indicator for determining DoS(I);
- the group 4 indicator and optionally the group 3 indicator, and/or the group 5 indicator for determining LoP(I); and/or
- the group 8 indicator and optionally the group 6 indicator, the group 7 indicator, the group 9 indicator, the group 10 indicator, and/or the group 11 indicator for determining LoA(I).

The measured data may comprise one or more data components. A data component is attributed to a single measurement data, for example EEG data, EMG data, muscular activity data, EDA data, ECG data, cardiovascular data, respiratory data, ocular movement data, EOG data, body temperature data .. , a single observation data, for example movement, skin color, ... , or a single self-reported event data, for example a self-reported level of anxiety, a self-reported level of pain perception, a self-reported level of dissociation, ...

The evaluation protocol may weight the data components of the response data equally or differently. The weighting given to a data component depends, amongst other things, on relevance and precision of the component. For instance, the evaluation protocol may give EEG data may have a higher rating, reflecting its high relevance and precision. The evaluation protocol may use data components which have less relevance and precision, that are still indicative enough for the situation. In some situations, the response data may comprise a smaller number of data components that have high relevance and precision that are indicative enough for the situation. In some situations, the response data may comprise a larger number of data components that have lower relevance and precision that are indicative enough for the situation.

The evaluation protocol may be refined as more subjects are evaluated using the method. A method of refining the evaluation protocol may comprise:

- receiving response data of a subject, for example representing a subject's response to a treatment session,
- receiving independently measured data of one or more of LoA(I), LoP(I), DoS(I), DoD(I), DoH(I),
- using the response data and independently measured data to refine the evaluation protocol.

A method of refining the evaluation protocol may comprise:

- Receiving measured response data of a subject, for example representing a subject's response to a treatment session,
- Receiving self-reported and/or observational measure of LoA, LoP, DoS, DoH and/or DoD;
- Receiving independently measured data of LoA(I), LoP(I), DoS(I), DoH(I) and/or DoD(I);
- Using the response data, the self-reported and/or observational data, and the independently measured data to refine the evaluation protocol.

The evaluation protocol may additionally or alternatively be refined using analytics. The data collected before, during and/or after one or more previous measurements, and/or one or more previous treatment sessions may contribute a refinement of the evaluation protocol.

As the evaluation protocol is created and/or refined, it may be apparent that one or more data components of the response data become redundant in determining and/or monitoring the level of Pain, or for example the modification of the Level of Pain by means of a pharmacological and/or non-pharmacological treatment, for example involving a modification of the state of consciousness of a subject. According to a preferred embodiment at least one, preferably all of the measured data, observation data and response data are used to create and/or refine the evaluation protocol. According to a preferred embodiment only measured data is used in the method for measuring the LoP.

An embodiment of an evaluation protocol for determining a LoPI is set out below. Generally speaking, it may for example comprise 5 principal steps:

Step 1: receive and digitize response data comprising EEG data, for example during a treatment session. The EEG data could for example be provided from one or more of C-EEG, P-EEG and F-EEG electrodes, or any other suitable EEG electrodes and optionally from one or more EMG electrodes.

Step 2: generate:

Group 4 indicators power(ta), for example one or more of C-power (ta), P-power (ta), F-power (ta), from the digitized response data, and/or
Group 3 indicators MSPA, for example one or more of C-MSPA, P-MSPA, F-MSPA, and/or
Group 5 indicator EMG, for example EMG-MSPA.

Step 3: generate one or more indicator values, which means values for any of the group indicators, such as for example group 4 indicator, group 3 indicator, group 5 indicator, or any other suitable group indicators, from step 2), using for instance, integrative analysis.

Step 4: comparing the value of Step 3) with reference values, for example a population-based index or subject-based index such as mentioned above.

Step 5: obtain from Step 4) a LoPI index *e.g.* a number of a scale 0 to 20, or a word, or category.

In step 2, the digitized response data may undergo a pre-processing protocol including one or more of re-referencing to average reference, bandpass and/or notch filtering to exclude low and high frequency artifacts, signal segmentation into so called epochs (= sets of x seconds time windows), artifact rejection (ocular, muscular, ...), and baseline adjustment. For time domain analysis of EEG signals, there is typically peak detection to determine peak amplitude and latency (in form of a time series if multiple epochs), and mathematical transformations performed (e.g.: mean or change variable if multiple epochs). For a frequency domain analysis of EEG signals: there is typically a time to frequency transform (e.g. Fourier transform), relevant frequency bands (e.g. theta-alpha (ta)) are extracted, amplitude/power and phase for each frequency band (and each epoch) are determined, and mathematical transformations performed (e.g.: mean if multiple epochs).

For each pair of EMG electrode (and each epoch) signals are analyzed in the time domain, peaks are detected, mean signal peak-to-peak amplitude (MSPA) and MSPA latency are determined, mathematical transformations performed (e.g.: mean if multiple epochs).

From this, one obtains at least one of the following features for at least one electrode: MSPA amplitude, MSPA power, MSPA latency, theta-alpha (ta) frequency band related amplitude/power, theta-alpha (ta)frequency band related phase, EMG MSPA, EMG MSPA latency. There may be a normalization step for instance to correct for the time of intervention to ensure that every subject contributes similarly.

In step 3, one or more group indicator values is generated using integrative analysis, for instance, using the equation:

$$LoP_{s,t} = \sum_{i=1}^{n} (\alpha_i \, f_i(F_{i,s,[t',t]}))$$

Where $\alpha_i$ is an optional parameter that evolves with the population size and characteristics, and may be determined using learning processes based on response data and/or independently measured data of the LoP, $f_i$ is a function determined using learning processes based on response data and/or independently measured data of the LoP, $F_{i,s,[t',t]}$ is the value(s) of the feature obtained from point 2 from time t' to time t (t'<t) and for the subjects. The above-mentioned [t' - t] interval means that the value of LoP at time t may be computed based on features extracted from several $F_{i,s,t}$ values. As example, taking the mean and the standard deviation from the past x milli-seconds leads to a more sensitive and more robust determination of LoP. It is clear that the [t' - t] interval thus refers to the time-window mentioned above.

In step 4, there is a comparison of values with reference values

If the index is a subject-based index (each subject is its own control),

$$\Delta_r LoP_{s,t} = S \left( \beta \left( LoP_{s,p}^* - LoP_{s,[t',t]} \right) + K \right)$$

If the index is absolute and/or population-based and that subject s is part of population p,

$$\Delta_a LoP_{s,t} = S \left( \beta \left( LoP_p^* - LoP_{s,[t',t]} \right) + K \right)$$

Where S is a scale-up parameter, $\beta$ is a parameter accounting for the population characteristics (age, sex, ethnicity, ...) determined using learning processes based on response data and/or independently measured data of the LoP, $LoP_{s,p}^*$ is the individual/subject-based reference state, $LoP_p^*$ is the absolute/population-based reference state using learning processes based on response data and/or independently measured data of the LoP, and K is a "translation" parameter allowing to adjust thresholds based on user preference.

In step 5 the LoPI index is generated. The value obtained in step 4 is compared with a predefined scale depending on subject/intervention characteristics determined following process described below in the section "refinement of the evaluation protocol", for example using trained machine-learning models and/or artificial intelligence, based on response data and/or independently measured data and/or reference data.

In LoPI, the index may be a population-based index, or in other words an absolute scale, in which a subject is compared with a population and/or any other suitable absolute reference. The first limit may indicate that the level of pain corresponds to no pain being perceived or being free from any pain as determined by the population. The second limit may indicate that the level of pain corresponds to perceiving the highest possible level of pain as determined by the population. Intermediate values represent intermediate levels of pain.

In LoPI, the index may be a subiect-based index, or in other words a relative scale, in which each subject is compared with itself. The first limit may indicate that the level of pain, corresponds to no pain being perceived or being free from any pain as determined by the subject itself. The second limit may indicate that the level of pain corresponds to perceiving the highest possible level of pain as determined by the subject itself.

The evaluation protocol may include a step of extracting from the response data one or more of the Group 1 indicator, Group 2 indicator for determining embodiments of DoS(I), DoD(I), and/or DoH(I).

An example of an evaluation protocol for determining a DoDI is set out below. Generally speaking, it may comprise 5 principal steps:

Step 1: receive and digitise response data during a treatment session from F-EEG- electrode and optionally P-EEG electrode.
Step 2: generate:

Group 1 indicators *i.e.* F-power (dt), and optionally P-power (dt) from the digitised response data and/or Group 2 indicators *i.e.* F-MSPA.

Step 3: generate one or more indicator value from step 2), using for instance, integrative analysis. As explained above with respect to the group 1 indicator, when P-Power(dt) is used in step 2, then it is always in combination with F-power(dt) and with a lower weight than F-power(dt).
Step 4: comparing the value of Step 3) with reference values (population-based index or subject-based index)
Step 5: obtain from Step 4) a DoDI index e.g. a number of a scale 0 to 20, or a word, or category.

In step 2, the digitised response data may undergo a pre-processing protocol including one or more of re-referencing to average reference, bandpass and/or notch filtering to exclude low and high frequency artifacts, signal segmentation into so called epochs, artifact rejection (ocular, muscular, ...), and baseline adjustment. Segmentation into epochs refers to a procedure in which specific time-windows are extracted from the continuous EEG signal. These time windows are called "epochs", and usually are time-locked with respect an event, e.g. electrical stimulus. ERP or MSPA are measured based on epochs. For time domain analysis of EEG signals, there is typically peak detection to determine peak amplitude and latency (in form of a time series if multiple epochs), and mathematical transformations performed (e.g.: mean or change variable if multiple epochs). For a frequency domain analysis of EEG signals: there is typically a time to frequency transform (e.g. Fourier transform), relevant frequency bands (e.g. delta-theta (td)) are extracted, amplitude/power and phase for each frequency band (and each epoch) are determined, and mathematical transformations performed (e.g: mean if multiple epochs). From this, one obtain at least one of the following features for at least one electrode: delta-theta (td) frequency band related amplitude/power, delta-theta (td) frequency band related phase, EEG mean signal peak-to-peak amplitude (MSPA), EEG MSPA latency. There may be a normalization step for instance to correct for the time of intervention to ensure that every subject contributes similarly.

In step 3, one or more indicator values are generated using integrative analysis, for instance, using the equation:

$$DoD_{s,t} = \sum_{i=1}^{n} \left( \alpha_i \, f_i(F_{i,s,[t',t]}) \right)$$

Where $\alpha_i$ is an optional parameter that evolves with the population size and characteristics, and may be determined using learning processes based on response data and/or independently measured data of the DoD, $f_i$ is a function determined using learning processes based on response data and/or independently measured data of the DoD, $F_{i,s,[t',t]}$ is the value(s) of the feature obtained from point 2 from time t' to time t (t'<t) and for the subjects. The upper [t' - t] interval means that the value of dissociation at time t may be computed based on features extracted from several $F_{i,s,t}$ values. As example, taking the mean and the standard deviation from the past x milli-seconds leave to a more sensitive and more robust determination of DoDI. It is clear that the [t' - t] interval thus refers to the time window mentioned above.

In step 4, there is a comparison of values with reference values

If the index is a subject-based index (each subject is its own control),

$$\Delta_r DoD_{s,t} = S \left( \beta \left( DoD_{s,p}^* - DoD_{s,[t',t]} \right) + K \right)$$

If the index is population-based (absolute) and that subject s is part of population p,

$$\Delta_a DoD_{s,t} = S \left( \beta \left( DoD_p^* - DoD_{s,[t',t]} \right) + K \right)$$

Where S is a scale-up parameter, $\beta$ is a parameter accounting for the population characteristics (age, sex, ethnicity, ...) determined using learning processes based on response data and/or independently measured data of the DoD, $DoD_{s,p}^*$ is the individual/subject-based reference state, $DoD_p^*$ is the absolute/population-based reference state determined using learning processes based on response data and/or independently measured data of the DoD, and K is a "translation" parameter allowing to adjust thresholds based on user preference.

In step 5 the DoDI index is generated. The value obtained in step 4 is compared with a predefined scale depending on subject/intervention characteristics determined following process described in the refine protocol below (Using trained machine-learning models and/or artificial intelligence, based on collected measured, observed and self-reported data / reference data).

In DoDI, the index may be a population-based index_(absolute scale, a subject is compared with a population). The first limit may represent "no dissociation", or full attention regarding stimulus coming from the actual environment and/or regarding internal event. The second limit may represent "the most complete dissociation", or complete inattention regarding stimulus coming from the actual environment and/or regarding internal event; the subject has fully a feeling of being present in an alternative reality. Intermediate values represent intermediate levels of dissociation which are intermediate levels of inattention regarding stimulus coming from the actual environment and/or regarding internal event.

In DoDI, the index may be a subject-based index (relative scale, each subject compared with itself). The first limit may indicate that the subject is dissociated as initially (reference state). The subject's degree of inattention regarding stimulus coming from the actual environment and/or regarding internal event is the same as before the treatment session. The second limit may indicate that the subject is fully dissociated. The subject's degree of inattention regarding stimulus coming from the actual environment and/or regarding internal event is maximal (for itself); the subject has fully a feeling of being present in an alternative reality. Intermediate values represent intermediate levels of dissociation which are intermediate levels of inattention regarding stimulus coming from the actual environment and/or regarding internal event.

An example of an evaluation protocol for determining a DoHI is set out below. In this protocol, steps 1 and 2 generate one or more groups indicators 1 to 2 from the response data. Generally speaking, it may comprise 5 principal steps:

Step 1: receive and digitise response data during a treatment session from F-EEG- electrode and optionally P-EEG electrode;
Step 2: generate one or more of Group indicators 1 to 2; As explained above with respect to the group 1 indicator, when P-Power(dt) is used in step 2, then it is always in combination with F-power(dt) and with a lower weight than F-power(dt).
Step 3: generate one or more indicator values from step 2), using for instance, integrative analysis;
Step 4: comparing the value of Step 3) with reference values (population-based index or subject-based index);
Step 5: obtain from Step 4) a DoHI index e.g. a number of a scale 0 to 20, or a word, or category.

Alternatively, the evaluation protocol for determining a DoHI may be based on one or more of DoDI already generated. Steps 1 and 2 may be replaced with steps of obtaining one or more of DoDI, and normalising them.

Step 1: Obtain DoDI;
Step 2: Normalise one or more of DoDI;
Step 3: generate one or more indicator values from step 2), using for instance, integrative analysis;
Step 4: comparing the value of Step 3) with reference values (population-based index or subject-based index);
Step 5: obtain from Step 4) a DoHI index e.g. a number of a scale 0 to 20, or a word, or category.

In step 2, the normalise step is a transformation to bring one or more of DoDI to a comparable scale.
In step 3, one or more indicator values is generated using integrative analysis, for instance, using the equation:

$$DoH_{s,t} = \sum_{i=1}^{n} \left( \alpha_i \, f_i (F_{i,s,[t',t]}) \right)$$

Where $\alpha_i$ is an optional parameter that evolves with the population size and characteristics, and may be determined using learning processes based on response data and/or computed DoD, DoH and/or independently measured data of the DoD, DoH, $f_i$ is a function determined using learning processes based on response data and/or computed DoD, DoH and/or independently measured data of the DoD, DoH, $F_{i,s,[t',t]}$ is the value(s) of the feature obtained from point 2 from time t' to time t (t'<t) and for the subjects. It is clear that the [t' - t] interval thus refers to the time window mentioned above.

In step 4, there is a comparison of values with reference values.
If the index is a subject-based index (each subject is its own control),

$$\Delta_r DoH_{s,t} = S\left(\beta\left(DoH_{s,p}^* - DoH_{s,t}\right) + K\right)$$

If the index is population-based (absolute) and that subject s is part of population p,

$$\Delta_a DoH_{s,t} = S\left(\beta\left(DoH_p^* - DoH_{s,t}\right) + K\right)$$

Where S is a scale-up parameter, $\beta$ is a parameter accounting for the population characteristics (age, sex, ethnicity, ...) determined using learning processes based on response data and/or computed DoD, DoH and/or independently measured data of the DoD, DoH, , $DoH_{s,p}^*$ is the individual/subject-based reference state, $DoH_p^*$ is the absolute/population-based reference state determined using learning processes based on response data and/or computed DoD, DoH and/or independently measured data of the DoD, DoH, and K is a "translation" parameter allowing to adjust thresholds based on user preference.

In step 5 the DoHI index is generated. The value obtained in step 4 is compared with a predefined scale depending on subject/intervention characteristics determined following process described below in the section "refinement of the evaluation protocol", (Using trained machine-learning models and/or artificial intelligence, based on collected measured, observed and self-reported data / reference data).

In DoHI, the index may be a population-based index_(absolute scale, a subject is compared with a population). The first limit may indicate that the subject level of consciousness is similar to the level of consciousness generally observed for normal awake subjects. The second limit may represent the deepest modified state of consciousness, or completely reduced perception of peripheral stimuli and a subject's modified perception of the self and the self in the environment. Intermediate values represent intermediate levels of modified state of consciousness.

In DoHI, the index may be a subject-based index (relative scale, each subject compared with itself). The first limit may indicate that the subject state of consciousness is not (or not significantly) modified as compared to the reference state; Both the subject perception of peripheral stimuli and perception of the self and the self in the environment are not modified as compared to the reference state. The second limit may indicate that the subject state of consciousness is fully/deeply modified as compared to the reference state. Intermediate values represent intermediate levels of modified state of consciousness.

An example of an evaluation protocol for determining a DoSI is set out below. In this protocol, steps 1 and 2 generate one or more groups indicators 1 to 2 from the response data. Generally speaking, it may comprise 5 principal steps:

Step 1: receive and digitise response data during a treatment session from F-EEG- electrode and optionally P-EEG electrode.
Step 2: generate one or more of Group indicators 1 to 2; As explained above with respect to the group 1 indicator, when P-Power(dt) is used in step 2, then it is always in combination with F-power(dt) and with a lower weight than F-power(dt).
Step 3: generate one or more indicator values from step 2), using for instance, integrative analysis
Step 4: comparing the value of Step 3) with reference values (population-based index or subject-based index)
Step 5: obtain from Step 4) a DoSI index e.g. a number of a scale 0 to 20, or a word, or category.

Alternatively, the evaluation protocol for determining a DoSI may be based on one or more of DoDI already generated. Steps 1 and 2 may be replaced with steps of obtaining one or more of DoDI, and normalising them.

Step 1: Obtain DoDI.
Step 2: Normalise one or more of DoDI.
Step 3: generate one or more indicator values from step 2), using for instance, integrative analysis
Step 4: comparing the value of Step 4) with reference values (population-based index or subject-based index)
Step 5: obtain from Step 3) a DoSI index e.g. a number of a scale 0 to 20, or a word, or category.

Alternatively, the evaluation protocol for determining a DoSI may be based on one or more of DoDI already generated. Steps 1 and 2 may be replaced with steps of obtaining one or more of DoDI, and normalising them.

> Step 1: Obtain one or more of DoDI, a measure of state consciousness induced pharmacologically, a measure of state of consciousness induced by other treatments;
> Step 2: Normalise one or more of DoDI, measure of state of consciousness induced pharmacologically, measure of state of consciousness induced by other treatments;
> Step 3: generate one or more indicator values from step 2), using for instance, integrative analysis
> Step 4: comparing the value of Step 4) with reference values (population-based index or subject-based index)
> Step 5: obtain from Step 3) a DoSI index e.g. a number of a scale 0 to 20, or a word, or category.

In step 2, the normalise step is a transformation to bring one or more of DoDI, measure of state of consciousness induced pharmacologically, measure of state of consciousness induced by other treatments to a comparable scale.

In step 3, one or more indicator values is generated using integrative analysis, for instance, using the equation:

$$DoS_{s,t} = \sum_{i=1}^{n} \left( \alpha_i \, f_i(F_{i,s,[t',t]}) \right)$$

Where $\alpha_i$ is an optional parameter that evolves with the population size and characteristics, using learning processes based on response data and/or computed one or more of DoD, DoH, DoS, measure of state of consciousness induced pharmacologically, measure of state of consciousness induced by other treatments and/or independently measured data of the one or more of DoD, DoH, DoS, measure of state of consciousness induced pharmacologically, measure of state of consciousness induced by other treatments , $f_i$ is a function determined using learning processes based on response data and/or computed one or more of DoD, DoH, DoS, measure of state of consciousness induced pharmacologically, measure of state of consciousness induced by other treatments and/or independently measured data of the one or more of DoD, DoH, measure of state of consciousness induced pharmacologically, measure of state of consciousness induced by other treatments, $F_{i,s,[t',t]}$ is the value(s) of the feature obtained from point 2 from time t' to time t (t'<t) and for the subjects. It is clear that the [t' - t] interval thus refers to the time window mentioned above.

In step 4, there is a comparison of values with reference values
If the index is a subject-based index (each subject is its own control),

$$\Delta_r DoS_{s,t} = S \left( \beta \left( DoS_{s,p}^* - DoS_{s,t} \right) + K \right)$$

If the index is population-based (absolute) and that subject s is part of population p,

$$\Delta_a DoS_{s,t} = S \left( \beta \left( DoS_p^* - DoS_{s,t} \right) + K \right)$$

Where S is a scale-up parameter, $\beta$ is a parameter accounting for the population characteristics (age, sex, ethnicity, ...)using learning processes based on response data and/or computed DoD, DoH, DoS, measure of state of consciousness induced pharmacologically, measure of state of consciousness induced by other treatments and/or independently measured data of the DoD, DoH, measure of state of consciousness induced pharmacologically, measure of state of consciousness induced by other treatments, $DoS_{s,p}^*$ is the individua/subject-based reference state, $DoS_p^*$ is the absolute/population-based reference state determined using learning processes based on response data and/or computed DoD, DoH, DoS and/or independently measured data of the DoD, DoH, Do, and K is a "translation" parameter allowing to adjust thresholds based on user preference.

In step 5 the DoSI index is generated. The value obtained in step 4 is compared with a predefined scale depending on subject/intervention characteristics determined following process described below in the section "refinement of the evaluation protocol", (Using trained machine-learning models and/or artificial intelligence, based on collected measured, observed and self-reported data / reference data).

In DoSI, the index may be a population-based index_(absolute scale, a subject is compared with a population). The first limit may represent "no modified state of consciousness". The first limit may indicate that the subject level of consciousness is similar to the level of consciousness generally observed for normal awake subjects. The second limit may represent the deepest modified state of consciousness, or completely reduced perception of peripheral stimuli and a subject's modified perception of the self and the self in the environment. Intermediate values represent intermediate levels of modified state of consciousness. As mentioned elsewhere, DoSI can be a measure of DoHI optionally with measure of state of consciousness induced pharmacologically and optionally with a measure of state of consciousness induced by other treatments.

In DoSI, the index may be a subject-based index (relative scale, each subject compared with itself). The first limit may indicate that the subject state of consciousness is not (or not significantly) modified as compared to the reference state; Both the subject perception of peripheral stimuli and perception of the self and the self in the environment are not modified as compared to the reference state. The second limit may indicate that the subject state of consciousness is fully/deeply modified as compared to the reference state. Intermediate values represent intermediate levels of modified state of consciousness. As mentioned elsewhere, DoSI can be a measure of DoHI optionally with measure of state of consciousness induced pharmacologically and optionally with a measure of state of consciousness induced by other treatments.

When more subjects have been evaluated, more data becomes available which can be used to refine the evaluations protocols for each of DoDI, DoHI and DoSI. Described herein are exemplary ways (a) to (c) in which the respective evaluation protocols can be refined, using at least one, preferably all of measured data, observational data and self-reported data.

(a) Model parameter's incrementation

Model parameters ($\alpha_i, f_i, \beta$) are evaluated based on measured data, and self-reported or independently measured DoD(I), DoH(I) and/or DoSI, using mathematical and/or statistical and/or machine learning processes.

As the evaluation protocol is refined, it may be apparent that one or more data components of the response data become redundant in determining and/or monitoring the level of consciousness / dissociation of a subject. This data will automatically be weighted by a (close to) zero weights $\alpha_i$ when using the upper described method.

(b) Identifying new relevant features for the computation of DoS / DoD / DoH and including them in the evaluation protocol

With new incoming data, it may be apparent that one or more data components of the response data appears as relevant in determining and/or monitoring the level of consciousness / dissociation of a subject and should be included it in the evaluation protocol. New model's parameters should be modified accordingly as described above.

(c) Building population-based reference states/ thresholds

Population-based thresholds and/or reference state are computed based on measured data and/or observed data and/or self-reported and/or computed DoD(I), DoH(I), DoS(I) and/or independently measured DoD(I), DoH(I) and DoSI, using mathematical and/or statistical and/or machine learning processes. They should further be validated for convergence and stability.

The response data is transformed into the DoS(I) and/or DoH(I) using an evaluation protocol. The evaluation protocol may comprise use of one or more of a mathematical (e.g. statistical) model, trained machine-leaning model, mathematical index, reference data. The evaluation protocol takes as input the response data, and outputs the DoSI and/or DoHI.

The measured data may comprise one or more data components. A data component is attributed to a single measurement (e.g. EEG, EMG, EDA, or ECG), single observation (e.g. movement, skin colour), or single self-reported event. The evaluation protocol may weight the data components of the response data equally or differently. The weighting given to a data component depends, amongst other things, on relevance and precision of the component. In this way it is clear that as explained above with respect to the group 1 indicator, when P-Power(dt) is used in the evaluation protocol for determining DoS(I), DoD(I) and/or DoH(I), then it is always in combination with F-power(dt) and with a lower weight than F-power(dt).

The method and system may determine an expected LoAI, LoPI, DoSI, DoDI or DoHI and/or ratio, for example for a point in time before, during and/or after the treatment session, for example based on the particular phase, acts of treatment already performed, etc. before, during and/or after the treatment session. For instance, if the treatment comprises a hypnotic session or alternatively any other treatment modifying a level of consciousness of a subject, an expected LoAI, LoPI, DoSI, DoDI and/or DoHI may be determined from the phase, for example one of phases (i) to (iv), as will be described in further detail below, of the hypnotic session, and for example from the position within such a phase. For instance, if the treatment comprises a pharmacological sedation session, an expected LoAI, LoPI, DoSI, DoDI may be determined from the dose, titration, timing of the administration of the sedative, anxiolytic, analgesic, etc.. The expected LoAI, LoPI, DoSI, DoDI and/or DoHI may for example be determined from population data or subject data, namely from historical and/or precedence data.

Because an administered treatment does not always lead to the expected LoA, LoP, DoS, DoD, DoH, a difference between the measured LoA, LoP, DoS, DoD, DoH and the expected LoA, LoP, DoS, DoD, DoH provides the user with guidance for corrective measures with respect to the treatment.

It is clear that still further alternative embodiments are possible, in which there is provided a computer-implemented method for determining and/or monitoring a level of anxiety and/or a level of pain of a subject, which comprises the method for measurement of a level of modified consciousness and a level of anxiety and a level of pain, according to one or more of the embodiments as described above, applied on a subject by receiving the measured data of a subject.

According to a further aspect, similar as described above, there is thus also provided a method for determining and/or monitoring a level of pain and/or a level of anxiety in a subject before, during and/or after a treatment. According to such a method, there is measured a LoP and/or LoA at at least two different points in time, before, during and/or after the treatment. Based on a comparison of at least two LoP and/or LoA measured at at least two different points in time, there is then determined and/or monitored a treatment-induced evolution of the LoP and/or LoA respectively.

According to some treatments, such as for example pain treatments, in which for example a pharmacological and/or non-pharmacological treatment is used to reduce a particular level of pain the subject is experiencing for example by means of a sedative, it is clear that the goal of the treatment is to reduce the LoP, and that such a reduction in the LoP, for example during and/or after the treatment, corresponds to a desired treatment-induced evolution of the LoP. However according to certain other treatments, for example in a context of an anesthetic treatment, for example prior to a chirurgical treatment, it could be desired to retain the LoP as experienced in the normal awake state, in which the subject does not undergo the chirurgical treatment, also during the chirurgical treatment. In this way, it is clear that the desired treatment induced evolution of the LoP is the prevention of an increase of the LoP, or the prevention of an increase of the LoP beyond an undesirable limit or outside a predetermined desired range. It is clear that according to such embodiments, the treatment-induced evolution of the LoP can for example be based on a comparison of the LoP measured before and during the treatment, or measured before and after the treatment, etc.. It is thus clear that, according to an embodiment in which the treatment is a pain reducing treatment, then the efficacy of such a pain treatment, such as for example a pain-reducing, pain-controlling, pain-preventing, or any other suitable pain treatment, can be determining and/or monitored based on the treatment-induced evolution of the LoP. In other words, when the LoP is sufficiently reduced, for example to within a desired range, or when the LoP does not undesirably increase, for example to outside a desired range. Preferably, this treatment-induced evolution of the LoP allows to optimizing and/or adapting a pain treatment, such as for example a pain-reducing, pain-controlling, pain-preventing, or any other suitable pain treatment. Similarly, as for the LoP, also for anxiety management treatments, similar embodiments of a desired treatment-induced evolution of the LoA can for example be allow to optimize and/or adapt an anxiety management treatment, such as for example an anxiety-reducing, anxiety -controlling, anxiety -preventing, or any other suitable anxiety management treatment.

It is further also clear that based on the measured LoA, LoP and/or DoS, there could be determining a treatment and/or determining an adjustment to a treatment and/or determining an intensity of a treatment. For example, a higher value for the LoA and/or LoP could lead to a higher dosage of a sedative treatment, for example when such increases are noted during a surgical intervention on the subject. According to another example, if a particular anxiety and/or pain management treatment doesn't result in a decrease of the LoA and/or LoP, and/or doesn't prevent an increase of the LoA and/or LoP, then an alternative treatment could be initiated. The desired level of titration of a pharmacological pain and/or anxiety management treatment, or the desired intensity of a non-pharmacological treatment, such as for example the depth of hypnosis, the level of absorption, etc. could for example be increased or decreased based on a desired LoA, LoP or a treatment induced change thereto.

FIG. 36 shows a schematic illustration of Group indicators 1 and 2 related to DoS, and 3 to 5 related to LoP, and 6 to 11 related to LoA, as extracted from the measured data. On the right are shown the links to anxiety and the use for determination of LoA, the links to pain and the use for determination of LoP, and the links to the modified state of consciousness, such as for example a depth of dissociation, a level of absorption, etc. ; which, according to such an embodiment are all linked to a pharmacologically and/or non-pharmacologically induced modified state of consciousness (SoC). On the left are shown links between group 1 and 2 indicators, group indicators 3 to 5 indicators and Group indicators 6 to 11 and pharmacologically and/or non-pharmacologically induced modified state of consciousness (SoC). SoC, according to the particular embodiment shown, in the context of a non-pharmacological treatment, making use of hypnosis, is linked to Depth of Hypnosis. According to such an embodiment Depth of State is linked to Depth of Hypnosis, optionally with a measure of the modified state of consciousness induced pharmacologically, and optionally with a measure of the modified state of induced by other treatments. However it is clear that alternative embodiments are possible in which DoS is linked to alternative and/or additional parameters configured for indicating a modified state of consciousness, such as for example a level of absorption of a subject, etc.. It is clear that alternative embodiments are possible in which any suitable combination of embodiments of the LoA, LoP and/or DoS provides for a suitable derived index, for example by means of a calculation in which each of the parameters is provided with suitable weights, and which for example could provide for a suitable indicator or signal, for example a signal that it is safe to perform a medical intervention, a signal that it is necessary to modify the current treatment in order to guarantee the subjects safety, etc.

The method and system may provide an output to a graphical user interface (GUI). The system may comprise a GUI. As will be described in further detail below the system may comprise a graphical user interface, GUI, configured to indicate numerically and/or graphically one or more of:

- a historic, current and/or expected (N)LoA(I)(S), (N)LoP(I)(S), and/or DoS(I)(S);
- the evolution of and/or ratio between (N)LoA(I)(S), (N)LoP(I)(S), and/or DoS(I)(S) measured at two different points in time;
- optionally, one or more components of the measured data, preferably one or more EEG data and optionally ECG data, cardiovascular data, respiratory data, EMG data, muscular movement data, EOG data, ocular movement data, body temperature data, ....
- optionally an aggregated level of anxiety score or LoAS or LoAIS based on an aggregation of at least two LoA or LoAI measured at at least two different points in time.
- optionally an aggregated level of pain score or LoPS or LoPIS based on an aggregation of at least two LoP or LoPI measured at at least two different points in time.

It is clear that alternative embodiments of the GUI are possible in which further data is displayed.

The output to the GUI may additionally or alternatively show one or more current data components (e.g. one or more measured data components, one or more observed data components, and/or one or more self-reported data components). Examples of current data components include those listed elsewhere herein, preferably one or more of EEG data, cardiovascular data, respiratory data, muscular activity data, ocular movement data, body temperature data, ....

The output to the GUI may comprise a graphical indicator wherein a position and/or color on the screen provide the user (e.g. doctor) an indication of the state of the subject and if necessary steps to be taken. For example, a green color may indicate a positive status in terms of patient experience of signal for the user. For example, a red color may indicate a negative status in terms of patient experience of signal for the user. For example, a left position may indicate a positive status in terms of patient experience of signal for the user. For example, a right position may indicate a negative status in terms of patient experience of signal for the user. For example, an upper position may indicate a positive status in terms of patient experience of signal for the user. For example, a lower position may indicate a negative status in terms of patient experience of signal for the user.

The output to the GUI may additionally or alternatively show one or more derived indexes. A derived index is an index derived from one or more data components and/or from another index.. A derived index might be based on a ratio of LoAI, LoPI and/or DoSI. These derived indexes may be based on collected data as observed for the subject, optionally refined using analytics (i.e. historical/population data).

A graphical output may comprise a timeline and a marker on the timeline indicating one or more of the LoAI, NLoAI, LoPI, NLoPI, DoSI, DoDI, DoHI, ... and/or a ratio thereof. One or more of the LoAI, NLoAI, LoPI, NLoPI, DoSI, DoDI, DoHI, ... and/or a ratio thereof may be on scale with a first and second limit e.g. 0 to 1, 0 to 100.

The GUI may show one or more of the current LoAI, NLoAI, LoPI, NLoPI, DoSI, DoDI, DoHI on one or more scales between the first and second limits; the scale may be linear, logarithmic, or other. The scale may be continuous, categorical, discreet, percentage, ratio.

Examples of GUIs (200, a to g) are given in FIG. 38 to FIG. 45 and FIG. 52 to FIG. 54.

FIG. 38 shows a GUI (200, a) that is a graphical display of current status of DoSI, LoPI and LoAI as separate bar charts (252, 254, 255 respectively). Reference is made to the DoSI bar chart (252); the LoPI bar chart (254) and the LoAI (255) bar chart contain equivalent features (not labelled). The DoSI (y-axis) bar chart displayed is divided into 4 zones: 0-25% (224), 26-50% (222), 51-75% (220), 76-100% (218); these may optionally be colored to indicate a level of safety (e.g. 76-100% - red, 51-75% - orange, 26-50% - light green, 0-25% - deep green. The 100-76% (218, red) zone may indicate that the subject is conscious, alert, agitated if provoked: wait for hypnotic session to advance and optionally administer analgesic. The 75-50% (220, orange) zone may indicate that the subject is conscious and calm, but not dissociated, not yet ready. The 50-26% (222, light green) zone may indicate that the subject is in a light dissociated state, not yet ready. The 0-25% (224, deep green) zone may indicate that the subject is deep in the dissociated state/immersive state. It is appreciated that the units of percent and the extent of the scale of 0 to 100% is exemplary, and other units (e.g. unitless) and scales (e.g. 0 to 1, 0 to 40, 0 to 50, 0 to 60 etc., linear, logarithmic) are within the scope of the disclosure. The height of the bar (256) is indicative of the DoSI at the indicated time (261). A numerical display (258) also indicates the DoSI. Current units (percent) are indicated (260), that can be changed (e.g. to unitless and/or other units) with a units button (262). Forwards (264, a) and backwards (264, b) buttons allow the display to scroll back and forth between current DoSI and previous readings. The percentages on the LoPI bar and the LoAI bar, might scale similarly as for a pain scale and/or an anxiety scale respectively, as mentioned above, in which for example 0% corresponds to no pain or no anxiety and 100% corresponds to the most severe pain possible or the most severe anxiety possible. Reference is made to the LoPI bar chart (254) and the LoAI bar chart (255). The LoPI and the LoAI (y-axis) bar chart displayed is divided into 4 zones: 0-25% (224), 26-50% (222), 51-75% (220), 76-100% (218); these may optionally be colored to indicate a level of safety (e.g. 76-100% - red, 51-75% - orange, 26-50% - light green, 0-25% - deep green. The 100-76% (218, red) zone may indicate that in his perception the subject is experiencing very high, undesirable amounts of pain or anxiety respectively, which might for example indicate the need to optionally administer analgesic or anxiolytic respectively. The 75-50% (220, orange) zone may indicate that the subject is perceiving a level of pain or anxiety that is uncomfortable. The 50-26% (222, light green) zone may indicate that the subject is perceiving a light level of pain or anxiety. The 0-25% (224, deep green) zone may indicate that the subject is not perceiving any pain or anxiety or a level of pain or anxiety that is not perceived as uncomfortable. It is appreciated that the units of percent and the extent of the scale of 0 to 100% is exemplary, and other units (e.g. unitless) and scales (e.g. 0 to 1, 0 to 40, 0 to 50, 0 to 60 etc., linear, logarithmic) are within the scope of the disclosure. The height of the bar (256) is indicative of the LoPI or LoAI at the indicated time (261). A numerical display (258) also indicates the LoPI or LoAI. Current units (percent) are indicated (260), that can be changed (e.g. to unitless and/or other units) with a units button (262). Forwards (264, a) and backwards (264, b) buttons allow the display to scroll back and forth between current LoPI or LoAI and previous readings. The percentages on the LoPI bar or the LoAI bar, might scale similarly as for a pain scale as mentioned above, in which for example 0% corresponds to no pain and 100% corresponds to the most severe pain or anxiety possible.

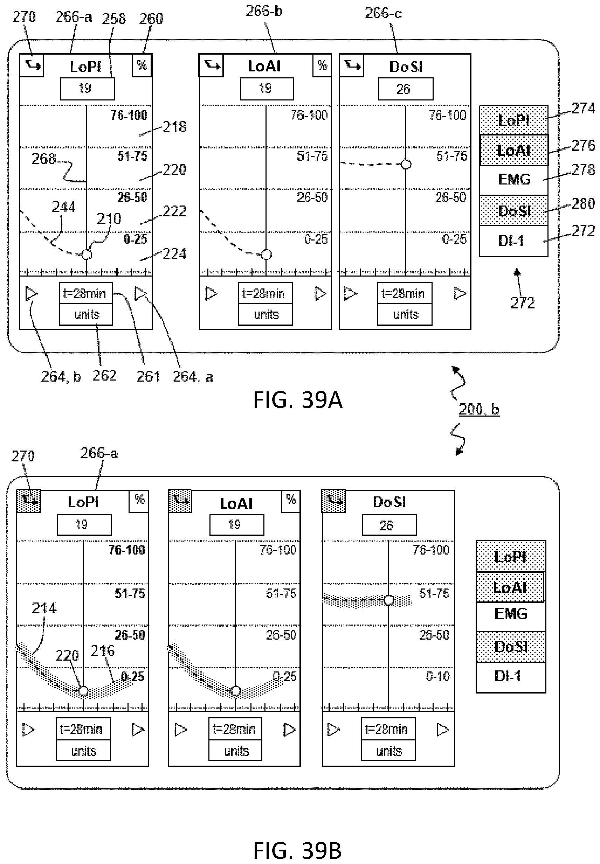

FIG. 39A and FIG. 38B shows a GUI (200, b) that is graphical display of current status of LoPI, LoAI and DoSI as separate scroll charts (266-a 266-b, 266-c respectively). Reference is made to the LoPI scroll chart (266-a) in FIG. 39A; the other scroll charts contain equivalent features (not labelled). The LoPI scroll chart displayed is divided into 4 zones: 0-25% (224), 26-50% (222), 51-75% (220), 76-100% (218); these may optionally be colored to indicate a level of safety (e.g. 76-100% - red, 51-75% - orange, 26-50% - light green, 0-25% - deep green as in FIG. 38. The LoPI scroll chart (266-a) contains a stationary time axis (268) along which the LoPI reading (210) at the indicated time (261) slides (up and down). A numerical display (258) also indicates the LoPI. The time axis (268) remains static, while the background scrolls in the direction of the time (typically from right to left). Historical or trending datapoints (244) are indicated. Current units (percent) are indicated (260) that can be changed (e.g. to unitless and/or other units) with a 'units' button (262). Forwards (264, a) and backwards (264, b) buttons allow the display to scroll back and forth between current LoPI and previous readings. A panel of buttons (272) allows the user to choose from a selection of scroll charts for display: buttons for LoPI (274), LoAI (276) and DoSI (280) are selected (grey background), and the selected scroll charts are displayed (266-a, 266-b, 266-c respectively). Unselected are buttons for EMG (278) and DI-1 (derived index-1, 272)).

In FIG. 39B, the same GUI as FIG. 39A are shown, and button (270) is unselected in FIG. 39A and is selected in FIG. 39B; button (270) toggles on or off a display of expected LoPI (216) for the subject that is superimposed on the scroll chart; current LoPI (210) and historical LoPI (214) can be visually compared with the expected LoPI (216).

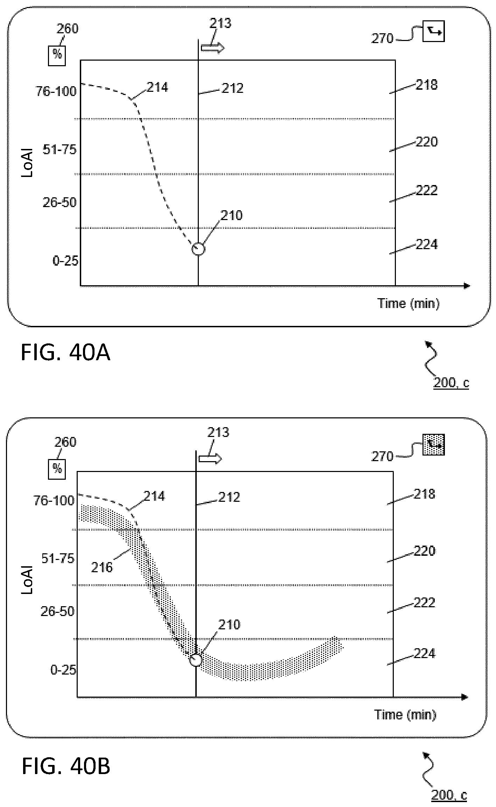

FIG. 39A and FIG. 40B shows a GUI (200, c) that is graphical display of a progress of a treatment session over time (x-axis). Reference is made to the LoAI graph in FIG. 40A; the graph in FIG. 40B contains equivalent features (not labelled). The current LoAI (210) of the subject at the current point of time shown as a circle (210) along a time axis (212) that advances in the direction of arrow (213), for example as the treatment session progresses. The LoAI graph y-axis displayed is divided into 4 zones: 0-25% (224), 26-50% (222), 51-75% (220), 76-100% (218); these may optionally be colored to indicate a level of safety (e.g. 76-100% - red, 51-75% - orange, 26-50% - light green, 0-25% - deep green as in FIG. 38. According to alternative embodiments, instead of, and/or in addition to LoAI, there could for example be represented LoPI in FIG. 40A and FIG.40B.

Historical or trending LoAI for the subject during the treatment session is shown as a dashed line (214). Current units (percent) are indicated (260); the box (260) may also serve as a button to cycle between different units (e.g. to unitless and/or other units). In FIG. 40B, the same graph as FIG. 40A is shown; button (270) is unselected in FIG. 40A and is selected in FIG. 40B. Button (270) toggles on or off a display of expected LoPI (216) for the subject that is superimposed on the graph; current LoPI (210) and historical LoPI (214) can be visually compared with the expected LoPI (216).

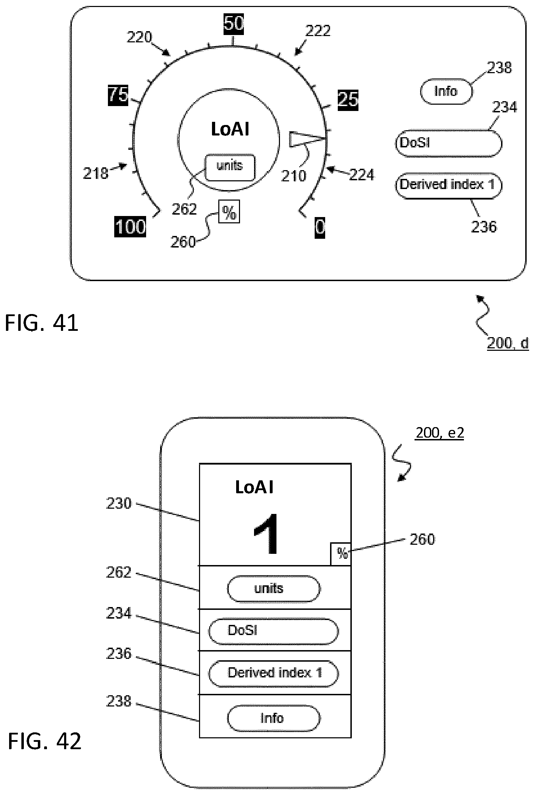

FIG. 41 shows a GUI (200, d) that contains a "speedometer" scale of the current LoAI (210) of the subject shown as a pointer. Current scale units (percent) are indicated (260), that can be changed (e.g. to unitless and/or other units) with a units button (262). The LoAI speedometer scale is divided into 4 zones: 0-25% (224), 26-50% (222), 51-75% (220), 76-100% (218); these may optionally be colored to indicate a level of safety (e.g. 76-100% - red, 51-75% - orange, 26-50% - light green, 0-25% - deep green as in FIG. 38. Buttons (234, 236, 238) are displayed from which the user can select one or more options to change the display of the GUI and for further information.

FIG. 42 shows a GUI (200, d2) that contains a numerical display of the current LoAI (230) of the subject. Current units (percent) are indicated (260), that can be changed (e.g. to unitless and/or other units) with a units button (262). Buttons (234, 236, 238) are displayed from which the user can select one or more options to change the display of the GUI and for further information.

FIG. 43 shows a GUI (200, e) wherein different indexes are represented. External point of each axis represents the maximal scale value for this index. Chart gives an overview of the values of different indexes. FIG. 44 shows a GUI (200, f) displaying current LoAI for the subject (240), for example during a therapeutic session, an anesthetics, analgesic treatment, etc. over time. Historical or trending datapoints (244) are also shown, and an average of the historical or trending data points is shown as a dashed line (242).

FIG. 45 shows a GUI (200, g) displaying an average measure per index (LoPI, DoSI, LoAI) compared to the rest of the population (analytics), for example at the same point in time of the treatment session.

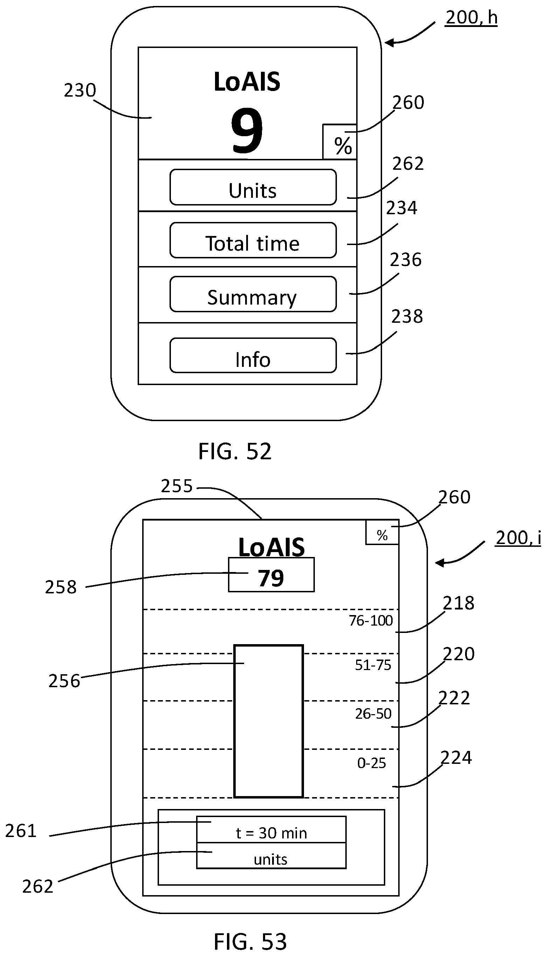

FIG. 52 shows a GUI (200, h), similar to that one of FIG. 42 and similar references have been used to indicate similar features, but now an LoAIS that contains a numerical display of the aggregated LoAIS (230) of the subject is shown instead of the current LoAI. Current units (percent) are indicated (260), that can be changed (e.g. to unitless and/or other units) with a units button (262). Buttons (234, 236, 238) are displayed from which the user can select one or more options to change the display of the GUI and for further information. Button 234 labeled "Total time" could for example be used to output and/or input an indication of the time during which LoAI has been aggregated. The button labeled "Summary" could for example be use to output a graphical diagram, such as a curve of the LoA(I) and the LoA(I)S during the period of time it has been aggregated, such as for example shown in FIG. 54.

FIG. 53 shows an embodiment of a GUI (200, i) similar to the embodiment of the GUI (200, a) of FIG. 38 and similar elements are indicated with similar references. However instead of a graphical display of current status of DoSI, LoPI and LoAI as bar charts (252, 254, 255 respectively) in FIG. 38, now this embodiment shows a graphical display of the aggregated LoAIS barchart 255. It is clear that alternative embodiments are possible, similar to FIG. 52 and FIG. 53, wherein one or more of LoA(I)S, LoP(I)S and/or DoS(I)S is show in a numerical and/or graphical way, alone and/or in combination. Similar as explained above The LoAIS (y-axis) bar chart displayed is divided into 4 zones: 0-25% (224), 26-50% (222), 51-75% (220), 76-100% (218); these may optionally be colored to indicate a level of safety (e.g. 76-100% - red, 51-75% - orange, 26-50% - light green, 0-25% - deep green. The height of the bar (256) is indicative of the aggregated LoAIS, which as aggregated during an indicated time period (261). According to the embodiment shown, this means that the LoAI was aggregated during a time period of 30 minutes for calculating the displayed LoAIS, however it is clear that alternative time periods are possible. A numerical display (258) also indicates the LoAIS. Current units (percent) are indicated (260), that can be changed (e.g. to unitless and/or other units) with a units button (262).

FIG. 54 shows an embodiment of a GUI (200, j), similar to that of FIG. 44, which for example also shows the measured LoAI for the subject (240) for example during the time period over which the LoAI is aggregated to calculate the LoAIS or any other suitable time period, such as for example during a therapeutic session, an anesthetics, analgesic treatment, etc.. As already mentioned according to some embodiments the dashed line represents the evolution of LoAI over time, for example by means an average of the historical or trending data points (242), or by means of any other suitable embodiment, for example making use of any historical, current or trending datapoints of the LoAI. The full line shows the evolution over time of the aggregated LoAIS 246, which is an aggregation over a predetermined time period of the LoAI. According to the embodiment shown, it is for example clear that LoA(I)S increases more rapidly when LoA(I) is higher, and the LoA(I)S increases more slowly as the LoA(I) is lower. It is clear that similar alternative embodiments of such a GUI are possible for displaying LoP(I) and LoP(I)S, or DoS(I) and DoS(I)S, alone and/or in combination.

FIG. 57 shows a further embodiment of a GUI (200, k), similar to that of FIG 52 and similar to that one of FIG. 42 and similar references have been used to indicate similar features, but now a derived index referred to as a "Risk index" is shown instead of a LoAI(S). As shown the "Risk Index" according to this embodiment contains a display, which by means of suitable labels and/or categories indicates a level of Risk for example, for engaging in and/or continuing a treatment based on the measured LoA, LoP and/or DoS and/or any correlations thereof. Current units (categories) are indicated (260), that can be changed (e.g. to percent to show a numerical value, unitless and/or other units) with a units button (262). Buttons (234, 236, 238) are displayed from which the user can select one or more options to change the display of the GUI and for further information. Button 234 labeled "LoPI" could for example be used to output a numerical or graphical indication of the LoPI. The button 236 labeled "LoAI" could for example be use to output a numerical or graphical indication of the LoAI. The button 238 labeled "LoPI" could for example be use to output a numerical or graphical indication of the LoPI. In this way the buttons allow access to a more detailed analysis of the constituent components of the derived indicator in the form of the Risk Index. It is however clear that alternative derived indicators are possible such as for example any suitable index, based on LoA(I)(S), LoP(I)(S), DoS(I)(S) and/or any suitable correlations thereof. According to some embodiments, such a derived index such as a risk index, comfort index, etc. could provide for a single and simple visualization of the patient's overall comfort and/or safety. Such a derived index is advantageous as it provides, for example to the anesthesiologist or any other suitable operator a single, simple indicator, that combines and/or correlates LoA, LoP and/or DoS, for measuring a more general state of the subject. Such an indicator and/or aggregated indicator relating to a general risk score, a general comfort score, etc. would allow an anesthesiologist for example to have an idea of the overall state of the patient prior to and/or during a treatment. Such a simple general index might suffice in most situations and/or when urgent assessments need to be made. Preferably, access to the individual components and/or the corresponding desired ranges for components such as LoP, LoA and/or DoS, for example in the form of limits and/or thresholds, is also provided to the health care professional, such as for example by means of the buttons as explained above, and/or by means of a concurrent display of these components.

As already mentioned above, it is clear that alternative embodiments are possible for such derived indexes, which optionally could be aggregated indexes. Such derived and/or aggregated indexes may be computed based on one or more of LoA, LoP and DoS, and/or any correlations, aggregation, indexing, range detection, limit detection, etc. thereof, for example by means of a suitable evaluation protocol. According to embodiments the evaluation protocol may dynamically weight LoA, LoP and DoS with different weights depending on for example the relevant population, the treatment, the type of intervention, the individual current values of LoA, LoP and DoS and/or current evolution of LoA, LoP and DoS, etc.. According to a particular example, if LoA suddenly increases by an amount that exceeds a certain limit or threshold, for example due to an anxiety triggering event, the evaluation function might give a higher weight to the LoA for the current computation of the derived and/or aggregated index.

According to such embodiments the derived and/or aggregated index at time t based on LoA, LoP and DoS might be calculated by an evaluation function based on the following formula:

$$\text{derived index}_t = S * f(f_{1,t}(LoA_t),\ f_{2,t}(LoP_t),\ f_{3,t}(DoS_t)) + K$$

Where S and K are scale up parameters and $f, f_{1,t}, f_{2,t}, f_{3,t}$ are any suitable functions, such as for example functions such as weighting, aggregating, limit monitoring, range monitoring, ..., which for example vary depending on the population, the treatment, the intervention, the individual current values of LoA, LoP and DoS, and/or any correlations thereof, and/or current evolution of LoA, LoP and DoS, etc..

According to the embodiment shown in FIG. 57, where the derived index is for example a risk Index, configured to show how safe it is to engage and/or continue a treatment. According to such an embodiment, the evaluation function could assess whether LoA, LoP and DoS, are all within pre-defined desired ranges, in which it is safe to engage and/or continue the treatment. The evaluation function could for example determine whether the LoA<2O%, the LoP<20% and the DoS>80%. Based on these evaluations the evaluation function could for example output, or determine three risk levels, or in other words three categories for the risk index, the risk level is scaled according to these three categories, for example Low = green; Medium = orange; High = red. It is clear that alternative embodiments are possible in which the Risk Index is presented graphically and/or numerically, similarly as explained above with respect to the different embodiments of the GUI. According to the embodiment of FIG. 57, the evaluation function could then be configured to calculate the category of the risk index based on whether LoA, LoP and/or DoS are within or outside their associated desired ranges. For example, when LoA, LoP and are all in their desired range, the evaluation function determines that the risk index = Low, when one of LoA, LoP and/or DoS is not in the desired range the evaluation function determines that the risk index = Medium, and when 2 or all of the LoA, LoP and/or Dos are not in the desired range the evaluation function determines that the risk index = High. It is however clear that numerous alternative embodiments of such a derived and/or aggregated index and/or corresponding evaluation functions are possible. For example, according to such an embodiment, instead of a risk index, there could be provided a comfort index, safety index, ... , or any other suitable derived index indicating the level of patient comfort and/or safety, which for example has values and/or categories that are opposite to those of the risk index, such as for example three categories for the comfort index, safety index, .. , in which comfort and/or safety level is scaled according to these three categories, for example High = green; Medium = orange; Low = red. It is clear that alternative embodiments are possible in which the Comfort Index, Safety Index, or any other suitable index is presented graphically and/or numerically, similarly as explained above with respect to the different embodiments of the GUI.

A system may be provided for measuring and/or monitoring a level of anxiety combined with and/or correlated with a level of pain and/or a modified state of consciousness of a subject, the system comprising:

- optionally, a media renderer configured for presenting the treatment session to the subject;
- a monitoring apparatus configured to obtained measured data of the subject;
- a controller module configured for receiving measured data from the monitoring apparatus, and transforming the response data comprising the measured data into one or more of (N)LoA(I)(S), (N)LoP(I)(S), DoS(I)(S), DoDI, DoHI. The evaluation protocol may comprise use of one or more of a mathematical (e.g. statistical) model, trained machine-learning model, mathematical index, reference data.

It is thus clear that, according to an embodiment, there is provided a system configured to measure a level of anxiety combined with and/or correlated with a level of pain and/or a modified state of consciousness according to the method for measuring the LoA, LoP and/or DoS as described above. Such a system comprises a monitoring apparatus configured to obtain measured data comprising electroencephalogram, EEG, data collected from one or more suitable electrodes. The EEG data comprises data, collected from at least one of: at least one parietal (P) electrode configured for collection of P-EEG data from the scalp anatomical region corresponding to a parietal lobe of a subject; and at least one frontal (F) electrode configured for collection of F-EEG data from the scalp anatomical region corresponding to a frontal lobe of a subject. It further comprises a controller module configured for receiving the measured data from the monitoring apparatus. The controller module is configured for extracting from the EEG data collected from the P-EEG data, a group 8 indicator based on a power, P-power (dt), associated with a delta-theta frequency band, dt, within a delta-theta frequency range. Optionally, the controller module is further configured for extracting from the EEG data, a group 1 indicator based on, a power, F-power(dt), associated with a delta-theta frequency band, dt, within a delta-theta frequency range extracted from the at least one F- EEG electrode data; and/or optionally for extracting from the EEG data, a group 4 indicator corresponding to a power, power (ta), associated with a theta-aipha frequency band, ta, within a theta-alpha frequency range. The controller module is configured for determining, based on said group 8 indicator, a level of anxiety, LoA, which is a value indicative for the level of anxiety in the subject. The controller module is further configured for determining, based on said group 1 indicator, a depth of state, DoS, which is a value indicative for the level of a modified state of consciousness; and/or determining, based on said group 4 indicator, a level of pain, LoP, which is a value indicative for the level of pain in the subject. Optionally, it is clear that according to similar embodiments as explained above, the LoP could be further determined by means of group 3 and/or group 5 indicators. Optionally, it is clear that according to similar embodiments as described above the LoA could be further determined by means of a group 6, 7, 9, 10, and/or 11 group indicator in combination with the group 8 indicator.

Preferably the monitoring apparatus comprises, for obtaining measured data, one or more parietal (P) electrodes configured for collection of P-EEG data from the scalp anatomical region corresponding to a parietal lobe of the subject, and one or more frontal (F) electrodes configured for collection of F-EEG data from the scalp anatomical region corresponding to a frontal lobe of the subject. Optionally, the monitoring apparatus comprises one or more central (C) electrodes configured for collection of C-EEG data from the scalp anatomical region corresponding to a precentral and postcentral gyrus of the subject, and optionally, one or more further electrodes configured for collection of electroencephalogram, EEG, data. Optionally the monitoring apparatus comprises one or more of the following: a respiratory data capturing unit; a cardiovascular data capturing unit; an ocular movement data capturing unit; a muscular activity data capturing unit; optionally a body temperature data capturing unit. It is clear, as will be explained in further detail below, that still further embodiments of the system are possible.

According to an embodiment the monitoring apparatus may be configured to capture response data of the subject, in particular measured data of the subject. The monitor apparatus comprises one or more units, each unit containing, depending on the type of unit one or more electrodes, sensors, cameras that capture measured data. The measured data component of each unit may or may not result from a processing of a signal captured by the one or more electrodes, sensors, cameras.

The monitoring apparatus may comprise an electroencephalogram (EEG) capturing unit. The response data i.e. measured data comprises outputted EEG data. EEG capturing unit may comprise at least two (e.g. 2, 3, 4, 5 or more), preferably a plurality of electrodes configured for acquiring electrical activity data from the subject brain. The electrodes may be configured for placement at predetermined positions across the subject's cephalic region; for instance to the frontal, parietal, and/or occipital region. An exemplary electrode configuration is 2 frontal electrodes, 2 lateral electrodes, 1 occipital electrode with respect to the head. However, it is clear that alternative embodiments are possible comprising other electrodes at other scalp locations.

In a preferred configuration, the EEG capturing unit may comprise at least three electrodes:

- the frontal EEG electrode (F-EEG electrode) configured for attachment to or contact with the scalp anatomical region corresponding to the frontal lobe;
- the parietal EEG electrode (P-EEG electrode) configured for attachment to or contact the scalp anatomical region corresponding to the parietal lobe;
- the central EEG electrode (C-EEG electrode) configured for attachment to or contact the scalp anatomical region corresponding to the precentral and postcentral gyrus.

The EEG capturing unit may further comprise a ground or reference electrode. This is situated away from the F-, P- or C-EEG electrode to allow spatial reconstruction. However according to alternative embodiments, the EEG capturing unit cold for example comprises only electrodes for capturing data at a single or two scalp locations.

The electrodes may be integrated into a wearable device e.g. wearable headset. The electrodes may be fixed to or detachable from the wearable device. The electrodes may be dry-contact electrodes. The electrodes may be held in place be gravity, or by a force (e.g. applied by a spring or elastic) or by adhesive. The electrodes may be reusable or one-time use. The electrodes may be integrated into a head-strap or fixing band. The electrodes may be integrated into a mask portion of a media renderer, as explained in further detail below. The electroencephalogram (EEG) capturing unit typically includes an amplifier for amplification of the detected signal. Signals are digitized for processing by a digital-to-analogue converter for processing by the processing unit. The sampling rate is typically at least 2.5 times higher than the highest frequency of interest. The digital-to-analogue converter may be separate or built into the processing unit.

According to an embodiment the monitoring apparatus may comprise a muscular movement data capturing unit, such as for example an electromyography (EMG) capturing unit. The response data i.e. measured data comprises outputted EMG data. EMG capturing unit may comprise at least one (e.g. 1, 2, 3, 4, 5 or more), preferably a plurality of electrodes configured for acquiring electrical activity data from the subject muscle tissue. Where an EEG capturing unit is present, the EMG capturing unit may share at least one electrode with the EEG capturing unit. Similarly, where an EEG capturing unit is present, an ECG capturing unit may share at least one electrode with the EEG capturing unit. The electrodes may be configured for placement at predetermined positions across the subject's cranial and/or facial region; preferably to the frontal region. The electrodes may be integrated into a wearable device e.g. wearable headset. The electrodes may be fixed to or detachable from the wearable device. The electrodes may be dry-contact electrodes. The electrodes may be held in place be gravity, or by a force (e.g. applied by a spring or elastic) or by adhesive. The electrodes may be reusable or one-time use. The electrodes may be integrated into a head-strap or fixing band. The electrodes may be integrated into a mask portion of the media renderer. The EMG capturing unit typically includes an amplifier for amplification of the detected signal. Signals are digitized for processing by a digital-to-analogue converter for processing by the processing unit. The digital-to-analogue converter may be separate or built into the processing unit.

According to an embodiment the monitoring apparatus may comprise a respiratory data (RD) capturing unit. The response data i.e. measured data comprises outputted respiratory data. RD capturing unit may comprise at least one (e.g. 1, 2, 3, 4, 5 or more) sensor(s) configured for acquiring RD from the subject. The respiratory data may comprise one or more of respiration rate, respiration rate variability, inhalation pressure. The RD capturing unit may comprise one or more photoplethysmogram (PPG) sensors. A PPG sensor detects blood volume changes in vasculature below the skin; the sensor is typically optical. The respiration rate and respiration rate variability may be determined from the PPG sensor. A PPG sensor may be placed at temple area and/or forehead area. The respiration rate and respiration rate variability may be determined from the PPG sensor. One or more of the sensor(s) may be configured for placement at predetermined positions across the subject's cranial and/or facial region. The sensor(s) may be integrated into a wearable device e.g. wearable headset. The sensor(s) may be fixed to or detachable from the wearable device. According to an embodiment, the sensor(s) may be dry-contact sensor(s) and/or electrode(s). The sensor(s) may be held in place be gravity, or by a force (e.g. applied by a spring or elastic) or by adhesive. The sensor(s) may be reusable or one-time use. The sensor(s) may be integrated into a head-strap or fixing band. The sensor(s) may be integrated into a mask portion of the media renderer e.g. so as to place PPG sensors at the forehead and/or temple region. The RD capturing unit may comprise a wearable chest band containing a force transducer that measures the expansion and contraction of the chest; the respiration rate and respiration rate variability may be determined from the wearable chest band sensor. The RD capturing unit may comprise an airways air pressure detector that measures the air pressure (e.g. during inhalation or exhalation). The RD capturing unit typically includes an amplifier for amplification of the detected signal. Signals are digitized for processing by a digital-to-analogue converter for processing by the processing unit. The digital-to-analogue converter may be separate or built into the processing unit.

According to an embodiment, the monitoring apparatus may comprise a cardiovascular data capturing unit, which according to an embodiment for example comprises a heart rate (HR) capturing unit. The response data i.e. measured data comprises outputted HR data. HR capturing unit may comprise at least one (e.g. 1, 2, 3, 4, 5 or more) sensor(s) and/or electrode(s) configured for acquiring heart data from the subject. The HR capturing unit may comprise one or more photoplethysmogram (PPG) sensors that detects blood volume changes vasculature below the skin; the sensor is typically optical. A PPG sensor may be placed at temple area and/or forehead area. The peaks of a captured PPG wave can be used to estimate the heart rate, heart rate variability as well as heartbeat interval, and blood pressure (two PPG sensors). The HR capturing unit may comprise one or more ECG electrodes, or any other suitable electrodes configured for acquiring electrical activity data from the subject heart. Where an EEG or EMG capturing unit is present, the HR capturing unit may share an electrode with the EEG or EMG capturing unit. One or more of the sensor(s) and/or electrode(s) may be configured for placement at predetermined positions across the subject's cranial and/or facial region; they may be placed bilaterally. Where the sensors are PPG sensors, may be configured for placement at the forehead and/or temple region. The sensor(s) and/or electrode(s) may be integrated into a wearable device e.g. wearable headset. The sensor(s) and/or electrode(s) may be fixed to or detachable from the wearable device. The sensor(s) and/or electrode(s) may be dry-contact sensor(s) and/or electrode(s). The sensor(s) and/or electrode(s) may be held in place be gravity, or by a force (e.g. applied by a spring or elastic) or by adhesive. The sensor(s) and/or electrode(s) may be reusable or one-time use. The sensor(s) and/or electrode(s) may be integrated into a head-strap or fixing band. The sensor(s) and/or electrode(s) may be integrated into a mask portion of the media renderer e.g. so as to place PPG sensors at the forehead and/or temple region. The HR capturing unit typically includes an amplifier for amplification of the detected signal. Signals are digitized for processing by a digital-to-analogue converter for processing by the processing unit. The digital-to-analogue converter may be separate or built into the processing unit.

According to an embodiment the monitoring apparatus may comprise an ocular movement data capturing unit, which according to an embodiment comprises an electrooculography (EOG) capturing unit. The response data i.e. measured data comprises outputted EOG data. EOG capturing unit may comprise at least one (e.g. 1, 2, 3, 4, 5 or more), preferably a plurality of electrodes configured for acquiring electrical activity data from around the subject eye. Where an EEG or EMG capturing unit is present, the EOG capturing unit may share an electrode with the EEG or EMG capturing unit. The electrode(s) may be configured for placement at predetermined positions across the subject's cranial and/or facial region; preferably to the around (e.g. above and below, to left and right of an eye). The electrode(s) may be integrated into a wearable device e.g. wearable headset. The electrode(s) may be fixed to or detachable from the wearable device. The electrode(s) may be dry-contact electrode(s). The electrode(s) may be held in place be gravity, or by a force (e.g. applied by a spring or elastic) or by adhesive. The electrode(s) may be reusable or one-time use. The electrode(s) may be integrated into a head-strap or fixing band. The electrode(s) may be integrated into a mask portion of the media renderer. The EOG capturing unit typically includes an amplifier for amplification of the detected signal. Signals are digitized for processing by a digital-to-analogue converter for processing by the processing unit. The digital-to-analogue converter may be separate or built into the processing unit.

According to an embodiment the monitoring apparatus may comprise an electrodermal activity (EDA) capturing unit. The response data i.e. measured data comprises outputted EDA data. EDA capturing unit may comprise at least one (e.g. 1, 2, 3, 4, 5 or more), preferably a plurality of electrodes configured for acquiring electrical activity data from the subject skin tissue. Where an EEG or EMG capturing unit is present, the EDA capturing unit may share an electrode with the EEG or EMG capturing unit. The electrode(s) may be configured for placement at predetermined positions across the subject's cranial and/or facial region; preferably to the frontal region.

The electrode(s) may be integrated into a wearable device e.g. wearable headset. The electrode(s) may be fixed to or detachable from the wearable device. The electrode(s) may be held in place be gravity, or by a force (e.g. applied by a spring or elastic) or by adhesive. The electrode(s) may be reusable or one-time use. The electrode(s) may be integrated into a head-strap or fixing band. The electrode(s) may be integrated into a mask portion of the media renderer. The EDA capturing unit typically includes an amplifier for amplification of the detected signal. Signals are digitized for processing by a digital-to-analogue converter for processing by the processing unit. The digital-to-analogue converter may be separate or built into the processing unit.

According to an embodiment, the EDA capturing unit may be configured for measurement of galvanic skin response. The response data i.e. measured data comprises outputted GSR data. EDA capturing unit may comprise at least one (e.g. 1, 2, 3, 4, 5 or more), preferably a plurality of GSR electrodes configured for acquiring galvanic skin response. Where an EEG or EMG capturing unit is present, the EDA capturing unit may share a localized or reference electrode with the EEG or EMG capturing unit for acquiring galvanic skin response. The electrode(s) may be configured for placement at predetermined positions across the subject's cranial and/or facial region; preferably to the frontal or forehead region. The GSR electrode(s) may be integrated into a wearable device e.g. wearable headset. The GSR electrodes may be fixed to or detachable from the wearable device. The GSR electrodes may be dry-contact electrode(s). The GSR electrode(s) may be held in place be gravity, or by a force (e.g. applied by a spring or elastic) or by adhesive. The GSR electrode(s) may be reusable or one-time use. The GSR electrode(s) may be integrated into a head-strap or fixing band. The GSR electrode(s) may be integrated into a mask portion of the media renderer.

According to an embodiment, the monitoring apparatus may comprise an electrocardiogram (ECG) capturing unit. The response data i.e. measured data comprises outputted heart rate or ECG data. ECG capturing unit may comprise at least one (e.g. 1, 2, 3, 4, 5 or more), preferably a plurality of electrodes configured for acquiring electrical activity data from the subject heart. Where an EEG, EMG, or EDA capturing unit is present, the ECG capturing unit may share an electrode with the EEG, EMG, or EDA capturing unit. The electrodes being configured for placement at predetermined positions across the subject's chest, or any other suitable location where the heart ECG data can be captured. The electrodes may be integrated into a wearable device e.g. wearable headset. The electrodes may be fixed to or detachable from the wearable device. The electrodes may be held in place by gravity, or by a force (e.g. applied by a spring or elastic) or by adhesive. The electrodes may be reusable or one-time use. An electrode may be integrated into a head-strap or fixing band. An electrode may be integrated into a portion of the media renderer.

According to an embodiment, the monitoring apparatus may further comprise a physiological monitoring unit. The response data i.e. measured data comprises outputted physiological data. The physiological monitoring unit may comprise at least one (e.g. 1, 2, 3, 4 or more) sensor for acquiring subject's physiological data, in particular a component of the physiological data. The physiological data may relate to one or more of the following: pulse rate, heart rate, heart rate variation, blood pressure, respiration rate, respiration rate variability, inhalation pressure, exhalation pressure, brain oxygenation, blood O2 saturation (SpO2), regional and/or central blood O2 saturation, skin conductance, galvanic skin response, body temperature. The physiological data may relate to one or more of the following: respiration rate, respiration rate variability, inhalation pressure, heart rate.

According to an embodiment, the monitoring apparatus may comprise a SpO2 capturing unit. The response data i.e. measured data comprises outputted SpO2 data. SpO2 capturing unit capturing unit may comprise at least one (e.g. 1, 2, 3, 4, 5 or more) sensor(s) configured for capturing SpO2 (blood oxygen saturation) data from the subject. The SpO2 data capturing unit may comprise one or more sensors e.g. photoplethysmogram (PPG) sensor; the sensor is typically optical. A sensor may be placed at temple area and/or forehead area. The sensor may be configured for placement at the forehead and/or temple region. The sensor(s) may be integrated into a wearable device e.g. wearable headset. The sensor(s) may be fixed to or detachable from the wearable device. The sensor(s) may be dry-contact sensor(s). The sensor(s) may be held in place be gravity, or by a force (e.g. applied by a spring or elastic) or by adhesive. The sensor(s) may be reusable or one-time use. The sensor(s) may be integrated into a head-strap or fixing band. The sensor(s) may be integrated into a mask portion of the media renderer e.g. so as to place sensor(s) at the forehead and/or temple region. The SpO2 capturing unit capturing unit typically includes an amplifier for amplification of the detected signal. Signals are digitized for processing by a digital-to-analogue converter for processing by the processing unit. The digital-to-analogue converter may be separate or built into the processing unit.

According to an embodiment the one or more sensors may be integrated into a wearable device e.g. wearable headset. The one or more sensors may be fixed to or detachable from the wearable device. The one or more sensors may be held in place be gravity, or by a force (e.g. applied by a spring or elastic) or by adhesive. The one or more sensors may be reusable or one-time use. The one or more sensors may be integrated into a head-strap. The sensors may be integrated into a mask portion of a media renderer as explained in further detail below.

According to an embodiment, the monitoring apparatus may further comprise a body motion tracking unit. The response data i.e. measured data comprises outputted body motion tracking data. The body motion tracking unit may comprise at least one (e.g. 1, 2, 3, 4 or more) motion sensor for acquiring subject's body motion, in particular a component of the body motion tracking data. Examples of motion sensors include a 2- or 3-axis accelerometer, a gyroscope, one or more cameras, magnetic/induction transducers. The body motion includes movements of the head, limb (arms, legs, hands, knee, elbow). The body motion tracking unit may be a head movement tracking unit.

According to an embodiment the one or more motion sensors may be integrated into a wearable device e.g. wearable headset. The one or more motion sensors may be fixed to or detachable from the wearable device. The one or more motion sensors may be held in place be gravity, or by a force (e.g. applied by a spring or elastic) or by adhesive. The one or more motion sensors may be reusable or one-time use. The one or more motion sensors may be integrated into a head-strap. The motion sensors may be integrated into a mask portion of the media renderer.

According to an embodiment the monitoring apparatus may further comprise an eye-tracking unit. The response data i.e. measured data comprises outputted eye tracking data. The eye tracking unit may comprise at least one (e.g. 1, 2, 3, 4 or more) camera for monitoring movement of one or both eyes of the subject. The eye tracking unit may comprise at least one light source (e.g. visible, infrared) configured to illuminate the eye. Captures images may be analyzed using eye tracking software to determine subject's focus of attention, drowsiness, consciousness or other mental states.

According to an embodiment the one or more cameras may be integrated into a wearable device e.g. wearable headset. The one or more cameras may be fixed to or detachable from the wearable device. The one or more cameras may be integrated into a mask portion of the media renderer.

According to an embodiment the monitoring apparatus may further comprise a facial expression capturing unit. The response data i.e. measured data comprises outputted facial expression data - e.g. emotions, nociception. The facial expression capturing unit may comprise at least one (e.g. 1, 2, 3, 4 or more) camera for monitoring facial expressions of the subject. The facial expression capturing unit may comprise at least one light source (e.g. visible, infrared) configured to illuminate the face. Captures images may be analyzed using facial expression recognition software to determine subject's facial expressions and responses, e.g. pain or (dis)comfort. The one or more cameras may be integrated into a wearable device e.g. wearable headset. The one or more cameras may be fixed to or detachable from the wearable device. The one or more cameras may be integrated into a portion of the media renderer.

According to an embodiment the monitoring apparatus may comprise an EEG capturing unit, an EMG capturing unit, an EDA capturing unit, an ECG capturing unit, a physiological monitoring unit, a head tracking unit, an eye tracking unit and a face capturing unit. The monitoring apparatus may comprise an EEG capturing unit, an EMG capturing unit, a HR capturing unit, and/or a respiratory data capturing unit.

According to a particular embodiment, in which the system is used during a non-pharmacological treatment session, the system may further comprise a media renderer that presents a treatment session to the subject. The treatment session may contain hypnosis and/or other evidence-based psychological and/or mind/body intervention. The media render may comprise a screen (e.g. LED, LCD, projector) on which moving images are displayed. The images immerse the attention of the subject and/or control the subject's experience and physiological response. The media render may comprise a sound transducer (e.g. earphone, headphone, speaker) to which audio is passed (e.g. music, dialogue, sound effects).

Preferably the media renderer is integrated as a wearable device, e.g. headset. The media rendered may be provided as a virtual reality headset, as an augmented reality headset, or mixed reality headset. Most preferably the media renderer is integrated into a wearable device that provides virtual/ augmented/ mixed/ etc. reality; which typically includes a display or projector, stereo sound (mono, stereo, multidimensional), and head motion tracking sensors (e.g. gyroscopes, accelerometers, structured light systems, etc.). Examples of suitable headsets are those supplied by Oculus (e.g. Oculus Rift, Oculus Go), Pico (e.g. G2 4K, G2 Pro), LG electronics (e.g. LG 360 VR), HTC (e.g. HTC Vive), Samsung (e.g. Samsung Gear VR), Google (e.g. Google Cardboard), Microsoft (Hololens), and other off-the-shelf or customized designs. The media renderer may be expanded by also rendering somatosensory content, such as vibrations (e.g.

vibration modules equipped in a seat), and/or olfactory content (e.g. releasing one or more fragrances into the nasal cavity). Optionally, the media renderer may be equipped with a computing unit for executing or playing data, or it may receive data from an external media server (i.e. streaming).

As mentioned previously, the media renderer may be integrated into a wearable device, e.g. headset. The monitoring apparatus may be integrated into the wearable device. One or more electrodes and/or one or more sensors, and/or one or more cameras of the monitoring apparatus may be integrated into the wearable device

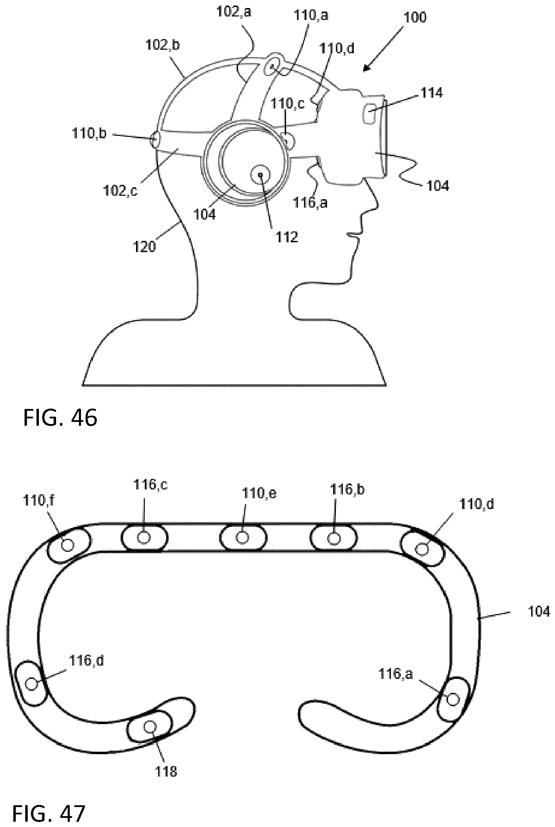

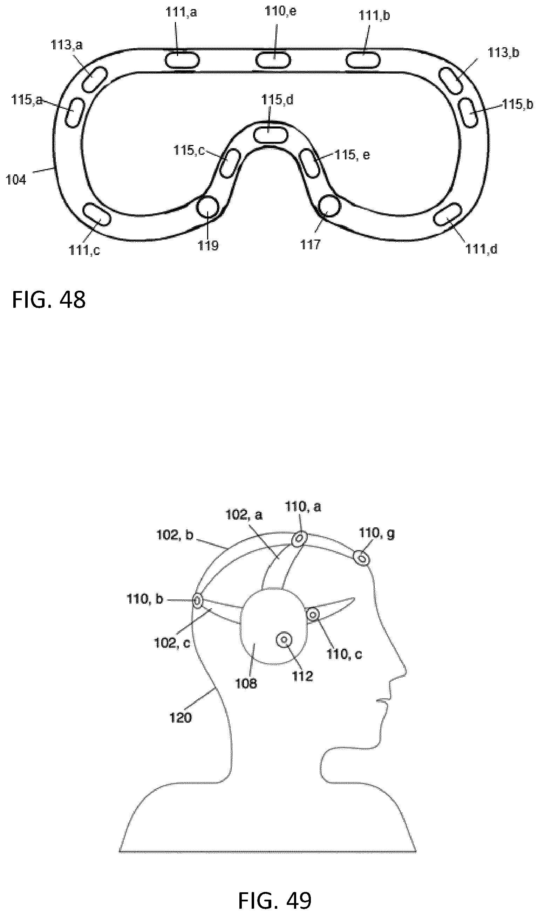

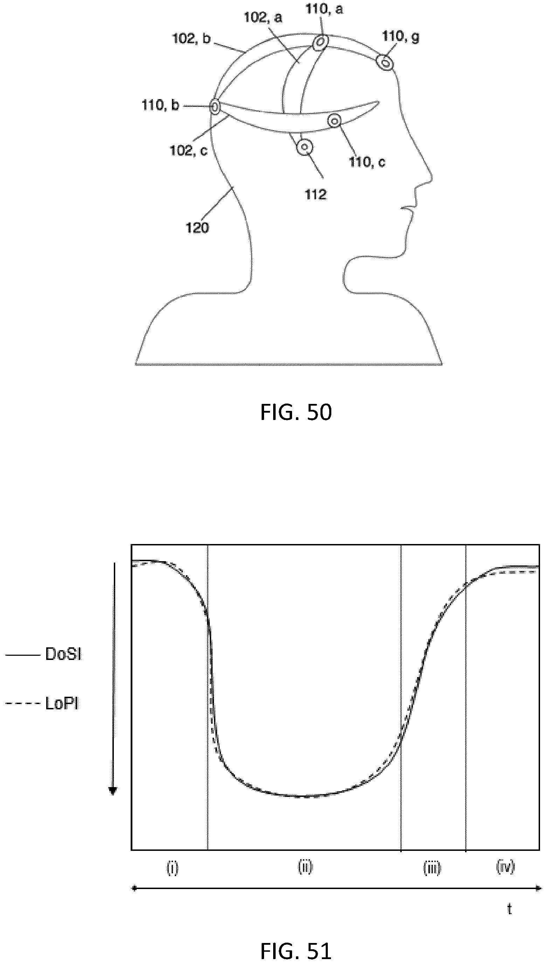

An exemplary embodiment of a wearable device is given in FIG. 46, Fig. 47 and Fig. 48. FIG. 46 depicts an embodiment of a wearable device (100) comprising a media rendered integrated into a virtual reality viewer (104). The wearable device (100) further comprises a plurality of elasticated straps (102,a,b,c) that hold an eye mask (104) supporting a virtual reality viewer and headphones (104) in position on the head (120) of the subject. It is however clear that alternative embodiments are possible of the wearable device (100), which for example do not comprise the eye mask such as for example shown in Fig. 49 and/or the headphones such as for example shown in FIG. 50, but only the electrodes and sensors. The elasticated straps (102,a,b,c) also hold a plurality of electrodes and sensors in position on the head (120) of the subject. Depicted are electrodes (110, a (central (C) EEG electrode), b (parietal (P) EEG electrode), c (temporal), d (frontal (F) EEG electrode)) for measurement of EEG data; sensor (112) for measurement of heart rate, heart rate variability, SPO2; and camera (114) for capture of eye movement and facial expression. FIG. 47 is a view of a face-contacting edge of the embodiment of a mask disposed with electrodes (116, a, b, c, d) for measurement of EEG data; electrode (118) for measurement of skin conductance. FIG. 19 is a view of a face-contacting edge of an embodiment of the mask (104) disposed with

- a frontal (F) EEG electrode (110e) for measurement of EEG data;
- EMG electrode (111a to 111d) for measurement of EMG data;
- PPG sensors (113a, 113b) for measurement of respiratory data, heart rate data, blood pressure, spO2;
- EOG electrodes (115a to 115e) for measurement of EOG data;
- GSR electrode (117) for measurement of GSR data;
- ground electrode (119) that can act a reference/ground electrode in combination with one or more of the EEG (110e), EMG (111a to 111d), EOG (115a to 115e), GSR (117) electrodes.

However it is clear that the system and/or wearable device may make use of any other suitable configuration and/or arrangement of EEG electrodes, such as for example devices comprising one or more electrodes, for example two, three, four or more electrodes arranged at one or more scalp locations referred to in the international 10-20 system, or any other suitable device comprising one or more EEG electrodes. It is further also clear that the system and/or wearable device may make use of any other and/or further suitable configuration and/or arrangement of sensors and/or electrodes, such as for example a body temperature sensor, respiratory data sensor, etc.

According to an embodiment, the controller module may be configured for receiving measured data from the monitoring apparatus, and transforming the response data comprising the measured data into one or more of (N)LoA(I)(S), (N)LoP(I)(S), DoS(I)(S), DoD(I), DoH(I),... . The evaluation protocol may comprise use of one or more of a mathematical (e.g. statistical) model, trained machine-leaning model, mathematical index, reference data.

According to an embodiment, the controller module may be configured for receiving measured data from the monitoring apparatus, and transforming the response data comprising the measured data into the LoA, which is the level of anxiety as experienced by the subject, the LoPI, which is representing the level of pain as perceived by the subject, and/or a DoSI, DoDI and/or DoHI representing a measure of the non-pharmacological and/or pharmacological modified state of consciousness of the subject, using an evaluation protocol as described above. The evaluation protocol may for example comprise use of one or more of a mathematical (e.g. statistical) model, trained machine-leaning model, mathematical index, reference data.

According to an embodiment, the controller module typically comprises a circuit (e.g. microprocessor) configured to perform processing steps and memory. The controller module may or may not be integrated into a wearable device.

The method may be a computer implemented method.

There is further provided is a computing device or system configured for performing the method as described herein.

There is provided a computer program or computer program product having instructions which when executed by a computing device or system causing the computing device or system to perform the method as described herein.

There is provided a computer readable medium having stored thereon instructions which when executed by a computing device or system cause the computing device or system to perform the method as described herein.

There is provided a data stream which is representative of a computer program or computer program product having instructions which when executed by a computing device or system cause the computing device or system to perform the method as described herein.

The method and system described herein enable the therapist to quantify the patients level of anxiety, the level of pain as consciously perceived by the subject, and optionally a state of consciousness, a hypnotic state, ... and/or enable optimal and potentially individual titration of initial dosages of analgesics, anesthetics, anxiolytics; ... hypnotics, and/or optimize pharmacological and/or non-pharmacological sedation (VR therapy titration); and/or provide safer progress by Quantifying, Visualizing, Trending and potentially Predicting the patient's physiological reaction / consciously perceived level of pain during clinical, medical, surgical or therapeutic interventions; and/or increase subject safety; and/or increase subject amnesia of medical intervention.

Also provided is use of a computer-implement method described herein for determining a level of sedation of a subject induced pharmacologically and/or non-pharmacologically. The sedation may for example be for a surgical intervention and/or for example replacing and/or supplementing a pharmacological anesthetic with a non-pharmacological anesthetic.

It is to be understood that this invention is not limited to particular systems and methods or combinations described, since such systems and methods and combinations may, of course, vary. It is also to be understood that the terminology used herein is not intended to be limiting, since the scope of the present invention is determined by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se*, by means of further exemplification, the term encompasses *inter alia* a reference to any one of said members, or to any two or more of said members, such as, e.g., any $\geq 3$, $\geq 4$, $\geq 5$, $\geq 6$ or $\geq 7$ etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilized and structural or logical changes may be made without departing from the scope of the present invention. The following detailed description, therefore, is not to be taken in a limiting sense, and the scope of the present invention is defined by the appended claims.

The inventors have found that for the first time a strong association between response data in particular measured data collected from at least one EEG electrode, such as for example at least one of a frontal-EEG electrode, a parietal -EEG electrode and central-EEG electrode and the LoP of the subject and at an association between data from a parietal -EEG electrode and the LoA and an association between a frontal -EEG electrode and a level of modified consciousness, for example during a pharmacological and/or non-pharmacological treatment session to modify the state of consciousness of the subject. For the first time, an objective measure of a level of pain and/or a level of anxiety of the subject can be determined in real time. This is applicable in many situations, for instance, when a subject is undergoing a surgically invasive procedure under pharmacological and/or non- pharmacological sedation or any alternatively useful situations. In particular it is the first time that a combined measurement, by means of such response data is possible of both a LoA, LoP and/or DoS, particularly during non-pharmacological treatments such as for example hypnosis.

State of consciousness refers to a measurement of a subject's wakefulness and/or self-awareness as well as environmental awareness determining the subject's presence sensation, arousability (emotional response) and (physical) responsiveness to an internal or external stimulus or stimuli, characterized by the physiological activity and/or by a neurological signature.

In a specific modified state of consciousness, for example where the level of consciousness is different or modified with respect to a normal awake state, the subject is for example more relaxed, perceives less or no pain, is more dissociated, is more prone to suggestions, and/or is optionally sedated. In such a specific modified state of consciousness, subject's cognitive functions may be absorbed in a specific task, and brain processes which usually happen together are separated. The creation of this dissociative state allows a reduced perception of peripheral stimuli and a subject's modified perception of the self and the self in the environment. Subject's physiology is usually modified (less involuntary movement *i.e.* swallowing, limb movement, eye blinking, higher stability of vital signs, larger respiratory patterns and improved oxygenation rates...). In such a specific modified state of consciousness, a subject might experience partial or total catalepsy as well as disconnect from the environment (i.e. not hear/ respond to verbal command). Senses may be under/overactivated according to requirements. For example, a subject could be induced into such a specific modified state of consciousness, in which the dissociative state suppresses the conscious perception of pain sufficiently so that it is possible to safely undergo a surgical intervention, for instance.

The state of consciousness induced non-pharmacologically can be determined from one or more of a depth of dissociation (DoD) of the subject during the treatment session as discussed later below.

According to an embodiment a treatment session is applied to the subject to modify the state of consciousness of the subject. The treatment session may include one or more of hypnosis and/or other evidence-based psychological and/or mind/body intervention (*i.e.* non-pharmacological treatment), administration of active pharmacological ingredient (*i.e.* pharmacological treatment), other treatments (e.g. acupuncture, mechanical treatment, other non-pharmacological treatment). Preferably it comprises hypnosis. The degree to which the state of consciousness of the subject has been modified by hypnosis may be referred to as depth of hypnosis (DoH). The degree to which the state of consciousness of the subject has been modified by hypnosis or any other suitable non-pharmacological treatment and/or optionally by active pharmacological ingredient and optionally by other treatments may be referred to as depth of state (DoS).

In particular a non-pharmacological treatment includes for example hypnosis but also other treatments which are used to potentialize the hypnosis session and/or improve dissociation/ a modified level of absorption, a modified state of consciousness/ therapeutic impact, mind-body interventions, etc.. It is clear that alternative non-pharmacological treatment sessions are possible. Examples of pharmacological treatment sessions may comprise administration of an anesthetic, analgesic and/or anxiolytic. Examples of anesthetics include:

- An inhalation agent such as Desflurane, Enflurane, Halothane, Isoflurane, Methoxyflurane, Nitrous oxide, Sevoflurane (inhaled)
- An intravenous agent such as Barbiturates, Amobarbital, Methohexital, Thiamylal, Thiopental, Benzodiazepines, Diazepam, Lorazepam, Midazolam, Etomidate, Ketamine, Propofol.

Examples of anxiolytics include: Barbiturates, Benzodiazepines, Carbamates, Antihistamines, Opioids, Antidepressants, Sympatholytics, etc.

As used herein the term "subject" refers to the beneficiary of the therapeutic session. The "user" refers to a person or persons operating the method or system. The user may be a care provider such as a physician, or medical assistant, or non-medical assistant (e.g. friend, relative, helper) of the subject. In some circumstances, the user may be the subject, for instance, where a treatment is self-administered at home.

Hypnosis is one way of inducing an altered state of consciousness in a subject. The hypnosis therapy may be medical (clinical) hypnosis therapy, or home hypnosis therapy, or hypnosis therapy provided in any care or wellness environment. The hypnosis therapy may be extramural hypnosis therapy that is not provided in a care institution (*i.e.* not provided in a clinic, hospital, care center). While immersed in the altered state of consciousness, the subject's self-perception and the peripheral awareness are affected, changing the subject's experience of his/her sensation, perception, and thoughts, and making the subject prone to follow suggestions. Subject is absorbed and dissociated from reality.

An embodiment of a hypnotic treatment session typically comprises 4 sequential phases as exemplified in FIG.

51, (i) an induction phase, (ii) a deepening phase, (iii) a transition phase, and/or (iv) a re-alerting phase. While immersed in the treatment sessions the subject's self-perception and the peripheral awareness are affected, the immersion depth increasing during the (i) phase and reaching a maximum during the (ii) phase. The subject becomes prone to suggestive control during the (ii) phase, during which time the subject may be sedated, induced to relax, etc. Afterwards the subject is returned to a normal state of a consciousness by passing the (iii) and (iv) phases.

The phases are described in detail below, namely:

(i) The induction phase, wherein the subject is prepared to be immersed into the altered state. In this phase the subject is typically provided with a feeling of comfort, safety and relaxation to the subject.

(ii) The deepening phase, wherein the subject is placed in an altered state of consciousness. This state is typically characterized by full dissociation of reality and lack of or reduced movement unless expressly suggested in the therapy.

(iii) The transition phase, wherein the subject is exposed to suggestive information, which may aid in remembering or forgetting specific event of the therapy and/or to addressing one or more subject specific issues (also called post-hypnotic suggestions).

(iv) Re-alerting phase, wherein the subject is returned to a normal state of consciousness. In this phase the subject typically returns his/her senses and the dissociative state ends.

The hypnotic treatment session may be delivered by a hypnotist, more preferably it is presented using a media renderer such as a virtual reality head set for a fully immersive effect as described below in more detail. A stored hypnotic treatment session may be played through the media renderer, the content of the treatment session may be specific to the treatment goals.

While in such an altered state of consciousness, for example induced by treatments or activities inducing a higher level of absorption and/or attention, induced by hypnosis, or induced by any other suitable treatments, or alternatively any other suitable treatment not necessarily involving an altered state of consciousness, the subject can better manage anxiety and/or pain as well as other symptoms/ issues. The subject can for example undergo medical interventions (e.g. invasive procedures) in the absence or with a lower dose of a pharmacological (anesthetic/analgesic/anxiolytic) agent.

**Claims**

1. A computer-implemented method for measurement of a level of anxiety combined with and/or correlated with a level of a modified state of consciousness, and/or a level of pain, wherein the method comprises the steps of:

- receiving measured data comprising EEG data, collected from at least one of:

- at least one parietal (P) electrode configured for collection of parietal EEG (P-EEG) data from the scalp anatomical region corresponding to a parietal lobe of a subject;
- at least one frontal (F) electrode configured for collection of frontal EEG (F-EEG) data from the scalp anatomical region corresponding to a frontal lobe of a subject,

and optionally, electromyography, EMG, data; and
- extracting from the EEG data collected from the P-EEG data, a group 8 indicator based on a power, P-power (dt), associated with a delta-theta frequency band, dt, within a delta-theta frequency range; combined with:

- extracting from the EEG data collected from the F-EEG data a group 1 indicator based on, a power, F-power(dt), associated with a delta-theta frequency band, dt, within a delta-theta frequency range;

- and, optionally, from the EEG data collected from the F-EEG data, a group 2 indicator based on a mean signal peak-to-peak amplitude, F-MSPA;

and/or
- extracting from the EEG data, a group 4 indicator corresponding to a power, power (ta), associated with a theta-alpha frequency band, ta, within a theta-alpha frequency range;

- and, optionally, from the EEG data, a group 3 indicator corresponding to a mean signal peak-to-peak amplitude, MSPA;

- and, optionally, from the EMG data, a group 5 indicator corresponding to a mean signal peak-to-peak amplitude, EMG-MSPA; and

- determining, based on said group 8 indicator, a level of anxiety, LoA, which is a value indicative for the level of anxiety in the subject; combined with:

- determining, based on said group 1 indicator, and optionally said group 2 indicator, a depth of state, DoS, which is a value indicative for the level of a modified state of consciousness in the subject; and/or
- determining, based on said group 4 indicator, and optionally said group 3 indicator and/or group 5 indicator, a level of pain, LoP, which is a value indicative for the level of pain in the subject.

2. The method according to claim 1, wherein the method comprises the steps of:

- correlating a plurality of LoA, LoP and/or DoS measurements and/or determining a correlation between a plurality of LoA, LoP and/or DoS measurements;
- determining a plurality of LoA at different DoS from measurement data and/or measurement data comprising simultaneous measurement of LoA and DoS; and/or
- determining the modulation of LoA by DoS, by evaluating the evolution of the LoA at different DoS and/or the evolution of the LoA with respect to the evolution of DoS from the measurement data.

3. The method according to claim 1 or 2, wherein the method comprises the steps of:

- determining a plurality of LoP at different DoS and/or LoA from measurement data and/or measurement data comprising simultaneous measurement of LoA and DoS; and/or
- determining the modulation of LoP by DoS and/or LoA, by evaluating the evolution of the LoP at different DoS and/or LoA and/or the evolution of the LoP with respect to the evolution of DoS and/or LoA from the measurement data;

4. The method according to any of the preceding claims, wherein the method comprises the further step of:

- adjusting said LoA based on said DoS to a level of normalized anxiety, NLoA, which is a value indicative for the level of anxiety in the subject at a predetermined reference DoS; and/or
- adjusting said LoA based on said LoP to a level of normalized anxiety, NLoA, which is a value indicative for the level of anxiety in the subject at a predetermined reference LoP;
- adjusting said LoP based on said DoS and/or LoA to a level of normalized pain, NLoP, which is a value indicative for the level of pain in the subject at a predetermined reference DoS and/or a predetermined reference LoA;
- calculating, based on said LoA and/or said LoP, at least one limit and/or range for said DoS, DoSLimit, which is indicative for the at least one limit and/or range of the modification of the level of consciousness attainable when the subject experiences said LoA and/or said LoP; and/or
- calculating, based on said LoA, and/or DoS at least one limit and/or range for said LoP, LoPLimit, which is indicative for the at least one limit and/or range of the level of pain attainable when the subject experiences said LoA and/or DoS; and/or
- calculating, based on said LoP, at least one limit and/or range for said LoA, LoALimit, which is indicative for the at least one limit and/or range of the level of pain attainable when the subject experiences said LoP.

5. A computer-implemented method for measuring and/or monitoring and/or predicting and/or aggregating a level of anxiety combined with and/or correlated with a level of a modified state of consciousness and/or a level of pain in a subject, preferably before, during and/or after a treatment, wherein the method comprises the steps of:

- measurement of the LoA combined with the DoS and/or the LoP according to any of the preceding claims at at least two different points in time, preferably before, during and/or after the treatment; and
- measuring and/or monitoring and/or predicting an evolution, preferably a treatment-induced evolution, of the LoA combined with the DoS and/or the LoP, based on a respective comparison of at least two LoA combined with and/or correlated with at least two DoS and/or at least two LoP measured at at least two different points in time; and/or
- aggregating a level of anxiety score or LoAS and/or a Depth of State Score or DoSS and/or a level of pain score or LoPS based on an aggregation of at least two LoA and/or at least two DoS and/or at least two LoP measured at at least two different points in time and/or over a period of time.

6. The method according to any of the preceding claims, wherein the method comprises the step of:

    - determining the treatment-induced evolution of the LoA combined with DoS and/or LoP, based on a respective comparison of the LoA combined with DoS and/or LoP determined during and/or after a treatment, with the LoA combined with DoS and/or LoP determined before a treatment.

7. The method according to claim 5 or 6, wherein:

    - the treatment is a pain management treatment and the method comprises the step of:

        - replacing and/or combining the pain management treatment with an anxiety management treatment when the LoP and/or the LoA is outside a predetermined range and/or until the LoP and/or the LoA is inside the predetermined range;

    - the treatment is an anxiety management treatment and the method comprises the step of:

        - replacing and/or combining the anxiety management treatment with a pain management treatment when the LoP and/or the LoA is outside a predetermined range and/or until the LoP and/or the LoA is inside the predetermined range;

    - the treatment is an anxiety management treatment and the method comprises the step of:

        - replacing and/or combining the anxiety management treatment with a treatment modifying the level of consciousness when the DoS and/or LoA is outside a predetermined range and/or until the DoS and/or the LoA is inside the predetermined range; and/or

    - the treatment is a treatment modifying the state of consciousness and the method comprises the step of:

        - replacing and/or combining the treatment modifying the state of consciousness with an anxiety and/or pain reducing treatment when the LoP and/or LoA and/or DoS is outside a predetermined range and/or until the LoP and/or the LoA and/or DoS is inside the predetermined range.

8. The method according to claim 6 or 7, when dependent on claim 4 (NLoA), wherein the treatment is an anxiety-reducing treatment and the method comprises the step of:

    - determining and/or monitoring the efficacy of an anxiety-reducing treatment based on the treatment-induced evolution of the NLoA.

9. The method according to claim 6 or 7, wherein the treatment is a treatment modifying the state of consciousness for reducing anxiety and the method comprises the steps of:

    - measuring, the DoS at at least two different points in time, before, during and/or after the treatment modifying the state of consciousness; and/or
    - determining the treatment-induced evolution of the DoS and/or the LoA, based on a comparison of the DoS and/or the LoA measured during and/or after the treatment modifying the state of consciousness with the DoS and/or the LoA measured before the treatment modifying the state of consciousness.

10. A computer-implemented method for determining a treatment and/or determining an adjustment to a treatment and/or determining an intensity of a treatment and/or determining the efficacy of a treatment and/or determining the choice of a treatment, wherein the method comprises the steps of:

    - measuring and/or monitoring and/or predicting and/or aggregating a NLoA and/or NLoP and/or DoSLimit and/or LoPLimit and/or LoALimit in a subject according to claim 4; and
    - determining a treatment and/or determining an adjustment to a treatment and/or determining an intensity of a treatment and/or determining the efficacy of a treatment and/or determining the choice of a treatment based on the measured and/or monitored and/or predicted and/or aggregated NLoA and/or NLoP and/or DoSLimit and/or LoPLimit and/or LoALimit.

11. The method according to any of the preceding claims, wherein the method comprises the steps of:

   - receiving reference data comprising measured data of a subject and/or a population correlated to at least one predetermined reference LoA, combined with DoS and/or LoP respectively;
   - determining at least one correlation of at least one predetermined reference LoA, combined with DoS and/or LoP respectively and at least one respective group indicator extracted from the reference data; and
   - scaling and/or indexing a subsequently measured LoA, combined with DoS and/or LoP with respect to the at least one predetermined reference LoA, combined with DoS and/or LoP respectively by means of said at least one respective correlation, and optionally determining a level of anxiety index or LoAI, and a Depth of State index or DoSI and/or a level of pain index or LoPI therefrom.

12. The method according to any of the preceding claims, wherein the theta-alpha frequency band, ta, comprises one or more of the following:

   - a frequency band encompassing both theta and alpha brain waves;
   - a frequency band extending from 6Hz up to and including 12Hz; and/or
   - a bandwidth of at least 2Hz and a frequency band comprising at least fast theta waves extending from 6Hz up to and including 8Hz, and/or

   wherein the theta-alpha frequency range:

   - comprises a frequency range encompassing both theta and alpha brain waves; and/or
   - extends from 4 Hz up to and including 12 Hz, and/or

   wherein the delta-theta (dt) frequency band, dt, comprises one or more of the following:

   - a frequencies band encompassing both delta and theta brain waves;
   - a frequency band extending from 0 Hz up to and including 8 Hz.
   - a bandwidth of at least 2 Hz and a frequency band comprising at least slow theta brain wave extending from 3 Hz up to and including 6 Hz; and/or

   wherein the delta-theta frequency range:

   - comprises a frequency range encompassing both delta and alpha brain waves; and/or
   - extends from 1 Hz up to and including 6 Hz.

13. The method according to claim any of the preceding claims, the method comprising steps of:

   - receiving measured data further comprising cardiovascular data
   - extracting from the cardiovascular data:

      - a group 7 indicator based on a heart rate and/or heart rate variability, preferably a combination of heart rate and heart rate variability, from the cardiovascular data; and
      - determining the LoA based on said group 8 indicator and at least the group 7 indicator;

   and/or the steps of:

   - receiving measured data further comprising respiratory data;
   - extracting from the respiratory data:

      - a group 6 indicator based on respiration rate and/or a respiration variability and/or oxygen saturation and/or predetermined respiratory patterns from the respiratory data;

   - determining the LoA based on said group 8 indicator and at least the group 6 indicator;

   and/or the steps of:

- receiving measured data further comprising

   - ocular movement data;

- extracting from the ocular movement data:

   - a group 9 indicator based on an ocular movement rate and/or ocular movement amplitude and/or predetermined ocular movement patterns from the ocular movement data;

- determining the LoA based on said group 8 indicator and at least the group 9 indicator;
and/or the optional steps of:

   - the ocular movement data comprises EOG data; and/or
   - the group 9 indicator is based on the detection of saccadic eye movements from the ocular movement data, and optionally one or more characteristics of the saccadic eye movements;

and/or the steps of:

- receiving measured data further comprising:

   - muscular activity data;

- extracting from the muscular activity data:

   - a group 10 indicator based on a muscular activity from the muscular activity data;

- determining the LoA based on said group 8 indicator and at least the group 10 indicator;
and/or the optional steps of:

   - the muscular activity data comprises electromyography, EMG, data; and/or
   - the group 10 indicator is based on an electromyographic, EMG, activity from the EMG data;

and/or the steps of:

- receiving measured data further comprising

   - body temperature data;

- extracting from the body temperature data:

   - a group 11 indicator based on a body temperature from the body temperature data;

- determining the LoA based on said group 8 indicator and at least the group 11 indicator.

14. A system configured to measure a LoA combined with and/or correlated with a DoS and/or a LoP according to the method according to any of the preceding claims, the system comprising:

- a monitoring apparatus configured to obtain measured data comprising EEG data comprising data, collected from at least one of:

   - at least one parietal (P) electrode configured for collection of P-EEG data from the scalp anatomical region corresponding to a parietal lobe of a subject;
   - at least one frontal (F) electrode configured for collection of F-EEG data from the scalp anatomical region corresponding to a frontal lobe of a subject,

- the measured data optionally further comprising electromyography, EMG, data; and
- a controller module configured for:

76

- receiving the measured data from the monitoring apparatus;
- extracting:

- from the EEG data collected from the P-EEG data, a group 8 indicator based on a power, P-power (dt), associated with a delta-theta frequency band, dt, within a delta-theta frequency range; and

- extracting:

- from the EEG data, a group 1 indicator based on, a power, F-power(dt), associated with a delta-theta frequency band, dt, within a delta-theta frequency range extracted from the at least one F- EEG electrode data;

- and, optionally, from the EEG data collected from the F-EEG data, a group 2 indicator based on a mean signal peak-to-peak amplitude, F-MSPA;

and/or
- from the EEG data, a group 4 indicator corresponding to a power, power(ta), associated with a theta-alpha frequency band, ta, within a theta-alpha frequency range;

- and, optionally, from the EEG data, a group 3 indicator corresponding to a mean signal peak-to-peak amplitude, MSPA;
- and, optionally, from the EMG data, a group 5 indicator corresponding to a mean signal peak-to-peak amplitude, EMG-MSPA

- determining, based on said group 8 indicator, a level of anxiety, LoA, which is a value indicative for the level of anxiety in the subject; and
- determining:

- based on said group 1 indicator, and optionally said group 2 indicator, a depth of state, DoS, which is a value indicative for the level of a modified state of consciousness; and/or
- based on said group 4 indicator and optionally said group 3 indicator and optionally said group 5 indicator, a level of pain, LoP, which is a value indicative for the level of pain in the subject.

15. A system according to claim 14, wherein the monitoring apparatus comprises, for obtaining measured data, one or more of:

- one or more electrodes configured for collection of electroencephalogram, EEG, data comprising:

- one or more parietal (P) electrodes configured for collection of P-EEG data from the scalp anatomical region corresponding to a parietal lobe of the subject,
- one or more frontal (F) electrodes configured for collection of F-EEG data from the scalp anatomical region corresponding to a frontal lobe of the subject,
- optionally, one or more central (C) electrodes configured for collection of C-EEG data from the scalp anatomical region corresponding to a precentral and postcentral gyrus of the subject,
- optionally, one or more further electrodes configured for collection of electroencephalogram, EEG, data;

- optionally a respiratory data capturing unit;
- optionally a cardiovascular data capturing unit;
- optionally an ocular movement data capturing unit;
- optionally a muscular activity data capturing unit;
- optionally a body temperature data capturing unit; and/or

wherein the system further comprises a graphical user interface, GUI, configured to indicate numerically and/or graphically one or more of:

- at least two LoA, DoS and/or LoP respectively measured at different points in time, preferably a historic, current and/or expected LoA, DoS, and/or LoP.
- the evolution of and/or ratio between and/or aggregation of at least two LoA, DoS and/or LoP respectively measured at different points in time;
- optionally, one or more components of the measured data, preferably one or more EEG data and optionally EMG data, respiratory data, cardiovascular data, muscular activity data, ocular movement data and/or body temperature data; and/or
- optionally any (N)LoA(I)(S), (N)LoP(I)(S), and/or DoS(I)(S), LoALimit, LoPLimit and/or DoSlimit.

# Fig. 1

## FIG. 2

FIG. 3

FIG. 4

300

Receive EEG data — 302

304

Extract group 8
indicator:
P-Power(dt)

Extract group 4
indicator:
Power(ta) — 314

Extract group 1
indicator:
F-Power(dt) — 308

310

Determine LoA

Determine LoP

Determine DoS

306

316

Correlate LoA / LoP / DoS

318

FIG. 5

treatment

400

408

Measure LoA / DoS / LoP
at first point in time — 402

Measure LoA / DoS / LoP
At second point in time — 404

410

300

300

Determine treatment-induced
evolution of LoA / DoS / LoP — 406

FIG. 6

FIG. 7

FIG. 8

FIG. 9

Self-Reported Anxiety

FIG. 10

FIG. 11

FIG. 12

FIG. 13

FIG. 14

FIG. 15

FIG. 16

Time domain response at central EEG electrode

FIG. 17

Time domain response at Parietal EEG electrode

FIG. 18

Time domain response at Frontal EEG electrode

FIG. 19

FIG. 20

FIG. 21

FIG. 22

FIG. 23

FIG. 24

FIG. 25

FIG. 26

FIG. 27

FIG. 28

FIG. 29

FIG. 30

FIG. 31

FIG. 32

FIG. 33

EP 3 955 257 A1

FIG. 34

FIG. 35

FIG. 36

**EP 3 955 257 A1**

FIG. 37

FIG. 38

97

FIG. 39A

200, b

FIG. 39B

FIG. 40A

200, c

FIG. 40B

200, c

FIG. 41

FIG. 42

FIG. 43

200, e

FIG. 44

200, f

242

244

240

LoAI

Time (min)

FIG. 45

200, g

LoPI     DoSI     LoAI

FIG. 46

FIG. 47

FIG. 48

FIG. 49

FIG. 50

FIG. 51

230

LoAIS

**9**

260

%

262

Units

234

Total time

236

Summary

238

Info

**FIG. 52**

255

260

%

200, i

LoAIS

258

79

218

76-100

220

51-75

256

26-50

222

0-25

224

261

t = 30 min

262

units

**FIG. 53**

FIG. 54

FIG. 55

$$\frac{LoA^*}{Min(LoA)}$$

**Recovery coefficient**

1.4

1

40%  50%   100 %
Reference
DoS

DoS

## FIG. 56

**Risk Index**

# LOW
C

230

260

Units

262

LoPI

234

LoAI

236

DoSI

238

200, k

## FIG. 57

# EP 3 955 257 A1

## EUROPEAN SEARCH REPORT

Application Number

EP 20 19 1014

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YPPARILA H: "Depth of sedation in intensive care patients. A neurophysiological study", 14 June 2004 (2004-06-14), DISSERTATION,, PAGE(S) 104PP, XP007917111, * abstract; figures 4.5, 7.1-7.3 * * page 19, paragraph 2 * * page 56, paragraph 3 - paragraph 6 * * page 37, paragraph 1 * * page 40, paragraphs 2,5 * * page 49, paragraph 1 - paragraph 3 * * page 13, paragraph 2; table 4.1 * ----- | 1-15 | INV. G16H20/00 ADD. G16H50/00 |

TECHNICAL FIELDS SEARCHED (IPC)

G16H

The present search report has been drawn up for all claims

| Place of search | Date of completion of the search | Examiner |
|---|---|---|
| Munich | 27 January 2021 | Schmitt, Constanze |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

**EP 3 955 257 A1**

REFERENCES CITED IN THE DESCRIPTION

**Patent documents cited in the description**

- EP 2020053136 W